# EUROPEAN PATENT SPECIFICATION

(11) **EP 2 195 426 B1**
(45) Date of publication and mention of the grant of the patent: **12.06.2013**
(21) Application number: 08836521.8
(22) Date of filing: 06.10.2008
(51) Int. Cl.: C12N 15/00, C12N 15/09, C07H 21/04, C07K 14/52, C07K 14/705

(54) **SYSTEM FOR CAPTURING AND MODIFYING LARGE PIECES OF GENOMIC DNA AND CONSTRUCTING ORGANISMS WITH SYNTHETIC CHLOROPLASTS**
SYSTEM ZUR ERFASSUNG UND MODIFIZIERUNG GROSSER TEILE GENOMISCHER DNA UND ZUR KONSTRUKTION VON ORGANISMEN MIT SYNTHETISCHEN CHLOROPLASTEN
SYSTÈME PERMETTANT DE CAPTURER ET DE MODIFIER DE GRANDS MORCEAUX D'ADN GÉNOMIQUE, ET DE CONSTRUIRE DES ORGANISMES COMPRENANT DES CHLOROPLASTES SYNTHÉTIQUES

(30) Priority: 05.10.2007 US 978024 P
(43) Date of publication of application: 16.06.2010
(73) Proprietor: Sapphire Energy, Inc., San Diego, CA 92121 (US)
(72) Inventor: MENDEZ, Michael, San Diego, CA 92130 (US); O'NEILL, Bryan, San Diego, CA 92130 (US); MIKKELSON, Kari, San Diego, CA 92106 (US)
(74) Representative: Kiddle, Simon John
(86) International application number: PCT/US2008/011540
(87) International publication number: WO 2009/045550

(56) References cited:
- WO-A1-00/75299
- WO-A1-99/10513
- US-A- 5 866 404
- US-B1- 7 129 391
- GUPTA M ET AL: "Entire maize chloroplast genome is stably maintained in a yeast artificial chromosome." PLANT MOLECULAR BIOLOGY SEP 1991 LNKD- PUBMED:1679355, vol. 17, no. 3, September 1991 (1991-09), pages 361-369, XP009139862 ISSN: 0167-4412
- M. SUZANNS BRADSHAW ET AL.: 'A new vector for recombination-based cloning of large DNA fragmentsfrom yeast artificial chromosomes' NUCLEIC ACIDS RESEARCH vol. 23, no. 23, 1995, pages 4850 - 4856, XP002027291
- DELIN LIANG ET AL.: 'Site-directed mutagenesis and generation of chimeric viruses by homologous recombination in yeast to facilitate analysis of plant-virus interactions' MPMI vol. 17, no. 6, 2004, pages 571 - 576, XP008131475

### SEQUENCE LISTING

<110> SAPPHIRE ENERGY, INC
<120> SYSTEM FOR CAPTURING AND MODIFYING LARGE PIECES OF GENOMIC DNA AND CONSTRUCTING ORGANISMS WITH SYNTHETIC CHLOROPLASTS
<130> SJK/FP6683858
<140> EP 08836521.8
   <141> 2008-10-06
<150> 60/978,024
   <151> 2007-10-05
<160> 99
<170> PatentIn version 3.5
<210> 1
   <211> 4572
   <212> DNA
   <213> Artificial Sequence
<220>
   <223> Description of Artificial Sequence: Synthetic polynucleotide
<400> 1
   tcgtgtagat aactacgata cgggagggct taccatctgg ccccagtgct gcaatgatac 60
   cgcgagaccc acgctcaccg gctccagatt tatcagcaat aaaccagcca gccggaaggg 120
   ccgagcgcag aagtggtcct gcaactttat ccgcctccat ccagtctatt aattgttgcc 180
   gggaagctag agtaagtagt tcgccagtta atagtttgcg caacgttgtt gccattgcta 240
   caggcatcgt ggtgtcacgc tcgtcgtttg gtatggcttc attcagctcc ggttcccaac 300
   gatcaaggcg agttacatga tcccccatgt tgtgcaaaaa agcggttagc tccttcggtc 360
   ctccgatcgt tgtcagaagt aagttggccg cagtgttatc actcatggtt atggcagcac 420
   tgcataattc tcttactgtc atgccatccg taagatgctt ttctgtgact ggtgagtact 480
   caaccaagtc attctgagaa tagtgtatgc ggcgaccgag ttgctcttgc ccggcgtcaa 540
   tacgggataa taccgcgcca catagcagaa ctttaaaagt gctcatcatt ggaaaacgtt 600
   cttcggggcg aaaactctca aggatcttac cgctgttgag atccagttcg atgtaaccca 660
   ctcgtgcacc caactgatct tcagcatctt ttactttcac cagcgtttct gggtgagcaa 720
   aaacaggaag gcaaaatgcc gcaaaaaagg gaataagggc gacacggaaa tgttgaatac 780
   tcatactctt cctttttcaa tattattgaa gcatttatca gggttattgt ctcatgagcg 840
   gatacatatt tgaatgtatt tagaaaaata aacaaatagg ggttccgcgc acatttcccc 900
   gaaaagtgcc acctgacgtc taagaaacca ttattatcat gacattaacc tataaaaata 960
   ggcgtatcac gaggcccttt cgtctcgcgc gtttcggtga tgacggtgaa aacctctgac 1020
   acatgcagct cccggagacg gtcacagctt gtctgtaagc ggatgccggg agcagacaag 1080
   cccgtcaggg cgcgtcagcg ggtgttggcg ggtgtcgggg ctggcttaac tatgcggcat 1140
   cagagcagat tgtactgaga gtgcaccata gggcggccgc ggcgcgccgt tccggatctg 1200
   catcctgcga tgcagatccg gaacataatg gtgcagggcg ctgacttccg cgtttccaga 1260
   ctttacgaaa cacggaaacc gaagaccatt catgttgttg ctcaggtcgc agacgttttg 1320
   cagcagcagt cgcttcacgt tcgctcgcgt atcggtgatt cattctgcta accagtaagg 1380
   caaccccgcc agcctagccg ggtcctcaac gacaggagca cgatcatgcg cacccgtggc 1440
   caggacccaa cgctgcccga gatgcgccgc gtgcggctgc tggagatggc ggacgcgatg 1500
   gatatgttct gccaagggtt ggtttgcgca ttcacagttc tccgcaagaa ttgattggct 1560
   ccaattcttg gagtggtgaa tccgttagcg aggtgccgcc ggcttccatt caggtcgagg 1620
   tggcccggct ccatgcaccg cgacgcaacg cggggaggca gacaaggtat agggcggcgc 1680
   ctacaatcca tgccaacccg ttccatgtgc tcgccgaggc ggcataaatc gccgtgacga 1740
   tcagcggtcc aatgatcgaa gttaggctgg taagagccgc gagcgatcct tgaagctgtc 1800
   cctgatggtc gtcatctacc tgcctggaca gcatggcctg caacgcgggc atcccgatgc 1860
   cgccggaagc gagaagaatc ataatgggga aggccatcca gcctcgcgtc gcgaacgcca 1920
   gcaagacgta gcccagcgcg tcggccgcca tgccggcgat aatggcctgc ttctcgccga 1980
   aacgtttggt ggcgggacca gtgacgaagg cttgagcgag ggcgtgcaag attccgaata 2040
   ccgcaagcga caggccgatc atcgtcgcgc tccagcgaaa gcggtcctcg ccgaaaatga 2100
   cccagagcgc tgccggcacc tgtcctacga gttgcatgat aaagaagaca gtcataagtg 2160
   cggcgacgat agtcatgccc cgcgcccacc ggaaggagct gactgggttg aaggctctca 2220
   agggcatcgg tcgagcttga cattgtagga cgtttaaaca ttaccctgtt atccctaggc 2280
   cggcctaaga aaccattatt atcatgacat taacctataa aaataggcgt atcacgaggc 2340
   cctttcgtct tcaagaaatt cggtcgaaaa aagaaaagga gagggccaag agggagggca 2400
   ttggtgacta ttgagcacgt gagtatacgt gattaagcac acaaaggcag cttggagtat 2460
   gtctgttatt aatttcacag gtagttctgg tccattggtg aaagtttgcg gcttgcagag 2520
   cacagaggcc gcagaatgtg ctctagattc cgatgctgac ttgctgggta ttatatgtgt 2580
   gcccaataga aagagaacaa ttgacccggt tattgcaagg aaaatttcaa gtcttgtaaa 2640
   agcatataaa aatagttcag gcactccgaa atacttggtt ggcgtgtttc gtaatcaacc 2700
   taaggaggat gttttggctc tggtcaatga ttacggcatt gatatcgtcc aactgcatgg 2760
   agatgagtcg tggcaagaat accaagagtt cctcggtttg ccagttatta aaagactcgt 2820
   atttccaaaa gactgcaaca tactactcag tgcagcttca cagaaacctc attcgtttat 2880
   tcccttgttt gattcagaag caggtgggac aggtgaactt ttggattgga actcgatttc 2940
   tgactgggtt ggaaggcaag agagccccga aagcttacat tttatgttag ctggtggact 3000
   gacgccagaa aatgttggtg atgcgcttag attaaatggc gttattggtg ttgatgtaag 3060
   cggaggtgtg gagacaaatg gtgtaaaaga ctctaacaaa atagcaaatt tcgtcaaaaa 3120
   tgctaagaaa taggttatta ctgagtagta tttatttaag tattgtttgt gcacttgcct 3180
   gcaggccttt tgaaaagcaa gcataaaaga tctaaacata aaatctgtaa aataacaaga 3240
   tgtaaagata atgctaaatc atttggcttt ttgattgatt gtacaggaaa atatacatcg 3300
   ttaattaagc ggccgcgagc ttggcgtaat catggtcata gctgtttcct gtgtgaaatt 3360
   gttatccgct cacaattcca cacaacatac gagccggaag cataaagtgt aaagcctggg 3420
   gtgcctaatg agtgagctaa ctcacattaa ttgcgttgcg ctcactgccc gctttccagt 3480
   cgggaaacct gtcgtgccag ctgcattaat gaatcggcca acgcgcgggg agaggcggtt 3540
   tgcgtattgg gcgctcttcc gcttcctcgc tcactgactc gctgcgctcg gtcgttcggc 3600
   tgcggcgagc ggtatcagct cactcaaagg cggtaatacg gttatccaca gaatcagggg 3660
   ataacgcagg aaagaacatg tgagcaaaag gccagcaaaa ggccaggaac cgtaaaaagg 3720
   ccgcgttgct ggcgtttttc cataggctcc gcccccctga cgagcatcac aaaaatcgac 3780
   gctcaagtca gaggtggcga aacccgacag gactataaag ataccaggcg tttccccctg 3840
   gaagctccct cgtgcgctct cctgttccga ccctgccgct taccggatac ctgtccgcct 3900
   ttctcccttc gggaagcgtg gcgctttctc atagctcacg ctgtaggtat ctcagttcgg 3960
   tgtaggtcgt tcgctccaag ctgggctgtg tgcacgaacc ccccgttcag cccgaccgct 4020
   gcgccttatc cggtaactat cgtcttgagt ccaacccggt aagacacgac ttatcgccac 4080
   tggcagcagc cactggtaac aggattagca gagcgaggta tgtaggcggt gctacagagt 4140
   tcttgaagtg gtggcctaac tacggctaca ctagaagaac agtatttggt atctgcgctc 4200
   tgctgaagcc agttaccttc ggaaaaagag ttggtagctc ttgatccggc aaacaaacca 4260
   ccgctggtag cggtggtttt tttgtttgca agcagcagat tacgcgcaga aaaaaaggat 4320
   ctcaagaaga tcctttgatc ttttctacgg ggtctgacgc tcagtggaac gaaaactcac 4380
   gttaagggat tttggtcatg agattatcaa aaaggatctt cacctagatc cttttaaatt 4440
   aaaaatgaag ttttaaatca atctaaagta tatatgagta aacttggtct gacagttacc 4500
   aatgcttaat cagtgaggca cctatctcag cgatctgtct atttcgttca tccatagttg 4560
   cctgactccc cg 4572
<210> 2
   <211> 2474
   <212> DNA
   <213> Artificial Sequence
<220>
   <223> Description of Artificial Sequence: Synthetic polynucleotide
<400> 2
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatagggc ggccgccagc tggaattcta cgtactgcag agtactgcgg ccgcgagctt 240
   ggcgtaatca tggtcatagc tgtttcctgt gtgaaattgt tatccgctca caattccaca 300
   caacatacga gccggaagca taaagtgtaa agcctggggt gcctaatgag tgagctaact 360
   cacattaatt gcgttgcgct cactgcccgc tttccagtcg ggaaacctgt cgtgccagct 420
   gcattaatga atcggccaac gcgcggggag aggcggtttg cgtattgggc gctcttccgc 480
   ttcctcgctc actgactcgc tgcgctcggt cgttcggctg cggcgagcgg tatcagctca 540
   ctcaaaggcg gtaatacggt tatccacaga atcaggggat aacgcaggaa agaacatgtg 600
   agcaaaaggc cagcaaaagg ccaggaaccg taaaaaggcc gcgttgctgg cgtttttcca 660
   taggctccgc ccccctgacg agcatcacaa aaatcgacgc tcaagtcaga ggtggcgaaa 720
   cccgacagga ctataaagat accaggcgtt tccccctgga agctccctcg tgcgctctcc 780
   tgttccgacc ctgccgctta ccggatacct gtccgccttt ctcccttcgg gaagcgtggc 840
   gctttctcat agctcacgct gtaggtatct cagttcggtg taggtcgttc gctccaagct 900
   gggctgtgtg cacgaacccc ccgttcagcc cgaccgctgc gccttatccg gtaactatcg 960
   tcttgagtcc aacccggtaa gacacgactt atcgccactg gcagcagcca ctggtaacag 1020
   gattagcaga gcgaggtatg taggcggtgc tacagagttc ttgaagtggt ggcctaacta 1080
   cggctacact agaagaacag tatttggtat ctgcgctctg ctgaagccag ttaccttcgg 1140
   aaaaagagtt ggtagctctt gatccggcaa acaaaccacc gctggtagcg gtggtttttt 1200
   tgtttgcaag cagcagatta cgcgcagaaa aaaaggatct caagaagatc ctttgatctt 1260
   ttctacgggg tctgacgctc agtggaacga aaactcacgt taagggattt tggtcatgag 1320
   attatcaaaa aggatcttca cctagatcct tttaaattaa aaatgaagtt ttaaatcaat 1380
   ctaaagtata tatgagtaaa cttggtctga cagttaccaa tgcttaatca gtgaggcacc 1440
   tatctcagcg atctgtctat ttcgttcatc catagttgcc tgactccccg tcgtgtagat 1500
   aactacgata cgggagggct taccatctgg ccccagtgct gcaatgatac cgcgagaccc 1560
   acgctcaccg gctccagatt tatcagcaat aaaccagcca gccggaaggg ccgagcgcag 1620
   aagtggtcct gcaactttat ccgcctccat ccagtctatt aattgttgcc gggaagctag 1680
   agtaagtagt tcgccagtta atagtttgcg caacgttgtt gccattgcta caggcatcgt 1740
   ggtgtcacgc tcgtcgtttg gtatggcttc attcagctcc ggttcccaac gatcaaggcg 1800
   agttacatga tcccccatgt tgtgcaaaaa agcggttagc tccttcggtc ctccgatcgt 1860
   tgtcagaagt aagttggccg cagtgttatc actcatggtt atggcagcac tgcataattc 1920
   tcttactgtc atgccatccg taagatgctt ttctgtgact ggtgagtact caaccaagtc 1980
   attctgagaa tagtgtatgc ggcgaccgag ttgctcttgc ccggcgtcaa tacgggataa 2040
   taccgcgcca catagcagaa ctttaaaagt gctcatcatt ggaaaacgtt cttcggggcg 2100
   aaaactctca aggatcttac cgctgttgag atccagttcg atgtaaccca ctcgtgcacc 2160
   caactgatct tcagcatctt ttactttcac cagcgtttct gggtgagcaa aaacaggaag 2220
   gcaaaatgcc gcaaaaaagg gaataagggc gacacggaaa tgttgaatac tcatactctt 2280
   cctttttcaa tattattgaa gcatttatca gggttattgt ctcatgagcg gatacatatt 2340
   tgaatgtatt tagaaaaata aacaaatagg ggttccgcgc acatttcccc gaaaagtgcc 2400
   acctgacgtc taagaaacca ttattatcat gacattaacc tataaaaata ggcgtatcac 2460
   gaggcccttt cgtc 2474
<210> 3
   <211> 6638
   <212> DNA
   <213> Artificial Sequence
<220>
   <223> Description of Artificial sequence: Synthetic polynucleotide
<400> 3
   tcgtgtagat aactacgata cgggagggct taccatctgg ccccagtgct gcaatgatac 60
   cgcgagaccc acgctcaccg gctccagatt tatcagcaat aaaccagcca gccggaaggg 120
   ccgagcgcag aagtggtcct gcaactttat ccgcctccat ccagtctatt aattgttgcc 180
   gggaagctag agtaagtagt tcgccagtta atagtttgcg caacgttgtt gccattgcta 240
   caggcatcgt ggtgtcacgc tcgtcgtttg gtatggcttc attcagctcc ggttcccaac 300
   gatcaaggcg agttacatga tcccccatgt tgtgcaaaaa agcggttagc tccttcggtc 360
   ctccgatcgt tgtcagaagt aagttggccg cagtgttatc actcatggtt atggcagcac 420
   tgcataattc tcttactgtc atgccatccg taagatgctt ttctgtgact ggtgagtact 480
   caaccaagtc attctgagaa tagtgtatgc ggcgaccgag ttgctcttgc ccggcgtcaa 540
   tacgggataa taccgcgcca catagcagaa ctttaaaagt gctcatcatt ggaaaacgtt 600
   cttcggggcg aaaactctca aggatcttac cgctgttgag atccagttcg atgtaaccca 660
   ctcgtgcacc caactgatct tcagcatctt ttactttcac cagcgtttct gggtgagcaa 720
   aaacaggaag gcaaaatgcc gcaaaaaagg gaataagggc gacacggaaa tgttgaatac 780
   tcatactctt cctttttcaa tattattgaa gcatttatca gggttattgt ctcatgagcg 840
   gatacatatt tgaatgtatt tagaaaaata aacaaatagg ggttccgcgc acatttcccc 900
   gaaaagtgcc acctgacgtc taagaaacca ttattatcat gacattaacc tataaaaata 960
   ggcgtatcac gaggcccttt cgtctcgcgc gtttcggtga tgacggtgaa aacctctgac 1020
   acatgcagct cccggagacg gtcacagctt gtctgtaagc ggatgccggg agcagacaag 1080
   cccgtcaggg cgcgtcagcg ggtgttggcg ggtgtcgggg ctggcttaac tatgcggcat 1140
   cagagcagat tgtactgaga gtgcaccata gggcggccgc ggcgcgccgt tccggatctg 1200
   catcctgcga tgcagatccg gaacataatg gtgcagggcg ctgacttccg cgtttccaga 1260
   ctttacgaaa cacggaaacc gaagaccatt catgttgttg ctcaggtcgc agacgttttg 1320
   cagcagcagt cgcttcacgt tcgctcgcgt atcggtgatt cattctgcta accagtaagg 1380
   caaccccgcc agcctagccg ggtcctcaac gacaggagca cgatcatgcg cacccgtggc 1440
   caggacccaa cgctgcccga gatgcgccgc gtgcggctgc tggagatggc ggacgcgatg 1500
   gatatgttct gccaagggtt ggtttgcgca ttcacagttc tccgcaagaa ttgattggct 1560
   ccaattcttg gagtggtgaa tccgttagcg aggtgccgcc ggcttccatt caggtcgagg 1620
   tggcccggct ccatgcaccg cgacgcaacg cggggaggca gacaaggtat agggcggcgc 1680
   ctacaatcca tgccaacccg ttccatgtgc tcgccgaggc ggcataaatc gccgtgacga 1740
   tcagcggtcc aatgatcgaa gttaggctgg taagagccgc gagcgatcct tgaagctgtc 1800
   cctgatggtc gtcatctacc tgcctggaca gcatggcctg caacgcgggc atcccgatgc 1860
   cgccggaagc gagaagaatc ataatgggga aggccatcca gcctcgcgtc gcgaacgcca 1920
   gcaagacgta gcccagcgcg tcggccgcca tgccggcgat aatggcctgc ttctcgccga 1980
   aacgtttggt ggcgggacca gtgacgaagg cttgagcgag ggcgtgcaag attccgaata 2040
   ccgcaagcga caggccgatc atcgtcgcgc tccagcgaaa gcggtcctcg ccgaaaatga 2100
   cccagagcgc tgccggcacc tgtcctacga gttgcatgat aaagaagaca gtcataagtg 2160
   cggcgacgat agtcatgccc cgcgcccacc ggaaggagct gactgggttg aaggctctca 2220
   agggcatcgg tcgagcttga cattgtagga cgtttaaaca ttaccctgtt atccctagga 2280
   tcctacgtat acatactccg aaggaggaca aatttattta ttgtggtaca ataaataagt 2340
   ggtacaataa ataaattgta tgtaaacccc ttccccttcg ggacgtcccc ttacgggaat 2400
   ataaatatta gtggcagttg cctgccaaca aatttattta ttgtattaac ataggcagtg 2460
   gcggtaccac tgccactggc gtcctaatat aaatattggg caactaaagt ttatcgcagt 2520
   attaacatag gcagtggcgg taccactgcc actggcgtcc tccttcggag tatgtaaacc 2580
   tgctaccgca gcaaataaat tttattctat tttaatacta caatatttag attcccgtta 2640
   ggggataggc caggcaattg tcactggcgt catagtatat caatattgta acagattgac 2700
   accctttaag taaacatttt ttttaggatt catatgaaat taaatggata tttggtacat 2760
   ttaattccac aaaaatgtcc aatacttaaa atacaaaatt aaaagtatta gttgtaaact 2820
   tgactaacat tttaaatttt aaattttttc ctaattatat attttacttg caaaatttat 2880
   aaaaatttta tgcattttta tatcataata ataaaacctt tattcatggt ttataatata 2940
   ataattgtga tgactatgca caaagcagtt ctagtcccat atatataact atatataacc 3000
   cgtttaaaga tttatttaaa aatatgtgtg taaaaaatgc ttatttttaa ttttatttta 3060
   tataagttat aatattaaat acacaatgat taaaattaaa taataataaa tttaacgtaa 3120
   cgatgagttg tttttttatt ttggagatac acgcaatgac aattgcgatc ggtacatatc 3180
   aagagaaacg cacatggttc gatgacgctg atgactggct tcgtcaagac cgtttcgtat 3240
   tcgtaggttg gtcaggttta ttactattcc cttgtgctta ctttgcaact ccggtccggc 3300
   ggccgcctcg agacgacttg tccgcttcat cagacacggc tttcctaacc atcaatggtg 3360
   gattttcagg aaagacgttt aaagaagtgg cataaagttt atttgttgaa gaattggttt 3420
   tgtttccatt caaagaattg ttagggataa aactttgcat ttttttataa tttgttataa 3480
   gtttttcaaa cttatatgtt tttaaaaatg catttaattg cttattaatg cgttcatttt 3540
   gtaatgtttc aataggtctt gcttgcgcta atcgcagtat tcccgatact ttgtctgctt 3600
   gtttttcggg tattgagaat aagtaagtat aatgatttaa aaaagtcatg ttttgattaa 3660
   atctttttta tatggttaaa aacattatgg tatatctaaa taaatttatt ttttactaaa 3720
   tctccaattt gcaatttaga gatataatta aaactataaa gttatttaag ttaatttgta 3780
   atcaaatcca acacaaaaat gtttttatat agttaacatg ttaaatttaa catatgttaa 3840
   acaactaaaa ttctgtaaca gagaacaata aaataaatgc tagattttgt gtaatgccga 3900
   agtatattta tatacttccc tttcaaaaaa ataaatactc ttgccactaa aattcatttg 3960
   cctaggacgt ccccttcccc ttacgggatg tttatatact aggacgtccc cttcccctta 4020
   cgggatattt atatactccg aaggacgtcc ccttcgggca aataaatttt agtggcagtt 4080
   gcctgccaac tgcctaggca agtaaactta gggattttaa tgcaataaat aaatttgtcc 4140
   ccttacggga cgtcagtggc agttgcctgc caactgccta atataaatat tagtggatat 4200
   ttatatactc cgaaggaggc agttacctgc caactgccga ggcaaataaa ttttagtggc 4260
   agtggtaccg ccactgcctg ctccctcctt ccccttcggg caagtaaact tagcatgttg 4320
   tcgacattac cctgttatcc ctaggccggc ctaagaaacc attattatca tgacattaac 4380
   ctataaaaat aggcgtatca cgaggccctt tcgtcttcaa gaaattcggt cgaaaaaaga 4440
   aaaggagagg gccaagaggg agggcattgg tgactattga gcacgtgagt atacgtgatt 4500
   aagcacacaa aggcagcttg gagtatgtct gttattaatt tcacaggtag ttctggtcca 4560
   ttggtgaaag tttgcggctt gcagagcaca gaggccgcag aatgtgctct agattccgat 4620
   gctgacttgc tgggtattat atgtgtgccc aatagaaaga gaacaattga cccggttatt 4680
   gcaaggaaaa tttcaagtct tgtaaaagca tataaaaata gttcaggcac tccgaaatac 4740
   ttggttggcg tgtttcgtaa tcaacctaag gaggatgttt tggctctggt caatgattac 4800
   ggcattgata tcgtccaact gcatggagat gagtcgtggc aagaatacca agagttcctc 4860
   ggtttgccag ttattaaaag actcgtattt ccaaaagact gcaacatact actcagtgca 4920
   gcttcacaga aacctcattc gtttattccc ttgtttgatt cagaagcagg tgggacaggt 4980
   gaacttttgg attggaactc gatttctgac tgggttggaa ggcaagagag ccccgaaagc 5040
   ttacatttta tgttagctgg tggactgacg ccagaaaatg ttggtgatgc gcttagatta 5100
   aatggcgtta ttggtgttga tgtaagcgga ggtgtggaga caaatggtgt aaaagactct 5160
   aacaaaatag caaatttcgt caaaaatgct aagaaatagg ttattactga gtagtattta 5220
   tttaagtatt gtttgtgcac ttgcctgcag gccttttgaa aagcaagcat aaaagatcta 5280
   aacataaaat ctgtaaaata acaagatgta aagataatgc taaatcattt ggctttttga 5340
   ttgattgtac aggaaaatat acatcgttaa ttaagcggcc gcgagcttgg cgtaatcatg 5400
   gtcatagctg tttcctgtgt gaaattgtta tccgctcaca attccacaca acatacgagc 5460
   cggaagcata aagtgtaaag cctggggtgc ctaatgagtg agctaactca cattaattgc 5520
   gttgcgctca ctgcccgctt tccagtcggg aaacctgtcg tgccagctgc attaatgaat 5580
   cggccaacgc gcggggagag gcggtttgcg tattgggcgc tcttccgctt cctcgctcac 5640
   tgactcgctg cgctcggtcg ttcggctgcg gcgagcggta tcagctcact caaaggcggt 5700
   aatacggtta tccacagaat caggggataa cgcaggaaag aacatgtgag caaaaggcca 5760
   gcaaaaggcc aggaaccgta aaaaggccgc gttgctggcg tttttccata ggctccgccc 5820
   ccctgacgag catcacaaaa atcgacgctc aagtcagagg tggcgaaacc cgacaggact 5880
   ataaagatac caggcgtttc cccctggaag ctccctcgtg cgctctcctg ttccgaccct 5940
   gccgcttacc ggatacctgt ccgcctttct cccttcggga agcgtggcgc tttctcatag 6000
   ctcacgctgt aggtatctca gttcggtgta ggtcgttcgc tccaagctgg gctgtgtgca 6060
   cgaacccccc gttcagcccg accgctgcgc cttatccggt aactatcgtc ttgagtccaa 6120
   cccggtaaga cacgacttat cgccactggc agcagccact ggtaacagga ttagcagagc 6180
   gaggtatgta ggcggtgcta cagagttctt gaagtggtgg cctaactacg gctacactag 6240
   aagaacagta tttggtatct gcgctctgct gaagccagtt accttcggaa aaagagttgg 6300
   tagctcttga tccggcaaac aaaccaccgc tggtagcggt ggtttttttg tttgcaagca 6360
   gcagattacg cgcagaaaaa aaggatctca agaagatcct ttgatctttt ctacggggtc 6420
   tgacgctcag tggaacgaaa actcacgtta agggattttg gtcatgagat tatcaaaaag 6480
   gatcttcacc tagatccttt taaattaaaa atgaagtttt aaatcaatct aaagtatata 6540
   tgagtaaact tggtctgaca gttaccaatg cttaatcagt gaggcaccta tctcagcgat 6600
   ctgtctattt cgttcatcca tagttgcctg actccccg 6638
<210> 4
   <211> 7855
   <212> DNA
   <213> Artificial Sequence
<220>
   <223> Description of Artificial sequence: Synthetic polynucleotide
<400> 4
   gtgcactctc agtacaatct gctctgatgc cgcatagtta agccagcccc gacacccgcc 60
   aacacccgct gacgcgccct gacgggcttg tctgctcccg gcatccgctt acagacaagc 120
   tgtgaccgtc tccgggagct gcatgtgtca gaggttttca ccgtcatcac cgaaacgcgc 180
   gagacgaaag ggcctcgtga tacgcctatt tttataggtt aatgtcatga taataatggt 240
   ttcttagacg tcaggtggca cttttcgggg aaatgtgcgc ggaaccccta tttgtttatt 300
   tttctaaata cattcaaata tgtatccgct catgagacaa taaccctgat aaatgcttca 360
   ataatattga aaaaggaaga gtatgagtat tcaacatttc cgtgtcgccc ttattccctt 420
   ttttgcggca ttttgccttc ctgtttttgc tcacccagaa acgctggtga aagtaaaaga 480
   tgctgaagat cagttgggtg cacgagtggg ttacatcgaa ctggatctca acagcggtaa 540
   gatccttgag agttttcgcc ccgaagaacg ttttccaatg atgagcactt ttaaagttct 600
   gctatgtggc gcggtattat cccgtattga cgccgggcaa gagcaactcg gtcgccgcat 660
   acactattct cagaatgact tggttgagta ctcaccagtc acagaaaagc atcttacgga 720
   tggcatgaca gtaagagaat tatgcagtgc tgccataacc atgagtgata acactgcggc 780
   caacttactt ctgacaacga tcggaggacc gaaggagcta accgcttttt tgcacaacat 840
   gggggatcat gtaactcgcc ttgatcgttg ggaaccggag ctgaatgaag ccataccaaa 900
   cgacgagcgt gacaccacga tgcctgtagc aatggcaaca acgttgcgca aactattaac 960
   tggcgaacta cttactctag cttcccggca acaattaata gactggatgg aggcggataa 1020
   agttgcagga ccacttctgc gctcggccct tccggctggc tggtttattg ctgataaatc 1080
   tggagccggt gagcgtgggt ctcgcggtat cattgcagca ctggggccag atggtaagcc 1140
   ctcccgtatc gtagttatct acacgacggg gagtcaggca actatggatg aacgaaatag 1200
   acagatcgct gagataggtg cctcactgat taagcattgg taactgtcag accaagttta 1260
   ctcatatata ctttagattg atttaaaact tcatttttaa tttaaaagga tctaggtgaa 1320
   gatccttttt gataatctca tgaccaaaat cccttaacgt gagttttcgt tccactgagc 1380
   gtcagacccc gtagaaaaga tcaaaggatc ttcttgagat cctttttttc tgcgcgtaat 1440
   ctgctgcttg caaacaaaaa aaccaccgct accagcggtg gtttgtttgc cggatcaaga 1500
   gctaccaact ctttttccga aggtaactgg cttcagcaga gcgcagatac caaatactgt 1560
   tcttctagtg tagccgtagt taggccacca cttcaagaac tctgtagcac cgcctacata 1620
   cctcgctctg ctaatcctgt taccagtggc tgctgccagt ggcgataagt cgtgtcttac 1680
   cgggttggac tcaagacgat agttaccgga taaggcgcag cggtcgggct gaacgggggg 1740
   ttcgtgcaca cagcccagct tggagcgaac gacctacacc gaactgagat acctacagcg 1800
   tgagctatga gaaagcgcca cgcttcccga agggagaaag gcggacaggt atccggtaag 1860
   cggcagggtc ggaacaggag agcgcacgag ggagcttcca gggggaaacg cctggtatct 1920
   ttatagtcct gtcgggtttc gccacctctg acttgagcgt cgatttttgt gatgctcgtc 1980
   aggggggcgg agcctatgga aaaacgccag caacgcggcc tttttacggt tcctggcctt 2040
   ttgctggcct tttgctcaca tgttctttcc tgcgttatcc cctgattctg tggataaccg 2100
   tattaccgcc tttgagtgag ctgataccgc tcgccgcagc cgaacgaccg agcgcagcga 2160
   gtcagtgagc gaggaagcgg aagagcgccc aatacgcaaa ccgcctctcc ccgcgcgttg 2220
   gccgattcat taatgcagct ggcacgacag gtttcccgac tggaaagcgg gcagtgagcg 2280
   caacgcaatt aatgtgagtt agctcactca ttaggcaccc caggctttac actttatgct 2340
   tccggctcgt atgttgtgtg gaattgtgag cggataacaa tttcacacag gaaacagcta 2400
   tgaccatgat tacgccaagc tcgcggccgc agtactctgc agattttatg caaaattaaa 2460
   gtcttgtgac aacagctttc tccttaagtg caaatatcgc ccattctttc ctcttttcgt 2520
   atataaatgc tgtaatagta ggatgtcgta cccgtaaagg tacgacattg aatattaata 2580
   tactcctaag tttactttcc caatatttat attaggacgt ccccttcggg taaataaatt 2640
   ttagtggcag tggtaccgcc actccctatt ttaatactgc gaaggaggca gttggcaggc 2700
   aactcgtcgt tcgcagtata taaatatcca ctaatattta tattcccgta aggggacgtc 2760
   ccgaagggga aggggaaaga agcagtcgcc tccttgcgaa aaggtttact tgcccgacca 2820
   gtgaaaagca tgctgtaaga tataaatcta ccctgaaagg gatgcatttc accataatac 2880
   tatacaaatg gtgttaccct ttgaggatca taacggtgct actggaatat atggtctctt 2940
   catggataga cgatagccat ttatttaccc attaagggga cattagtggc ctgtcactgc 3000
   tccttacgag acgccagtgg acgttcgtcc tagaaaattt atgcgctgcc tagaagcccc 3060
   aaaagggaag tttactgact cgttagagcg tgcgctaaca ggtttaaata cttcaatatg 3120
   tatattagga cgccggtggc agtggtaccg ccactgccac cgtcggagga cgtcccttac 3180
   ggtatattat atactaggat tttaatactc cgaaggaggc agtggcggta ccactgccac 3240
   taatatttat attcccgtaa gggacgtcct ccttcggagt atgtaaacat tctaagttta 3300
   cttgcccaat atttatatta ggcagttggc aggcaactgc tagctctcct ccttcggagt 3360
   atgtaaacat cgcagtatat aaatatccac taatatttat attcccgtaa ggggacgtcc 3420
   cgaaggggaa ggggaaggac gtcagtggca gttgcctgcc aactgcctag gcaagtaaac 3480
   ttaggagtat ataaatatag gcagtcgcgg taccactgcc actgacgtcc tgccaactgc 3540
   ctaggcaagt aaacttaagt ggcactaaaa tgcatttgcc cgaaggggaa ggaggacgcc 3600
   agtggcagtg gtaccgccac tgcctccttc ggagtattaa aatcctagta tgtaaatctg 3660
   ctagcgcagg aaataaattt tattctattt atatactccg ttaggaggta agtaaacccc 3720
   ttccccttcg ggacgtcagt gcagttgcct gccaactgcc taatataaat attagaccac 3780
   taaagtttgg caactgccaa ctgttgtcct tcggaggaaa aaaaatggtt aactcgcaag 3840
   cagttaacat aactaaagtt tgttacttta ccgaagacgt ttaccctttc tcggttaagg 3900
   agacggagac agttgcactg tgactgccta gtatagcaat tttgtttttg tttatatgct 3960
   cgacaaaatg actttcataa aaatataaag tagttagcta gttattttat atcactataa 4020
   ctagggttct cagaggcacc gaagtcactt gtaaaaatag tactttttaa cttgtttaat 4080
   cttcgtgttc ttcaaaagga tcacgtaatt tttttgaagg tggaccaaaa ctaacataaa 4140
   ctgaatagcc agttacactt aacagaagaa accataaaaa aaaggtaaag aaaaaagctg 4200
   gactttccat agctcattta ataataaaat tattctcttt tcaacatatc tcttagatag 4260
   ttcaaaagac ttgacgactg tgtcccacat ttttaaacaa aattaatcta ctcaaaattt 4320
   tgccctgaga aagaataact tacttcgttt ttgcagtagc cattcatgtc actttgaaac 4380
   tgtccttaca aagttaaaca ttaattaaaa attatttaat ttttatataa caaatattat 4440
   attaaataaa aaatgaacaa agaacttcta agatcgtctt tagtgagtaa ttaaagagtt 4500
   ttacttacca gacaaggcag ttttttcatt cttttaaagc aggcagttct gaaggggaaa 4560
   agggactgcc tactgcggtc ctaggtaaat acatttttat gcaatttatt tcttgtgcta 4620
   gtaggtttct atactcacaa gaagcaaccc cttgacgaga gaacgttatc ctcagagtat 4680
   ttataatcct gagagggaat gcactgaaga atattttcct tattttttac agaaagtaaa 4740
   taaaatagcg ctaataacgc ttaattcatt taatcaatta tggcaacagg aacttctaaa 4800
   gctaaaccat caaaagtaaa ttcagacttc caagaacctg gtttagttac accattaggt 4860
   actttattac gtccacttaa ctcagaagca ggtaaagtat taccaggctg gggtacaact 4920
   gttttaatgg ctgtatttat ccttttattt gcagcattct tattaatcat tttagaaatt 4980
   tacaacagtt ctttaatttt agatgacgtt tctatgagtt gggaaacttt agctaaagtt 5040
   tcttaatttt atttaacaca aacataaaat ataaaactgt ttgttaaggc tagctgctaa 5100
   gtcttctttt cgctaaggta aactaagcaa ctcaaccata tttatattcg gcagtggcac 5160
   cgccaactgc cactggcctt ccgttaagat aaacgcgtgg atctcacgtg actagtgata 5220
   tctacgtaat cgatgaattc gatcccattt ttataactgg atctcaaaat acctataaac 5280
   ccattgttct tctcttttag ctctaagaac aatcaattta taaatatatt tattattatg 5340
   ctataatata aatactatat aaatacattt acctttttat aaatacattt accttttttt 5400
   taatttgcat gattttaatg cttatgctat cttttttatt tagtccataa aacctttaaa 5460
   ggaccttttc ttatgggata tttatatttt cctaacaaag caatcggcgt cataaacttt 5520
   agttgcttac gacgcctgtg gacgtccccc ccttcccctt acgggcaagt aaacttaggg 5580
   attttaatgc aataaataaa tttgtcctct tcgggcaaat gaattttagt atttaaatat 5640
   gacaagggtg aaccattact tttgttaaca agtgatctta ccactcacta tttttgttga 5700
   attttaaact tatttaaaat tctcgagaaa gattttaaaa ataaactttt ttaatctttt 5760
   atttattttt tcttttttcg tatggaattg cccaatatta ttcaacaatt tatcggaaac 5820
   agcgttttag agccaaataa aattggtcag tcgccatcgg atgtttattc ttttaatcga 5880
   aataatgaaa ctttttttct taagcgatct agcactttat atacagagac cacatacagt 5940
   gtctctcgtg aagcgaaaat gttgagttgg ctctctgaga aattaaaggt gcctgaactc 6000
   atcatgactt ttcaggatga gcagtttgaa tttatgatca ctaaagcgat caatgcaaaa 6060
   ccaatttcag cgcttttttt aacagaccaa gaattgcttg ctatctataa ggaggcactc 6120
   aatctgttaa attcaattgc tattattgat tgtccattta tttcaaacat tgatcatcgg 6180
   ttaaaagagt caaaattttt tattgataac caactccttg acgatataga tcaagatgat 6240
   tttgacactg aattatgggg agaccataaa acttacctaa gtctatggaa tgagttaacc 6300
   gagactcgtg ttgaagaaag attggttttt tctcatggcg atatcacgga tagtaatatt 6360
   tttatagata aattcaatga aatttatttt ttagaccttg gtcgtgctgg gttagcagat 6420
   gaatttgtag atatatcctt tgttgaacgt tgcctaagag aggatgcatc ggaggaaact 6480
   gcgaaaatat ttttaaagca tttaaaaaat gatagacctg acaaaaggaa ttatttttta 6540
   aaacttgatg aattgaattg attccaagca ttatctaaaa tactctgcag gcacgctagc 6600
   ttgtactcaa gctcgtaacg aaggtcgtga ccttgctcgt gaaggtggcg acgtaattcg 6660
   ttcagcttgt aaatggtctc cagaacttgc tgctgcatgt gaagtttgga aagaaattaa 6720
   attcgaattt gatactattg acaaacttta atttttattt ttcatgatgt ttatgtgaat 6780
   agcataaaca tcgtttttat ttttatggtg tttaggttaa atacctaaac atcattttac 6840
   atttttaaaa ttaagttcta aagttatctt ttgtttaaat ttgcctgtct ttataaatta 6900
   cgatgtgcca gaaaaataaa atcttagctt tttattatag aatttatctt tatgtattat 6960
   attttataag ttataataaa agaaatagta acatactaaa gcggatgtag cgcgtttatc 7020
   ttaacggaag gaattcggcg cctacgtacc cgggtcgcga ggatccacgc gttaatagct 7080
   cacttttctt taaatttaat ttttaattta aaggtgtaag caaattgcct gacgagagat 7140
   ccacttaaag gatgacagtg gcgggctact gcctacttcc ctccgggata aaatttattt 7200
   gaaaaacgtt agttacttcc taacggagca ttgacatccc catatttata ttaggacgtc 7260
   cccttcgggt aaataaattt tagtggacgt ccccttcggg caaataaatt ttagtggaca 7320
   ataaataaat ttgttgcctg ccaactgcct aggcaagtaa acttgggagt attaaaatag 7380
   gacgtcagtg gcagttgcct gccaactgcc tatatttata tactgcgaag caggcagtgg 7440
   cggtaccact gccactggcg tcctaatata aatattgggc aactaaagtt tatagcagta 7500
   ttaacatcct atatttatat actccgaagg aacttgttag ccgataggcg aggcaacaaa 7560
   tttatttatt gtcccgtaaa aggatgcctc cagcatcgaa ggggaagggg acgtcctagg 7620
   ccataaaact aaagggaaat ccatagtaac tgatgttata aatttataga ctccaaaaaa 7680
   cagctgcgtt ataaataact tctgttaaat atggccaagg ggacaggggc actttcaact 7740
   aagtgtacat taaaaattga caattcaatt ttttttaatt ataatatata tttagtaaaa 7800
   tataacaaaa agcccccatc gtctaggtag aattccagct ggcggccgcc ctatg 7855
<210> 5
   <211> 8458
   <212> DNA
   <213> Artificial Sequence
<220>
   <223> Description of Artificial Sequence: Synthetic polynucleotide
<400> 5
   tcgtgtagat aactacgata cgggagggct taccatctgg ccccagtgct gcaatgatac 60
   cgcgagaccc acgctcaccg gctccagatt tatcagcaat aaaccagcca gccggaaggg 120
   ccgagcgcag aagtggtcct gcaactttat ccgcctccat ccagtctatt aattgttgcc 180
   gggaagctag agtaagtagt tcgccagtta atagtttgcg caacgttgtt gccattgcta 240
   caggcatcgt ggtgtcacgc tcgtcgtttg gtatggcttc attcagctcc ggttcccaac 300
   gatcaaggcg agttacatga tcccccatgt tgtgcaaaaa agcggttagc tccttcggtc 360
   ctccgatcgt tgtcagaagt aagttggccg cagtgttatc actcatggtt atggcagcac 420
   tgcataattc tcttactgtc atgccatccg taagatgctt ttctgtgact ggtgagtact 480
   caaccaagtc attctgagaa tagtgtatgc ggcgaccgag ttgctcttgc ccggcgtcaa 540
   tacgggataa taccgcgcca catagcagaa ctttaaaagt gctcatcatt ggaaaacgtt 600
   cttcggggcg aaaactctca aggatcttac cgctgttgag atccagttcg atgtaaccca 660
   ctcgtgcacc caactgatct tcagcatctt ttactttcac cagcgtttct gggtgagcaa 720
   aaacaggaag gcaaaatgcc gcaaaaaagg gaataagggc gacacggaaa tgttgaatac 780
   tcatactctt cctttttcaa tattattgaa gcatttatca gggttattgt ctcatgagcg 840
   gatacatatt tgaatgtatt tagaaaaata aacaaatagg ggttccgcgc acatttcccc 900
   gaaaagtgcc acctgacgtc taagaaacca ttattatcat gacattaacc tataaaaata 960
   ggcgtatcac gaggcccttt cgtctcgcgc gtttcggtga tgacggtgaa aacctctgac 1020
   acatgcagct cccggagacg gtcacagctt gtctgtaagc ggatgccggg agcagacaag 1080
   cccgtcaggg cgcgtcagcg ggtgttggcg ggtgtcgggg ctggcttaac tatgcggcat 1140
   cagagcagat tgtactgaga gtgcaccata gggcggccgc ggcgcgccgt tccggatctg 1200
   catcctgcga tgcagatccg gaacataatg gtgcagggcg ctgacttccg cgtttccaga 1260
   ctttacgaaa cacggaaacc gaagaccatt catgttgttg ctcaggtcgc agacgttttg 1320
   cagcagcagt cgcttcacgt tcgctcgcgt atcggtgatt cattctgcta accagtaagg 1380
   caaccccgcc agcctagccg ggtcctcaac gacaggagca cgatcatgcg cacccgtggc 1440
   caggacccaa cgctgcccga gatgcgccgc gtgcggctgc tggagatggc ggacgcgatg 1500
   gatatgttct gccaagggtt ggtttgcgca ttcacagttc tccgcaagaa ttgattggct 1560
   ccaattcttg gagtggtgaa tccgttagcg aggtgccgcc ggcttccatt caggtcgagg 1620
   tggcccggct ccatgcaccg cgacgcaacg cggggaggca gacaaggtat agggcggcgc 1680
   ctacaatcca tgccaacccg ttccatgtgc tcgccgaggc ggcataaatc gccgtgacga 1740
   tcagcggtcc aatgatcgaa gttaggctgg taagagccgc gagcgatcct tgaagctgtc 1800
   cctgatggtc gtcatctacc tgcctggaca gcatggcctg caacgcgggc atcccgatgc 1860
   cgccggaagc gagaagaatc ataatgggga aggccatcca gcctcgcgtc gcgaacgcca 1920
   gcaagacgta gcccagcgcg tcggccgcca tgccggcgat aatggcctgc ttctcgccga 1980
   aacgtttggt ggcgggacca gtgacgaagg cttgagcgag ggcgtgcaag attccgaata 2040
   ccgcaagcga caggccgatc atcgtcgcgc tccagcgaaa gcggtcctcg ccgaaaatga 2100
   cccagagcgc tgccggcacc tgtcctacga gttgcatgat aaagaagaca gtcataagtg 2160
   cggcgacgat agtcatgccc cgcgcccacc ggaaggagct gactgggttg aaggctctca 2220
   agggcatcgg tcgagcttga cattgtagga cgtttaaaca ttaccctgtt atccctagga 2280
   tcctacgtaa tcgatgaatt cgatcccatt tttataactg gatctcaaaa tacctataaa 2340
   cccattgttc ttctctttta gctctaagaa caatcaattt ataaatatat ttattattat 2400
   gctataatat aaatactata taaatacatt taccttttta taaatacatt tacctttttt 2460
   ttaatttgca tgattttaat gcttatgcta tcttttttat ttagtccata aaacctttaa 2520
   aggacctttt cttatgggat atttatattt tcctaacaaa gcaatcggcg tcataaactt 2580
   tagttgctta cgacgcctgt ggacgtcccc cccttcccct tacgggcaag taaacttagg 2640
   gattttaatg caataaataa atttgtcctc ttcgggcaaa tgaattttag tatttaaata 2700
   tgacaagggt gaaccattac ttttgttaac aagtgatctt accactcact atttttgttg 2760
   aattttaaac ttatttaaaa ttctcgagaa agattttaaa aataaacttt tttaatcttt 2820
   tatttatttt ttcttttttc gtatggaatt gcccaatatt attcaacaat ttatcggaaa 2880
   cagcgtttta gagccaaata aaattggtca gtcgccatcg gatgtttatt cttttaatcg 2940
   aaataatgaa actttttttc ttaagcgatc tagcacttta tatacagaga ccacatacag 3000
   tgtctctcgt gaagcgaaaa tgttgagttg gctctctgag aaattaaagg tgcctgaact 3060
   catcatgact tttcaggatg agcagtttga atttatgatc actaaagcga tcaatgcaaa 3120
   accaatttca gcgctttttt taacagacca agaattgctt gctatctata aggaggcact 3180
   caatctgtta aattcaattg ctattattga ttgtccattt atttcaaaca ttgatcatcg 3240
   gttaaaagag tcaaaatttt ttattgataa ccaactcctt gacgatatag atcaagatga 3300
   ttttgacact gaattatggg gagaccataa aacttaccta agtctatgga atgagttaac 3360
   cgagactcgt gttgaagaaa gattggtttt ttctcatggc gatatcacgg atagtaatat 3420
   ttttatagat aaattcaatg aaatttattt tttagacctt ggtcgtgctg ggttagcaga 3480
   tgaatttgta gatatatcct ttgttgaacg ttgcctaaga gaggatgcat cggaggaaac 3540
   tgcgaaaata tttttaaagc atttaaaaaa tgatagacct gacaaaagga attatttttt 3600
   aaaacttgat gaattgaatt gattccaagc attatctaaa atactctgca ggcacgctag 3660
   cttgtactca agctcgtaac gaaggtcgtg accttgctcg tgaaggtggc gacgtaattc 3720
   gttcagcttg taaatggtct ccagaacttg ctgctgcatg tgaagtttgg aaagaaatta 3780
   aattcgaatt tgatactatt gacaaacttt aatttttatt tttcatgatg tttatgtgaa 3840
   tagcataaac atcgttttta tttttatggt gtttaggtta aatacctaaa catcatttta 3900
   catttttaaa attaagttct aaagttatct tttgtttaaa tttgcctgtc tttataaatt 3960
   acgatgtgcc agaaaaataa aatcttagct ttttattata gaatttatct ttatgtatta 4020
   tattttataa gttataataa aagaaatagt aacatactaa agcggatgta gcgcgtttat 4080
   cttaacggaa ggaattcggc gcctacgtat acatactccg aaggaggaca aatttattta 4140
   ttgtggtaca ataaataagt ggtacaataa ataaattgta tgtaaacccc ttccccttcg 4200
   ggacgtcccc ttacgggaat ataaatatta gtggcagttg cctgccaaca aatttattta 4260
   ttgtattaac ataggcagtg gcggtaccac tgccactggc gtcctaatat aaatattggg 4320
   caactaaagt ttatcgcagt attaacatag gcagtggcgg taccactgcc actggcgtcc 4380
   tccttcggag tatgtaaacc tgctaccgca gcaaataaat tttattctat tttaatacta 4440
   caatatttag attcccgtta ggggataggc caggcaattg tcactggcgt catagtatat 4500
   caatattgta acagattgac accctttaag taaacatttt ttttaggatt catatgaaat 4560
   taaatggata tttggtacat ttaattccac aaaaatgtcc aatacttaaa atacaaaatt 4620
   aaaagtatta gttgtaaact tgactaacat tttaaatttt aaattttttc ctaattatat 4680
   attttacttg caaaatttat aaaaatttta tgcattttta tatcataata ataaaacctt 4740
   tattcatggt ttataatata ataattgtga tgactatgca caaagcagtt ctagtcccat 4800
   atatataact atatataacc cgtttaaaga tttatttaaa aatatgtgtg taaaaaatgc 4860
   ttatttttaa ttttatttta tataagttat aatattaaat acacaatgat taaaattaaa 4920
   taataataaa tttaacgtaa cgatgagttg tttttttatt ttggagatac acgcaatgac 4980
   aattgcgatc ggtacatatc aagagaaacg cacatggttc gatgacgctg atgactggct 5040
   tcgtcaagac cgtttcgtat tcgtaggttg gtcaggttta ttactattcc cttgtgctta 5100
   ctttgcaact ccggtccggc ggccgcctcg agacgacttg tccgcttcat cagacacggc 5160
   tttcctaacc atcaatggtg gattttcagg aaagacgttt aaagaagtgg cataaagttt 5220
   atttgttgaa gaattggttt tgtttccatt caaagaattg ttagggataa aactttgcat 5280
   ttttttataa tttgttataa gtttttcaaa cttatatgtt tttaaaaatg catttaattg 5340
   cttattaatg cgttcatttt gtaatgtttc aataggtctt gcttgcgcta atcgcagtat 5400
   tcccgatact ttgtctgctt gtttttcggg tattgagaat aagtaagtat aatgatttaa 5460
   aaaagtcatg ttttgattaa atctttttta tatggttaaa aacattatgg tatatctaaa 5520
   taaatttatt ttttactaaa tctccaattt gcaatttaga gatataatta aaactataaa 5580
   gttatttaag ttaatttgta atcaaatcca acacaaaaat gtttttatat agttaacatg 5640
   ttaaatttaa catatgttaa acaactaaaa ttctgtaaca gagaacaata aaataaatgc 5700
   tagattttgt gtaatgccga agtatattta tatacttccc tttcaaaaaa ataaatactc 5760
   ttgccactaa aattcatttg cctaggacgt ccccttcccc ttacgggatg tttatatact 5820
   aggacgtccc cttcccctta cgggatattt atatactccg aaggacgtcc ccttcgggca 5880
   aataaatttt agtggcagtt gcctgccaac tgcctaggca agtaaactta gggattttaa 5940
   tgcaataaat aaatttgtcc ccttacggga cgtcagtggc agttgcctgc caactgccta 6000
   atataaatat tagtggatat ttatatactc cgaaggaggc agttacctgc caactgccga 6060
   ggcaaataaa ttttagtggc agtggtaccg ccactgcctg ctccctcctt ccccttcggg 6120
   caagtaaact tagcatgttg tcgacattac cctgttatcc ctaggccggc ctaagaaacc 6180
   attattatca tgacattaac ctataaaaat aggcgtatca cgaggccctt tcgtcttcaa 6240
   gaaattcggt cgaaaaaaga aaaggagagg gccaagaggg agggcattgg tgactattga 6300
   gcacgtgagt atacgtgatt aagcacacaa aggcagcttg gagtatgtct gttattaatt 6360
   tcacaggtag ttctggtcca ttggtgaaag tttgcggctt gcagagcaca gaggccgcag 6420
   aatgtgctct agattccgat gctgacttgc tgggtattat atgtgtgccc aatagaaaga 6480
   gaacaattga cccggttatt gcaaggaaaa tttcaagtct tgtaaaagca tataaaaata 6540
   gttcaggcac tccgaaatac ttggttggcg tgtttcgtaa tcaacctaag gaggatgttt 6600
   tggctctggt caatgattac ggcattgata tcgtccaact gcatggagat gagtcgtggc 6660
   aagaatacca agagttcctc ggtttgccag ttattaaaag actcgtattt ccaaaagact 6720
   gcaacatact actcagtgca gcttcacaga aacctcattc gtttattccc ttgtttgatt 6780
   cagaagcagg tgggacaggt gaacttttgg attggaactc gatttctgac tgggttggaa 6840
   ggcaagagag ccccgaaagc ttacatttta tgttagctgg tggactgacg ccagaaaatg 6900
   ttggtgatgc gcttagatta aatggcgtta ttggtgttga tgtaagcgga ggtgtggaga 6960
   caaatggtgt aaaagactct aacaaaatag caaatttcgt caaaaatgct aagaaatagg 7020
   ttattactga gtagtattta tttaagtatt gtttgtgcac ttgcctgcag gccttttgaa 7080
   aagcaagcat aaaagatcta aacataaaat ctgtaaaata acaagatgta aagataatgc 7140
   taaatcattt ggctttttga ttgattgtac aggaaaatat acatcgttaa ttaagcggcc 7200
   gcgagcttgg cgtaatcatg gtcatagctg tttcctgtgt gaaattgtta tccgctcaca 7260
   attccacaca acatacgagc cggaagcata aagtgtaaag cctggggtgc ctaatgagtg 7320
   agctaactca cattaattgc gttgcgctca ctgcccgctt tccagtcggg aaacctgtcg 7380
   tgccagctgc attaatgaat cggccaacgc gcggggagag gcggtttgcg tattgggcgc 7440
   tcttccgctt cctcgctcac tgactcgctg cgctcggtcg ttcggctgcg gcgagcggta 7500
   tcagctcact caaaggcggt aatacggtta tccacagaat caggggataa cgcaggaaag 7560
   aacatgtgag caaaaggcca gcaaaaggcc aggaaccgta aaaaggccgc gttgctggcg 7620
   tttttccata ggctccgccc ccctgacgag catcacaaaa atcgacgctc aagtcagagg 7680
   tggcgaaacc cgacaggact ataaagatac caggcgtttc cccctggaag ctccctcgtg 7740
   cgctctcctg ttccgaccct gccgcttacc ggatacctgt ccgcctttct cccttcggga 7800
   agcgtggcgc tttctcatag ctcacgctgt aggtatctca gttcggtgta ggtcgttcgc 7860
   tccaagctgg gctgtgtgca cgaacccccc gttcagcccg accgctgcgc cttatccggt 7920
   aactatcgtc ttgagtccaa cccggtaaga cacgacttat cgccactggc agcagccact 7980
   ggtaacagga ttagcagagc gaggtatgta ggcggtgcta cagagttctt gaagtggtgg 8040
   cctaactacg gctacactag aagaacagta tttggtatct gcgctctgct gaagccagtt 8100
   accttcggaa aaagagttgg tagctcttga tccggcaaac aaaccaccgc tggtagcggt 8160
   ggtttttttg tttgcaagca gcagattacg cgcagaaaaa aaggatctca agaagatcct 8220
   ttgatctttt ctacggggtc tgacgctcag tggaacgaaa actcacgtta agggattttg 8280
   gtcatgagat tatcaaaaag gatcttcacc tagatccttt taaattaaaa atgaagtttt 8340
   aaatcaatct aaagtatata tgagtaaact tggtctgaca gttaccaatg cttaatcagt 8400
   gaggcaccta tctcagcgat ctgtctattt cgttcatcca tagttgcctg actccccg 8458
<210> 6
   <211> 22908
   <212> DNA
   <213> Artificial Sequence
<220>
   <223> Description of Artificial sequence: Synthetic polynucleotide
<400> 6
   tgaatttatc agatctaact gaggagtaag aaacccccat gtcaaagaaa aacagaccaa 60
   caattgggcg aacccttaat ccttcaatat taagcggatt tgatagttct tcagcctctg 120
   gcgatcgagt cgagcaggta ttcaagttat caactggtcg ccaggccaca tttattgaag 180
   aggtaatacc tccgaaccag gtagaaagcg atacctttgt tgatcagcat aacaacgggc 240
   gtgaccaggc atctcttacg ccaaaatcat taaaaagtat ccgaagcact attaagcatc 300
   agcaatttta ccctgcaata ggtgttagac gggctacagg gaaaattgaa attttggatg 360
   gttcccggcg tcgagcttct gccatcttag agaacgtagg gttgcgggtt ttagtcacgg 420
   accaggagat cagcgttcag gaagcgcaaa atttagcgaa agacgttcag acagcattgc 480
   agcacagcat tcgagaaata ggtctgcgtt tgatgcgaat gaaaaatgat gggatgagtc 540
   agaaggatat tgcagccaaa gaagggctgt ctcaggcgaa ggtcacgcgt gctctccagg 600
   cagcgagtgc tccggaagaa ttagtcgccc ttttccctgt gcagtcggaa ttaacctttt 660
   cggactacaa aacgctttgt gctgttggcg acgaaatggg gaacaagaat ttagagtttg 720
   atcagcttat tcaaaacata tccccggaaa taaacgacat cttatccatt gaagaaatgg 780
   ccgaagatga agttaaaaat aaaatcctgc gcttgataac aaaggaagcc tcactactca 840
   cggataaagg ttctaaagat aagtccgtag ttactgaatt atggaaattt gaggacaagg 900
   atcgctttgc aaggaagcgc gtgaaaggcc gtgcattttc ttatgagttt aatcgactct 960
   caaaagagtt acaggaagaa ctcgacagga tgattgggca tatccttaga aagagcctcg 1020
   ataaaaagcc gaagccttaa actttcgcca ttcaaatttc actattaact gactgttttt 1080
   aaagtaaatt actctaaaat ttcaaggtga aatcgccacg atttcacctt ggattttacc 1140
   ttcctcccct cctcccgaaa aaaataaaaa aattgcttgt cacgagaaag tcaacaagtg 1200
   actttcaata aaatctcttc cgaaaaggga ttcacacaag tgccttgtgt ttaaggaaga 1260
   gtaaattgag taacttacgc gaataccaga atcgtattgc agatatcgca aaacgctcta 1320
   aagctgtgct tggctgggca agcactgcgc agttcggtac tgataaccaa ttcattaaag 1380
   atgatgccgc gcgtgccgca tctatccttg aagctgcacg taaagacccg gtttttgcgg 1440
   gtatctctga taatgccacc gctcaaatcg ctacagcgtg ggcaagtgca ctggctgact 1500
   acgccgcagc acataaatct atgccgcgtc cggaaattct ggcctcctgc caccagacgc 1560
   tggaaaactg cctgatagag tccacccgca atagcatgga tgccactaat aaagcgatgc 1620
   tggaatctgt cgcagcagag atgatgagcg tttctgacgg tgttatgcgt ctgcctttat 1680
   tcctcgcgat gatcctgcct gttcagttgg gggcagctac cgctgatgcg tgtaccttca 1740
   ttccggttac gcgtgaccag tccgacatct atgaagtctt taacgtggca ggttcatctt 1800
   ttggttctta tgctgctggt gatgttctgg acatgcaatc cgtcggtgtg tacagccagt 1860
   tacgtcgccg ctatgtgctg gtggcaagct ccgatggcac cagcaaaacc gcaaccttca 1920
   agatggaaga cttcgaaggc cagaatgtac caatccgaaa aggtcgcact aacatctacg 1980
   ttaaccgtat taagtctgtt gttgataacg gttccggcag cctacttcac tcgtttacta 2040
   atgctgctgg tgagcaaatc actgttacct gctctctgaa ctacaacatt ggtcagattg 2100
   ccctgtcgtt ctccaaagcg ccggataaag gcactgagat cgcaattgag acggaaatca 2160
   atattgaagc cgctcctgag ctgatcccgc tgatcaacca cgaaatgaag aaatacaccc 2220
   tgttcccaag tcagttcgtt atcgcggctg agcacacggt acaggcggcg tatgaagcac 2280
   agcgtgaatt tggtctggac ctgggttccc tacagttccg caccctgaag gaatacctgt 2340
   ctcatgaaca ggatatgctg cgtcttcgca tcatgatctg gcgcactctt gcgaccgaca 2400
   cctttgacat cgctctgccg gttaaccagt cctttgatgt atgggcaacc atcattcgtg 2460
   gcaaattcca gactgtatat cgcgacatta ttgagcgcgt taaatcttct ggtgcgatgg 2520
   ggatgtttgc tggtgctgat gcagcatctt tcttcaaaca gttgccgaag gatttcttcc 2580
   agccagccga agactatatc cagactccgt atgttcacta catcggtacc ccatttagga 2640
   ccacccacag cacctaacaa aacggcatca gccttcttgg aggcttccag cgcctcatct 2700
   ggaagtggaa cacctgtagc atcgacctgc aggggggggg gggcgctgag gtctgcctcg 2760
   tgaagaaggt gttgctgact cataccaggc ctgaatcgcc ccatcatcca gccagaaagt 2820
   gagggagcca cggttgatga gagctttgtt gtaggtggac cagttggtga ttttgaactt 2880
   ttgctttgcc acggaacggt ctgcgttgtc gggaagatgc gtgatctgat ccttcaactc 2940
   agcaaaagtt cgatttattc aacaaagccg ccgtcccgtc aagtcagcgt aatgctctgc 3000
   cagtgttaca accaattaac caattctgat tagaaaaact catcgagcat caaatgaaac 3060
   tgcaatttat tcatatcagg attatcaata ccatattttt gaaaaagccg tttctgtaat 3120
   gaaggagaaa actcaccgag gcagttccat aggatggcaa gatcctggta tcggtctgcg 3180
   attccgactc gtccaacatc aatacaacct attaatttcc cctcgtcaaa aataaggtta 3240
   tcaagtgaga aatcaccatg agtgacgact gaatccggtg agaatggcaa aagcttatgc 3300
   atttctttcc agacttgttc aacaggccag ccattacgct cgtcatcaaa atcactcgca 3360
   tcaaccaaac cgttattcat tcgtgattgc gcctgagcga gacgaaatac gcgatcgctg 3420
   ttaaaaggac aattacaaac aggaatcgaa tgcaaccggc gcaggaacac tgccagcgca 3480
   tcaacaatat tttcacctga atcaggatat tcttctaata cctggaatgc tgttttcccg 3540
   gggatcgcag tggtgagtaa ccatgcatca tcaggagtac ggataaaatg cttgatggtc 3600
   ggaagaggca taaattccgt cagccagttt agtctgacca tctcatctgt aacatcattg 3660
   gcaacgctac ctttgccatg tttcagaaac aactctggcg catcgggctt cccatacaat 3720
   cgatagattg tcgcacctga ttgcccgaca ttatcgcgag cccatttata cccatataaa 3780
   tcagcatcca tgttggaatt taatcgcggc ctcgagcaag acgtttcccg ttgaatatgg 3840
   ctcataacac cccttgtatt actgtttatg taagcagaca gttttattgt tcatgatgat 3900
   atatttttat cttgtgcaat gtaacatcag agattttgag acacaacgtg gctttccccc 3960
   ccccccctgc aggtcgatag cagcaccacc aattaaatga ttttcgaaat cgaacttgac 4020
   attggaacga acatcagaaa tagctttaag aaccttaatg gcttcggctg tgatttcttg 4080
   accaacgtgg tcacctggca aaacgacgat cttcttaggg gcagacatta gaatggtata 4140
   tccttgaaat atatatatat attgctgaaa tgtaaaaggt aagaaaagtt agaaagtaag 4200
   acgattgcta accacctatt ggaaaaaaca ataggtcctt aaataatatt gtcaacttca 4260
   agtattgtga tgcaagcatt tagtcatgaa cgcttctcta ttctatatga aaagccggtt 4320
   ccggcgctct cacctttcct ttttctccca atttttcagt tgaaaaaggt atatgcgtca 4380
   ggcgacctct gaaattaaca aaaaatttcc agtcatcgaa tttgattctg tgcgatagcg 4440
   cccctgtgtg ttctcgttat gttgaggaaa aaaataatgg ttgctaagag attcgaactc 4500
   ttgcatctta cgatacctga gtattcccac agttaactgc ggtcaagata tttcttgaat 4560
   caggcgcctt agaccgctcg gccaaacaac caattacttg ttgagaaata gagtataatt 4620
   atcctataaa tataacgttt ttgaacacac atgaacaagg aagtacagga caattgattt 4680
   tgaagagaat gtggattttg atgtaattgt tgggattcca tttttaataa ggcaataata 4740
   ttaggtatgt agatatacta gaagttctcc tcgacgctct cccttatgcg actcctgcat 4800
   taggaagcag cccagtagta ggttgaggcc gttgagcacc gccgccgcaa ggaatggtgc 4860
   atgcaaggag atggcgccca acagtccccc ggccacgggg cctgccacca tacccacgcc 4920
   gaaacaagcg ctcatgagcc cgaagtggcg agcccgatct tccccatcgg tgatgtcggc 4980
   gatataggcg ccagcaaccg cacctgtggc gccggtgatg ccggccacga tgcgtccggc 5040
   gtagaggatc tacaactcca cttattgtta ggtagaattg tccgttagtt gtttattaat 5100
   tgcaataatg gggcgtccag ttttggcaac agtgtcctct taccaggaca cctatgagtt 5160
   tgcctcatgg caaactagag gtgttgaaag tatgcatggt tataattaga gcaattcatt 5220
   accctctgaa tcctgccggt ataccccatt gttcgttatc tttatttttg gctaaaaccg 5280
   cattaagagc ttcgtttacc gtcatgcaat gcggtaggtt atcgaagttt gatatcccgc 5340
   caatatcagg cgaacgcttg ttcttcaggt aagcatattt ccgcgcagcc gcctctactt 5400
   tctgcttgaa ctcatgtttt tgagtgcgtt ttttggataa ccgcagattg tcagcctttg 5460
   cttttgcctt agcgatccat gaagtcaatt ttttgaggct ggttgttccg gcaccgccgg 5520
   aaactgatct tttgtttttt ttaacttgtg acttcttatt ctttattgcc acgtcatcct 5580
   gacaggggga gggggtatca ttttgacatg ggggtgtgga taaaaaatta aataaagcca 5640
   atgtcttagc gagaacagct ttaaccttgg ttgccgctga agagatcttt aatttgcttt 5700
   ctatcagcgc atttttggct tgttgtgcga aggccaaaaa ggatggtgta aaccggtaca 5760
   ggttagcgcg acgttcacgg tgatcgccga taacaatctc tacagacaga attcctttgt 5820
   ttacagcttc acggaatgca cgaacgacgg ttgattggct ataaccagtt tctgccgcga 5880
   tcaggcggtg aggcttgtga atgaagtatt cactggttgt tgccgcgaga tttgcacatt 5940
   gcgacaggat atgcccggcg ctacgggata gaccggagtg tgttacaaag caggccaatt 6000
   catagccaga aaaagtaaaa tcgctcatcg ttatacagct caggaaagtg actttagcca 6060
   gcattacaat gctggtggtt cttactacgt ctgttagcgc gttgccgcga caggtaccag 6120
   cacaccagca tcaagcaatc gcttcatcag ccactgctga cctttgccgg ttatacgagt 6180
   cgtgaaagaa atcctgcttc cattgcttgt atcgatcacg gtttctttaa gggtgaaata 6240
   cccacgagat atgtattctt gtttggggac gttcctgcgt tcaccggttg cgatcagaat 6300
   tccgttatca cgcaaccagg tgaagagata gttttggccc aggccgagca ctttggcata 6360
   gttgccgatt agaaccccgc tggcggtagc aacgcgttca gcgaattcga ctttaggtgc 6420
   atccataagc attttttgct ccagccgttg cttttgctct gccaggtcgg cagccaaacg 6480
   gagagcttca gggagactct gcggaatagc aggttgtaat cttccggttc gatagtcgat 6540
   aaatgtctgg tttaccttca gccgaaacgc gggagaaatc cagcctgcgt actccacagc 6600
   gagcaattca tgggcaaaag tgccgccgcc acggccttcg acctgcaggc atgcaagctt 6660
   ggcgtaatca tggtcatagc tgtttcctgt gtgaaattgt tatccgctca caattccaca 6720
   caacatacga gccggaagca taaagtgtaa agcctggggt gcctaatgag tgagctaact 6780
   cacattaatt gcgttgcgct cactgcccgc tttccagtcg ggaaacctgt cgtgccaggt 6840
   agtcgatatg gtgcactctc agtacaatct gctctgatgc cgcatagtta agccagtata 6900
   cactccgcta tcgctacgtg actgggtcat ggctgcgccc cgacacccgc caacacccgc 6960
   tgacgcgccc tgacgggctt gtctgctccc ggcatccgct tacagacaag ctgtgaccgt 7020
   ctccgggagc tgcatgtgtc agaggttttc accgtcatca ccgaaacgcg cgaggcagct 7080
   gcggtaaagc tcatcagcgt ggtcgtgaag cgattcacag atgtctgcct gttcatccgc 7140
   gtccagctcg ttgagtttct ccagaagcgt taatgtctgg cttctgataa agcgggccat 7200
   gttaagggcg gttttttcct gtttggtcac tgatgcctcc gtgtaagggg gatttctgtt 7260
   catgggggta atgataccga tgaaacgaga gaggatgctc acgatacggg ttactgatga 7320
   tgaacatgcc cggttactgg aacgttgtga gggtaaacaa ctggcggtat ggatgcggcg 7380
   ggaccagaga aaaatcactc agggtcaatg ccagcgcttc gttaatacag atgtaggtgt 7440
   tccacagggt agccagcagc atcctgcgat gcagatccgg aacataatgg tgcagggcgc 7500
   tgacttccgc gtttccagac tttacgaaac acggaaaccg aagaccattc atgttgttgc 7560
   tcaggtcgca gacgttttgc agcagcagtc gcttcacgtt cgctcgcgta tcggtgattc 7620
   attctgctaa ccagtaaggc aaccccgcca gcctagccgg gtcctcaacg acaggagcac 7680
   gatcatgcgc acccgtggcc aggacccaac gctgcccgag atgcgccgcg tgcggctgct 7740
   ggagatggcg gacgcgatgg atatgttctg ccaagggttg gtttgcgcat tcacagttct 7800
   ccgcaagaat tgattggctc caattcttgg agtggtgaat ccgttagcga ggtgccgccg 7860
   gcttccattc aggtcgaggt ggcccggctc catgcaccgc gacgcaacgc ggggaggcag 7920
   acaaggtata gggcggcgcc tacaatccat gccaacccgt tccatgtgct cgccgaggcg 7980
   gcataaatcg ccgtgacgat cagcggtcca atgatcgaag ttaggctggt aagagccgcg 8040
   agcgatcctt gaagctgtcc ctgatggtcg tcatctacct gcctggacag catggcctgc 8100
   aacgcgggca tcccgatgcc gccggaagcg agaagaatca taatggggaa ggccatccag 8160
   cctcgcgtcg cgaacgccag caagacgtag cccagcgcgt cggccgccat gccggcgata 8220
   atggcctgct tctcgccgaa acgtttggtg gcgggaccag tgacgaaggc ttgagcgagg 8280
   gcgtgcaaga ttccgaatac cgcaagcgac aggccgatca tcgtcgcgct ccagcgaaag 8340
   cggtcctcgc cgaaaatgac ccagagcgct gccggcacct gtcctacgag ttgcatgata 8400
   aagaagacag tcataagtgc ggcgacgata gtcatgcccc gcgcccaccg gaaggagctg 8460
   actgggttga ggctctcaag ggcatcggtc gagcttgaca ttgtaggacg tttaaacatt 8520
   accctgttat ccctaggatc ctacgtaatc gatgaattcg atcccatttt tataactgga 8580
   tctcaaaata cctataaacc cattgttctt ctcttttagc tctaagaaca atcaatttat 8640
   aaatatattt attattatgc tataatataa atactatata aatacattta cctttttata 8700
   aatacattta cctttttttt aatttgcatg attttaatgc ttatgctatc ttttttattt 8760
   agtccataaa acctttaaag gaccttttct tatgggatat ttatattttc ctaacaaagc 8820
   aatcggcgtc ataaacttta gttgcttacg acgcctgtgg acgtcccccc cttcccctta 8880
   cgggcaagta aacttaggga ttttaatgca ataaataaat ttgtcctctt cgggcaaatg 8940
   aattttagta tttaaatatg acaagggtga accattactt ttgttaacaa gtgatcttac 9000
   cactcactat ttttgttgaa ttttaaactt atttaaaatt ctcgagaaag atttaaaaaa 9060
   taaacttttt taatctttta tttatttttt cttttttcgt atggaattgc ccaatattat 9120
   tcaacaattt atcggaaaca gcgttttaga gccaaataaa attggtcagt cgccatcgga 9180
   tgtttattct tttaatcgaa ataatgaaac tttttttctt aagcgatcta gcactttata 9240
   tacagagacc acatacagtg tctctcgtga agcgaaaatg ttgagttggc tctctgagaa 9300
   attaaaggtg cctgaactca tcatgacttt tcaggatgag cagtttgaat ttatgatcac 9360
   taaagcgatc aatgcaaaac caatttcagc gcttttttta acagaccaag aattgcttgc 9420
   tatctataag gaggcactca atctgttaaa ttcaattgct attattgatt gtccatttat 9480
   ttcaaacatt gatcatcggt taaaagagtc aaaatttttt attgataacc aactccttga 9540
   cgatatagat caagatgatt ttgacactga attatgggga gaccataaaa cttacctaag 9600
   tctatggaat gagttaaccg agactcgtgt tgaagaaaga ttggtttttt ctcatggcga 9660
   tatcacggat agtaatattt ttatagataa attcaatgaa atttattttt tagaccttgg 9720
   tcgtgctggg ttagcagatg aatttgtaga tatatccttt gttgaacgtt gcctaagaga 9780
   ggatgcatcg gaggaaactg cgaaaatatt tttaaagcat ttaaaaaatg atagacctga 9840
   caaaaggaat tattttttaa aacttgatga attgaattga ttccaagcat tatctaaaat 9900
   actctgcagg cacgctagct tgtactcaag ctcgtaacga aggtcgtgac cttgctcgtg 9960
   aaggtggcga cgtaattcgt tcagcttgta aatggtctcc agaacttgct gctgcatgtg 10020
   aagtttggaa agaaattaaa ttcgaatttg atactattga caaactttaa tttttatttt 10080
   tcatgatgtt tatgtgaata gcataaacat cgtttttatt tttatggtgt ttaggttaaa 10140
   tacctaaaca tcattttaca tttttaaaat taagttctaa agttatcttt tgtttaaatt 10200
   tgcctgtctt tataaattac gatgtgccag aaaaataaaa tcttagcttt ttattataga 10260
   atttatcttt atgtattata ttttataagt tataataaaa gaaatagtaa catactaaag 10320
   cggatgtagc gcgtttatct taacggaagg aattcggcgc ctacgtatac atactccgaa 10380
   ggaggacaaa tttatttatt gtggtacaat aaataagtgg tacaataaat aaattgtatg 10440
   taaacccctt ccccttcggg acgtcccctt acgggaatat aaatattagt ggcagttgcc 10500
   tgccaacaaa tttatttatt gtattaacat aggcagtggc ggtaccactg ccactggcgt 10560
   cctaatataa atattgggca actaaagttt atcgcagtat taacataggc agtggcggta 10620
   ccactgccac tggcgtcctc cttcggagta tgtaaacctg ctaccgcagc aaataaattt 10680
   tattctattt taatactaca atatttagat tcccgttagg ggataggcca ggcaattgtc 10740
   actggcgtca tagtatatca atattgtaac agattgacac cctttaagta aacatttttt 10800
   ttaggattca tatgaaatta aatggatatt tggtacattt aattccacaa aaatgtccaa 10860
   tacttaaaat acaaaattaa aagtattagt tgtaaacttg actaacattt taaattttaa 10920
   attttttcct aattatatat tttacttgca aaatttataa aaattttatg catttttata 10980
   tcataataat aaaaccttta ttcatggttt ataatataat aattgtgatg actatgcaca 11040
   aagcagttct agtcccatat atataactat atataacccg tttaaagatt tatttaaaaa 11100
   tatgtgtgta aaaaatgctt atttttaatt ttattttata taagttataa tattaaatac 11160
   acaatgatta aaattaaata ataataaatt taacgtaacg atgagttgtt tttttatttt 11220
   ggagatacac gcaatgacaa ttgcgatcgg tacatatcaa gagaaacgca catggttcga 11280
   tgacgctgat gactggcttc gtcaagaccg tttcgtattc gtaggttggt caggtttatt 11340
   actattccct tgtgcttact ttgcaactcc ggtccggcgg ccgcctcgag acgacttgtc 11400
   cgcttcatca gacacggctt tcctaaccat caatggtgga ttttcaggaa agacgtttaa 11460
   agaagtggca taaagtttat ttgttgaaga attggttttg tttccattca aagaattgtt 11520
   agggataaaa ctttgcattt ttttataatt tgttataagt ttttcaaact tatatgtttt 11580
   taaaaatgca tttaattgct tattaatgcg ttcattttgt aatgtttcaa taggtcttgc 11640
   ttgcgctaat cgcagtattc ccgatacttt gtctgcttgt ttttcgggta ttgagaataa 11700
   gtaagtataa tgatttaaaa aagtcatgtt ttgattaaat cttttttata tggttaaaaa 11760
   cattatggta tatctaaata aatttatttt ttactaaatc tccaatttgc aatttagaga 11820
   tataattaaa actataaagt tatttaagtt aatttgtaat caaatccaac acaaaaatgt 11880
   ttttatatag ttaacatgtt aaatttaaca tatgttaaac aactaaaatt ctgtaacaga 11940
   gaacaataaa ataaatgcta gattttgtgt aatgccgaag tatatttata tacttccctt 12000
   tcaaaaaaat aaatactctt gccactaaaa ttcatttgcc taggacgtcc ccttcccctt 12060
   acgggatgtt tatatactag gacgtcccct tccccttacg ggatatttat atactccgaa 12120
   ggacgtcccc ttcgggcaaa taaattttag tggcagttgc ctgccaactg cctaggcaag 12180
   taaacttagg gattttaatg caataaataa atttgtcccc ttacgggacg tcagtggcag 12240
   ttgcctgcca actgcctaat ataaatatta gtggatattt atatactccg aaggaggcag 12300
   ttacctgcca actgccgagg caaataaatt ttagtggcag tggtaccgcc actgcctgct 12360
   ccctccttcc ccttcgggca agtaaactta gcatgttgtc gacattaccc tgttatccct 12420
   aggccggcct aagaaaccat tattatcatg acattaacct ataaaaatag gcgtatcacg 12480
   aggccctttc gtcttcaaga aattcggtcg aaaaaagaaa aggagagggc caagagggag 12540
   ggcattggtg actattgagc acgtgagtat acgtgattaa gcacacaaag gcagcttgga 12600
   gtatgtctgt tattaatttc acaggtagtt ctggtccatt ggtgaaagtt tgcggcttgc 12660
   agagcacaga ggccgcagaa tgtgctctag attccgatgc tgacttgctg ggtattatat 12720
   gtgtgcccaa tagaaagaga acaattgacc cggttattgc aaggaaaatt tcaagtcttg 12780
   taaaagcata taaaaatagt tcaggcactc cgaaatactt ggttggcgtg tttcgtaatc 12840
   aacctaagga ggatgttttg gctctggtca atgattacgg cattgatatc gtccaactgc 12900
   atggagatga gtcgtggcaa gaataccaag agttcctcgg tttgccagtt attaaaagac 12960
   tcgtatttcc aaaagactgc aacatactac tcagtgcagc ttcacagaaa cctcattcgt 13020
   ttattccctt gtttgattca gaagcaggtg ggacaggtga acttttggat tggaactcga 13080
   tttctgactg ggttggaagg caagagagcc ccgaaagctt acattttatg ttagctggtg 13140
   gactgacgcc agaaaatgtt ggtgatgcgc ttagattaaa tggcgttatt ggtgttgatg 13200
   taagcggagg tgtggagaca aatggtgtaa aagactctaa caaaatagca aatttcgtca 13260
   aaaatgctaa gaaataggtt attactgagt agtatttatt taagtattgt ttgtgcactt 13320
   gcctgcaggc cttttgaaaa gcaagcataa aagatctaaa cataaaatct gtaaaataac 13380
   aagatgtaaa gataatgcta aatcatttgg ctttttgatt gattgtacag gaaaatatac 13440
   atcgcagggg gttgactttt accatttcac cgcaatggaa tcaaacttgt tgaagagaat 13500
   gttcacaggc gcatacgcta caatgacccg attcttgcta gccttttctc ggtcttgcaa 13560
   acaaccgccg gcagcttagt atataaatac acatgtacat acctctctcc gtatcctcgt 13620
   aatcattttc ttgtatttat cgtcttttcg ctgtaaaaac tttatcacac ttatctcaaa 13680
   tacacttatt aaccgctttt actattatct tctacgctga cagtaatatc aaacagtgac 13740
   acatattaaa cacagtggtt tctttgcata aacaccatca gcctcaagtc gtcaagtaaa 13800
   gatttcgtgt tcatgcagat agataacaat ctatatgttg ataattagcg ttgcctcatc 13860
   aatgcgagat ccgtttaacc ggaccctagt gcacttaccc cacgttcggt ccactgtgtg 13920
   ccgaacatgc tccttcacta ttttaacatg tggaattaat tctaaatcct ctttatatga 13980
   tctgccgata gatagttcta agtcattgag gttcatcaac aattggattt tctgtttact 14040
   cgacttcagg taatgaaatg agatgatact tgcttatctc atagttaact ggcataaatt 14100
   ttagtatagg ttaactctaa gaggtgatac ttatttactg taaaactgtg acgataaaac 14160
   cggaaggaag aataagaaaa ctcgaactga tctataatgc ctattttctg taaagagttt 14220
   aagctatgaa agcctcggca ttttggccgc tcctaggtag tgcttttttt ccaaggacaa 14280
   aacagtttct ttttcttgag caggttttat gtttcggtaa tcataaacaa taaataaatt 14340
   atttcattta tgtttaaaaa taaaaaataa aaaagtattt taaattttta aaaaagttga 14400
   ttataagcat gtgacctttt gcaagcaatt aaattttgca atttgtgatt taggcaaaag 14460
   ttactatttc tggctcgtgt aatatatgta tgctaatgtg aacttttaca aagtcgatat 14520
   ggacttagtc aaaagaaatt ttcttaaaaa tatatagcac tagccaattt agcacttctt 14580
   tatgagatat attatagact ttattaagcc agatttgtgt attatatgta tttacccggc 14640
   gaatcatgga catacattct gaaataggta atattctcta tggtgagaca gcatagataa 14700
   cctaggatac aagttaaaag ctagtactgt tttgcagtaa tttttttctt ttttataaga 14760
   atgttaccac ctaaataagt tataaagtca atagttaagt ttgatatttg attgtaaaat 14820
   accgtaatat atttgcatga tcaaaaggct caatgttgac tagccagcat gtcaaccact 14880
   atattgatca ccgatattag gacttccaca ccaactagta atatgacaat aaattcaaga 14940
   tattcttcat gagaatggcc cagctcatgt ttgacagctt atcatcgata agctttaatg 15000
   cggtagttta tcacagttaa attgctaacg cagtcaggca ccgtgtatga aatctaacaa 15060
   tgcgctcatc gtcatcctcg gcaccgtcac cctggatgct gtaggcatag gcttggttat 15120
   gccggtactg ccgggcctct tgcgggatat cgtccattcc gacagcatcg ccagtcacta 15180
   tggcgtgctg ctagcgctat atgcgttgat gcaatttcta tgcgcacccg ttctcggagc 15240
   actgtccgac cgctttggcc gccgcccagt cctgctcgct tcgctacttg gagccactat 15300
   cgactacgcg atcatggcga ccacacccgt cctgtggatc aattctttag tataaatttc 15360
   actctgaacc atcttggaag gaccggataa ttatttgaaa tctctttttc aattgtatat 15420
   gtgttatgta gtatactctt tcttcaacaa ttaaatactc tcggtagcca agttggttta 15480
   aggcgcaaga ctttaattta tcactacgga attggcctat taggcctacc cactagtcaa 15540
   ttcgggagga tcgaaacggc agatcgcaaa aaacagtaca tacagaagga gacatgaaca 15600
   tgaacatcaa aaaaattgta aaacaagcca cagttctgac ttttacgact gcacttctgg 15660
   caggaggagc gactcaagcc ttcgcgaaag aaaataacca aaaagcatac aaagaaacgt 15720
   acggcgtctc tcatattaca cgccatgata tgctgcagat ccctaaacag cagcaaaacg 15780
   aaaaatacca agtgcctcaa ttcgatcaat caacgattaa aaatattgag tctgcaaaag 15840
   gacttgatgt gtgggacagc tggccgctgc aaaacgctga cggaacagta gctgaataca 15900
   acggctatca cgttgtgttt gctcttgcgg gaagcccgaa agacgctgat gacacatcaa 15960
   tctacatgtt ttatcaaaag gtcggcgaca actcaatcga cagctggaaa aacgcgggcc 16020
   gtgtctttaa agacagcgat aagttcgacg ccaacgatcc gatcctgaaa gatcagacgc 16080
   aagaatggtc cggttctgca acctttacat ctgacggaaa aatccgttta ttctacactg 16140
   actattccgg taaacattac ggcaaacaaa gcctgacaac agcgcaggta aatgtgtcaa 16200
   aatctgatga cacactcaaa atcaacggag tggaagatca caaaacgatt tttgacggag 16260
   acggaaaaac atatcagaac gttcagcagt ttatcgatga aggcaattat acatccggcg 16320
   acaaccatac gctgagagac cctcactacg ttgaagacaa aggccataaa taccttgtat 16380
   tcgaagccaa cacgggaaca gaaaacggat accaaggcga agaatcttta tttaacaaag 16440
   cgtactacgg cggcggcacg aacttcttcc gtaaagaaag ccagaagctt cagcagagcg 16500
   ctaaaaaacg cgatgctgag ttagcgaacg gcgccctcgg tatcatagag ttaaataatg 16560
   attacacatt gaaaaaagta atgaagccgc tgatcacttc aaacacggta actgatgaaa 16620
   tcgagcgcgc gaatgttttc aaaatgaacg gcaaatggta cttgttcact gattcacgcg 16680
   gttcaaaaat gacgatcgat ggtattaact caaacgatat ttacatgctt ggttatgtat 16740
   caaactcttt aaccggccct tacaagccgc tgaacaaaac agggcttgtg ctgcaaatgg 16800
   gtcttgatcc aaacgatgtg acattcactt actctcactt cgcagtgccg caagccaaag 16860
   gcaacaatgt ggttatcaca agctacatga caaacagagg cttcttcgag gataaaaagg 16920
   caacatttgc gccaagcttc ttaatgaaca tcaaaggcaa taaaacatcc gttgtcaaaa 16980
   acagcatcct ggagcaagga cagctgacag tcaactaata acagcaaaaa gaaaatgccg 17040
   atacttcatt ggcattttct tttatttctc aacaagatgg tgaattgact agtgggtaga 17100
   tccacaggac gggtgtggtc gccatgatcg cgtagtcgat agtggctcca agtagcgaag 17160
   cgagcaggac tgggcggcgg ccaaagcggt cggacagtgc tccgagaacg ggtgcgcata 17220
   gaaattgcat caacgcatat agcgctagca gcacgccata gtgactggcg atgctgtcgg 17280
   aatggacgat atcccgcaag aggcccggca gtaccggcat aaccaagcct atgcctacag 17340
   catccagggt gacggtgccg aggatgacga tgagcgcatt gttagatttc atacacggtg 17400
   cctgactgcg ttagcaattt aactgtgata aactaccgca ttaaagctta tcgatgataa 17460
   gctgtcaaac atgagaattg atccggaacc cttaatataa cttcgtataa tgtatgctat 17520
   acgaagttat taggtccctc gactacgtcg ttaaggccgt ttctgacaga gtaaaattct 17580
   tgagggaact ttcaccatta tgggaaatgg ttcaagaagg tattgactta aactccatca 17640
   aatggtcagg tcattgagtg tttttatttt gttgtatttt ttttttttag agaaaatcct 17700
   ccaatatata aattaggaat catagtttca tgattttctg ttacacctaa ctttttgtgt 17760
   ggtgccctcc tccttgtcaa tattaatgtt aaagtgcaat tctttttcct tatcacgttg 17820
   agccattagt atcaatttgc ttacctgtat tcctttacat cctccttttt ctccttcttg 17880
   ataaatgtat gtagattgcg tatatagttt cgtctaccct atgaacatat tccattttgt 17940
   aatttcgtgt cgtttctatt atgaatttca tttataaagt ttatgtacaa atatcataaa 18000
   aaaagagaat ctttttaagc aaggattttc ttaacttctt cggcgacagc atcaccgact 18060
   tcggtggtac tgttggaacc acctaaatca ccagttctga tacctgcatc caaaaccttt 18120
   ttaactgcat cttcaatggc cttaccttct tcaggcaagt tcaatgacaa tttcaacatc 18180
   attgcagcag acaagatagt ggcgataggg ttgaccttat tctttggcaa atctggagca 18240
   gaaccgtggc atggttcgta caaaccaaat gcggtgttct tgtctggcaa agaggccaag 18300
   gacgcagatg gcaacaaacc caaggaacct gggataacgg aggcttcatc ggagatgata 18360
   tcaccaaaca tgttgctggt gattataata ccatttaggt gggttgggtt cttaactagg 18420
   atcatggcgg cagaatcaat caattgatgt tgaaccttca atgtagggaa ttcgttcttg 18480
   atggtttcct ccacagtttt tctccataat cttgaagagg ccaaaacatt agctttatcc 18540
   aaggaccaaa taggcaatgg tggctcatgt tgtagggcca tgaaagcggc cattcttgtg 18600
   attctttgca cttctggaac ggtgtattgt tcactatccc aagcgacacc atcaccatcg 18660
   tcttcctttc tcttaccaaa gtaaatacct cccactaatt ctctgacaac aacgaagtca 18720
   gtacctttag caaattgtgg cttgattgga gataagtcta aaagagagtc ggatgcaaag 18780
   ttacatggtc ttaagttggc gtacaattga agttctttac ggatttttag taaaccttgt 18840
   tcaggtctaa cactaccggt accgcgcttg cggaagcatc agcaaataag gccagcacag 18900
   ccagcgcagt tgccgctttg gttcctgatt ctgttcttga tgaattaaac aaagcggcac 18960
   agtaacaaag gacttcattg ataatttttc ttcaggagga agacatgtca ttcttttcta 19020
   cgttaaaaac agctttgtct ttgaaggaga aacttgctgc tactggtgtt cttgttctga 19080
   tttgcgcact tgttggtgct gggtttgcat gggaacgtca tcagctaaag caagccatag 19140
   agaaaattgg cagtcttgat caggctgtta aggaacgtga taagtcaata atggatctta 19200
   accagaccat tgagacgatg aacaaagcag agcaacattt tcacagccag gaagtgaaaa 19260
   atgaatcaga acaagccaag tatgctgaca ggcaaatgga acgaaaagct gaagttcaga 19320
   aacaactggt tgcggcgggt aatgttcgcc agcgtattcc tgctgacact cagcggttgc 19380
   tccgggagtc gatcagcgaa tttaacgccg acgccgacaa aggttaacca ccctgccccc 19440
   aaaagtgcat ttatgtgtag gatgccagag tttagcagtg aatattttga tgatctgcca 19500
   gcgtatatcc tcgatacaga aacgatgctg atggggatta acaggaagaa tcgcaacgtt 19560
   aatgattaca accgagctat tagcggtaac taaaagggat ttttatgtct gataaagtaa 19620
   cagtaaagca aactatcaac aaagcgactt caatctacaa aattgagcaa atcactgttg 19680
   gcaagccagg atctgaacaa taccgtcgtg ctttcgagct tgccgatcag cttggtttaa 19740
   aacacccgga ttgcattgag catgtatttc cgacctatgc tgatgagcaa tgtactcatg 19800
   ttcttaccga agaggatttt ttcagcactg aagaacgaga aggcgttgat cgctgcattg 19860
   gtgtgatttg ttcttcggta agtgatgagt tattccctaa tgtgcctgaa tatggtggta 19920
   ttggatacca attcctgtac gagggcgatg agcttaaatg ctatgaacat ggtcttctca 19980
   tcgaaagcgt agaataatac gactcccttc caaccggcta cgttggccgg tttttcactt 20040
   atccacatta tccactggat agatccaata atcaggtcca tacagatccc aattagatcc 20100
   atatagatcc ctgatcgttg caggccgcgc cacgtctggc ttagaagtgt atcgcgatgt 20160
   gtgctggagg gaaaacgatg tgtgctggag ggataaaaat gtgtgctgac gggttgctaa 20220
   tgtgtgctgg cgggatatag gatgtgtgtt gacgggaaag cttgggtagt tatcaccact 20280
   tataaaaact atccacacaa ttcggaaaaa gtaatatgaa tcaatcattt atctccgata 20340
   ttctttacgc agacattgaa agtaaggcaa aagaactaac agttaattca aacaacactg 20400
   tgcagcctgt agcgttgatg cgcttggggg tattcgtgcc gaagccatca aagagcaaag 20460
   gagaaagtaa agagattgat gccaccaaag cgttttccca gctggagata gctaaagccg 20520
   agggttacga tgatattaaa atcaccggtc ctcgactcga tatggatact gatttcaaaa 20580
   cgtggatcgg tgtcatctac gcgttcagca aatacggctt gtcctcaaac accatccagt 20640
   tatcgtttca ggaattcgct aaagcctgtg gtttcccctc aaaacgtctg gatgcgaaac 20700
   tgcgtttaac cattcatgaa tcacttggac gcttgcgtaa caagggtatc gcttttaagc 20760
   gcggaaaaga tgctaaaggc ggctatcaga ctggtctgct gaaggtcggg cgttttgatg 20820
   ctgaccttga tctgatagag ctggaggctg attcgaagtt gtgggagctg ttccagcttg 20880
   attatcgcgt tctgttgcaa caccacgcct tgcgtgccct tccgaagaaa gaagctgcac 20940
   aagccattta cactttcatc gaaagccttc cgcagaaccc gttgccgcta tcgttcgcgc 21000
   gaatccgtga gcgcctggct ttgcagtcag ctgttggcga gcaaaaccgt atcattaaga 21060
   aagcgataga acagcttaaa acaatcggct atctcgactg ttctattgag aagaaaggcc 21120
   gggaaagttt tgtaatcgtc cattctcgca atccaaagct gaaactcccc gaataagtgt 21180
   gtgctggagg gaaaccgcat taaaaagatg tgtgctgccg ggaaggcttg tccaatttcc 21240
   tgtttttgat gtgcgctgga gggggacgcc cctcagtttg cccagacttt ccctccagca 21300
   cacatctgtc catccgcttt tccctccagt gcacatgtaa ttctctgcct ttccctccag 21360
   cacacatatt tgataccagc gatccctcca cagcacataa ttcaatgcga cttccctcta 21420
   tcgcacatct tagactttta ttctccctcc agcacacatc gaagctgccg ggcaagccgt 21480
   tctcaccagt tgatagagag tgaagcttgg ctgcccattg aagcaggaaa tcaccaaaat 21540
   gattcaggct acaacctgaa cgtagaagaa atccgcgtcc tttatgcgtg gaggatgcca 21600
   aagcatgttg tgacacactt ggcaaaggag taagcatgca gagaatgcta tgtacaagca 21660
   tctacgcata cattattatt ttatgcagca tttttaatta aattcaaaaa tacagcataa 21720
   aggatgactt tcgatgagtg attccagcca gcttcacaag gttgctcaaa gagcaaacag 21780
   aatgctcaat gttctgactg aacaagtaca gttgcaaaag gatgagctac acgcgaacga 21840
   gttttaccag gtctatgcga aagcggcact ggcaaaattg cctctactga ctcgagcgaa 21900
   cgttgactat gccgtaagtg aaatggaaga aaagggttat gttttcgata aacgccctgc 21960
   tggctcttca atgaaatatg cgatgtcaat tcagaacatc attgacatat atgaacatcg 22020
   cggagtgcca aaataccggg atcgctacag cgaagcgtat gtgattttca tctccaatct 22080
   taaaggcggt gtgtcaaaaa ctgtatcgac ggtttctctg gcgcatgcaa tgcgtgctca 22140
   ccctcatctt cttatggagg atttaaggat tctggttatt gaccttgatc cgcaatcttc 22200
   agcaacgatg tttttaagcc ataaacactc tattggtatc gtaaacgcaa catctgcaca 22260
   ggctatgttg cagaatgtaa gccgtgaaga gctgttagag gagtttattg ttccttctgt 22320
   tgtacctggg gttgacgtta tgcctgcgtc gattgacgat gcctttattg catccgattg 22380
   gagagagctg tgcaatgagc atctaccggg tcagaacatc catgctgtcc tgaaagaaaa 22440
   tgtgattgat aagctgaaga gcgattatga ctttatcctc gttgatagtg gtcctcacct 22500
   tgacgccttc ctgaaaaatg ctttggcctc ggccaatata ctgtttacac ctctgccgcc 22560
   agcaactgtc gatttccact catcgcttaa atacgttgcc cgccttcctg agttggtgaa 22620
   actcatttcg gatgaaggct gcgagtgcca gcttgcgact aacattggtt ttatgtccaa 22680
   gttgagtaac aaggcagacc ataagtattg ccatagcctg gctaaagaag tgttcggtgg 22740
   ggatatgctt gatgtcttcc tccctcgcct tgacggtttt gaacgctgcg gcgagtcttt 22800
   tgacactgtt atttcagcta acccggcaac gtatgttggt agtgctgatg cattgaagaa 22860
   cgcgcgaatt gccgcggaag attttgctaa agcagttttt gaccgtat 22908
<210> 7
   <211> 7870
   <212> DNA
   <213> Artificial Sequence
<220>
   <223> Description of Artificial sequence: Synthetic polynucleotide
<400> 7
   gtgcactctc agtacaatct gctctgatgc cgcatagtta agccagcccc gacacccgcc 60
   aacacccgct gacgcgccct gacgggcttg tctgctcccg gcatccgctt acagacaagc 120
   tgtgaccgtc tccgggagct gcatgtgtca gaggttttca ccgtcatcac cgaaacgcgc 180
   gagacgaaag ggcctcgtga tacgcctatt tttataggtt aatgtcatga taataatggt 240
   ttcttagacg tcaggtggca cttttcgggg aaatgtgcgc ggaaccccta tttgtttatt 300
   tttctaaata cattcaaata tgtatccgct catgagacaa taaccctgat aaatgcttca 360
   ataatattga aaaaggaaga gtatgagtat tcaacatttc cgtgtcgccc ttattccctt 420
   ttttgcggca ttttgccttc ctgtttttgc tcacccagaa acgctggtga aagtaaaaga 480
   tgctgaagat cagttgggtg cacgagtggg ttacatcgaa ctggatctca acagcggtaa 540
   gatccttgag agttttcgcc ccgaagaacg ttttccaatg atgagcactt ttaaagttct 600
   gctatgtggc gcggtattat cccgtattga cgccgggcaa gagcaactcg gtcgccgcat 660
   acactattct cagaatgact tggttgagta ctcaccagtc acagaaaagc atcttacgga 720
   tggcatgaca gtaagagaat tatgcagtgc tgccataacc atgagtgata acactgcggc 780
   caacttactt ctgacaacga tcggaggacc gaaggagcta accgcttttt tgcacaacat 840
   gggggatcat gtaactcgcc ttgatcgttg ggaaccggag ctgaatgaag ccataccaaa 900
   cgacgagcgt gacaccacga tgcctgtagc aatggcaaca acgttgcgca aactattaac 960
   tggcgaacta cttactctag cttcccggca acaattaata gactggatgg aggcggataa 1020
   agttgcagga ccacttctgc gctcggccct tccggctggc tggtttattg ctgataaatc 1080
   tggagccggt gagcgtgggt ctcgcggtat cattgcagca ctggggccag atggtaagcc 1140
   ctcccgtatc gtagttatct acacgacggg gagtcaggca actatggatg aacgaaatag 1200
   acagatcgct gagataggtg cctcactgat taagcattgg taactgtcag accaagttta 1260
   ctcatatata ctttagattg atttaaaact tcatttttaa tttaaaagga tctaggtgaa 1320
   gatccttttt gataatctca tgaccaaaat cccttaacgt gagttttcgt tccactgagc 1380
   gtcagacccc gtagaaaaga tcaaaggatc ttcttgagat cctttttttc tgcgcgtaat 1440
   ctgctgcttg caaacaaaaa aaccaccgct accagcggtg gtttgtttgc cggatcaaga 1500
   gctaccaact ctttttccga aggtaactgg cttcagcaga gcgcagatac caaatactgt 1560
   tcttctagtg tagccgtagt taggccacca cttcaagaac tctgtagcac cgcctacata 1620
   cctcgctctg ctaatcctgt taccagtggc tgctgccagt ggcgataagt cgtgtcttac 1680
   cgggttggac tcaagacgat agttaccgga taaggcgcag cggtcgggct gaacgggggg 1740
   ttcgtgcaca cagcccagct tggagcgaac gacctacacc gaactgagat acctacagcg 1800
   tgagctatga gaaagcgcca cgcttcccga agggagaaag gcggacaggt atccggtaag 1860
   cggcagggtc ggaacaggag agcgcacgag ggagcttcca gggggaaacg cctggtatct 1920
   ttatagtcct gtcgggtttc gccacctctg acttgagcgt cgatttttgt gatgctcgtc 1980
   aggggggcgg agcctatgga aaaacgccag caacgcggcc tttttacggt tcctggcctt 2040
   ttgctggcct tttgctcaca tgttctttcc tgcgttatcc cctgattctg tggataaccg 2100
   tattaccgcc tttgagtgag ctgataccgc tcgccgcagc cgaacgaccg agcgcagcga 2160
   gtcagtgagc gaggaagcgg aagagcgccc aatacgcaaa ccgcctctcc ccgcgcgttg 2220
   gccgattcat taatgcagct ggcacgacag gtttcccgac tggaaagcgg gcagtgagcg 2280
   caacgcaatt aatgtgagtt agctcactca ttaggcaccc caggctttac actttatgct 2340
   tccggctcgt atgttgtgtg gaattgtgag cggataacaa tttcacacag gaaacagcta 2400
   tgaccatgat tacgccaagc tcgcggccgc agtactctgc agattttatg caaaattaaa 2460
   gtcttgtgac aacagctttc tccttaagtg caaatatcgc ccattctttc ctcttttcgt 2520
   atataaatgc tgtaatagta ggatgtcgta cccgtaaagg tacgacattg aatattaata 2580
   tactcctaag tttactttcc caatatttat attaggacgt ccccttcggg taaataaatt 2640
   ttagtggcag tggtaccgcc actccctatt ttaatactgc gaaggaggca gttggcaggc 2700
   aactcgtcgt tcgcagtata taaatatcca ctaatattta tattcccgta aggggacgtc 2760
   ccgaagggga aggggaaaga agcagtcgcc tccttgcgaa aaggtttact tgcccgacca 2820
   gtgaaaagca tgctgtaaga tataaatcta ccctgaaagg gatgcatttc accataatac 2880
   tatacaaatg gtgttaccct ttgaggatca taacggtgct actggaatat atggtctctt 2940
   catggataga cgatagccat ttatttaccc attaagggga cattagtggc ctgtcactgc 3000
   tccttacgag acgccagtgg acgttcgtcc tagaaaattt atgcgctgcc tagaagcccc 3060
   aaaagggaag tttactgact cgttagagcg tgcgctaaca ggtttaaata cttcaatatg 3120
   tatattagga cgccggtggc agtggtaccg ccactgccac cgtcggagga cgtcccttac 3180
   ggtatattat atactaggat tttaatactc cgaaggaggc agtggcggta ccactgccac 3240
   taatatttat attcccgtaa gggacgtcct ccttcggagt atgtaaacat tctaagttta 3300
   cttgcccaat atttatatta ggcagttggc aggcaactgc tagctctcct ccttcggagt 3360
   atgtaaacat cgcagtatat aaatatccac taatatttat attcccgtaa ggggacgtcc 3420
   cgaaggggaa ggggaaggac gtcagtggca gttgcctgcc aactgcctag gcaagtaaac 3480
   ttaggagtat ataaatatag gcagtcgcgg taccactgcc actgacgtcc tgccaactgc 3540
   ctaggcaagt aaacttaagt ggcactaaaa tgcatttgcc cgaaggggaa ggaggacgcc 3600
   agtggcagtg gtaccgccac tgcctccttc ggagtattaa aatcctagta tgtaaatctg 3660
   ctagcgcagg aaataaattt tattctattt atatactccg ttaggaggta agtaaacccc 3720
   ttccccttcg ggacgtcagt gcagttgcct gccaactgcc taatataaat attagaccac 3780
   taaagtttgg caactgccaa ctgttgtcct tcggaggaaa aaaaatggtt aactcgcaag 3840
   cagttaacat aactaaagtt tgttacttta ccgaagacgt ttaccctttc tcggttaagg 3900
   agacggagac agttgcactg tgactgccta gtatagcaat tttgtttttg tttatatgct 3960
   cgacaaaatg actttcataa aaatataaag tagttagcta gttattttat atcactataa 4020
   ctagggttct cagaggcacc gaagtcactt gtaaaaatag tactttttaa cttgtttaat 4080
   cttcgtgttc ttcaaaagga tcacgtaatt tttttgaagg tggaccaaaa ctaacataaa 4140
   ctgaatagcc agttacactt aacagaagaa accataaaaa aaaggtaaag aaaaaagctg 4200
   gactttccat agctcattta ataataaaat tattctcttt tcaacatatc tcttagatag 4260
   ttcaaaagac ttgacgactg tgtcccacat ttttaaacaa aattaatcta ctcaaaattt 4320
   tgccctgaga aagaataact tacttcgttt ttgcagtagc cattcatgtc actttgaaac 4380
   tgtccttaca aagttaaaca ttaattaaaa attatttaat ttttatataa caaatattat 4440
   attaaataaa aaatgaacaa agaacttcta agatcgtctt tagtgagtaa ttaaagagtt 4500
   ttacttacca gacaaggcag ttttttcatt cttttaaagc aggcagttct gaaggggaaa 4560
   agggactgcc tactgcggtc ctaggtaaat acatttttat gcaatttatt tcttgtgcta 4620
   gtaggtttct atactcacaa gaagcaaccc cttgacgaga gaacgttatc ctcagagtat 4680
   ttataatcct gagagggaat gcactgaaga atattttcct tattttttac agaaagtaaa 4740
   taaaatagcg ctaataacgc ttaattcatt taatcaatta tggcaacagg aacttctaaa 4800
   gctaaaccat caaaagtaaa ttcagacttc caagaacctg gtttagttac accattaggt 4860
   actttattac gtccacttaa ctcagaagca ggtaaagtat taccaggctg gggtacaact 4920
   gttttaatgg ctgtatttat ccttttattt gcagcattct tattaatcat tttagaaatt 4980
   tacaacagtt ctttaatttt agatgacgtt tctatgagtt gggaaacttt agctaaagtt 5040
   tcttaatttt atttaacaca aacataaaat ataaaactgt ttgttaaggc tagctgctaa 5100
   gtcttctttt cgctaaggta aactaagcaa ctcaaccata tttatattcg gcagtggcac 5160
   cgccaactgc cactggcctt ccgttaagat aaacgcgtgg atctcacgtg actagtgata 5220
   tctacgtaat cgatgaattc gatcccattt ttataactgg atctcaaaat acctataaac 5280
   ccattgttct tctcttttag ctctaagaac aatcaattta taaatatatt tattattatg 5340
   ctataatata aatactatat aaatacattt acctttttat aaatacattt accttttttt 5400
   taatttgcat gattttaatg cttatgctat cttttttatt tagtccataa aacctttaaa 5460
   ggaccttttc ttatgggata tttatatttt cctaacaaag caatcggcgt cataaacttt 5520
   agttgcttac gacgcctgtg gacgtccccc ccttcccctt acgggcaagt aaacttaggg 5580
   attttaatgc aataaataaa tttgtcctct tcgggcaaat gaattttagt atttaaatat 5640
   gacaagggtg aaccattact tttgttaaca agtgatctta ccactcacta tttttgttga 5700
   attttaaact tatttaaaat tctcgagaaa gattttaaaa ataaactttt ttaatctttt 5760
   atttattttt tcttttttcg tatggctcgt gaagcggtta tcgccgaagt atcaactcaa 5820
   ctatcagagg tagttggcgt catcgagcgc catctcgaac cgacgttgct ggccgtacat 5880
   ttgtacggct ccgcagtgga tggcggcctg aagccacaca gtgatattga tttgctggtt 5940
   acggtgaccg taaggcttga tgaaacaacg cggcgagctt tgatcaacga ccttttggaa 6000
   acttcggctt cccctggaga gagcgagatt ctccgcgctg tagaagtcac cattgttgtg 6060
   cacgacgaca tcattccgtg gcgttatcca gctaagcgcg aactgcaatt tggagaatgg 6120
   cagcgcaatg acattcttgc aggtatcttc gagccagcca cgatcgacat tgatctggct 6180
   atcttgctga caaaagcaag agaacatagc gttgccttgg taggtccagc ggcggaggaa 6240
   ctctttgatc cggttcctga acaggatcta tttgaggcgc taaatgaaac cttaacgcta 6300
   tggaactcgc cgcccgactg ggctggcgat gagcgaaatg tagtgcttac gttgtcccgc 6360
   atttggtaca gcgcagtaac cggcaaaatc gcgccgaagg atgtcgctgc cgactgggca 6420
   atggagcgcc tgccggccca gtatcagccc gtcatacttg aagctagaca ggcttatctt 6480
   ggacaagaag aagatcgctt ggcctcgcgc gcagatcagt tggaagaatt tgtccactac 6540
   gtgaaaggcg agatcactaa ggtagttggc aaataattcc aagcattatc taaaatactc 6600
   tgcaggcacg ctagcttgta ctcaagctcg taacgaaggt cgtgaccttg ctcgtgaagg 6660
   tggcgacgta attcgttcag cttgtaaatg gtctccagaa cttgctgctg catgtgaagt 6720
   ttggaaagaa attaaattcg aatttgatac tattgacaaa ctttaatttt tatttttcat 6780
   gatgtttatg tgaatagcat aaacatcgtt tttattttta tggtgtttag gttaaatacc 6840
   taaacatcat tttacatttt taaaattaag ttctaaagtt atcttttgtt taaatttgcc 6900
   tgtctttata aattacgatg tgccagaaaa ataaaatctt agctttttat tatagaattt 6960
   atctttatgt attatatttt ataagttata ataaaagaaa tagtaacata ctaaagcgga 7020
   tgtagcgcgt ttatcttaac ggaaggaatt cggcgcctac gtacccgggt cgcgaggatc 7080
   cacgcgttaa tagctcactt ttctttaaat ttaattttta atttaaaggt gtaagcaaat 7140
   tgcctgacga gagatccact taaaggatga cagtggcggg ctactgccta cttccctccg 7200
   ggataaaatt tatttgaaaa acgttagtta cttcctaacg gagcattgac atccccatat 7260
   ttatattagg acgtcccctt cgggtaaata aattttagtg gacgtcccct tcgggcaaat 7320
   aaattttagt ggacaataaa taaatttgtt gcctgccaac tgcctaggca agtaaacttg 7380
   ggagtattaa aataggacgt cagtggcagt tgcctgccaa ctgcctatat ttatatactg 7440
   cgaagcaggc agtggcggta ccactgccac tggcgtccta atataaatat tgggcaacta 7500
   aagtttatag cagtattaac atcctatatt tatatactcc gaaggaactt gttagccgat 7560
   aggcgaggca acaaatttat ttattgtccc gtaaaaggat gcctccagca tcgaagggga 7620
   aggggacgtc ctaggccata aaactaaagg gaaatccata gtaactgatg ttataaattt 7680
   atagactcca aaaaacagct gcgttataaa taacttctgt taaatatggc caaggggaca 7740
   ggggcacttt caactaagtg tacattaaaa attgacaatt caattttttt taattataat 7800
   atatatttag taaaatataa caaaaagccc ccatcgtcta ggtagaattc cagctggcgg 7860
   ccgccctatg 7870
<210> 8
   <211> 1353
   <212> DNA
   <213> Saccharomyces cerevisiae
<400> 8
   tcgcgcgttt cggtgatgac ggtgaaaacc tctgacacat gcagctcccg gagacggtca 60
   cagcttgtct gtaagcggat gccgggagca gacaagcccg tcagggagcg cgtcagcggg 120
   tgttggcggg tgtcggggct ggcttaacta tgcggcatca gagcagattg tactgagagt 180
   gcaccatacc acagcttttc aattcaattc atcatttttt ttttattctt ttttttgatt 240
   tcggtttctt tgaaattttt ttgattcggt aatctccgaa cagaaggaag aacgaaggaa 300
   ggagcacaga cttagattgg tatatatacg catatgtagt gttgaagaaa catgaaattg 360
   cccagtattc ttaacccaac tgcacagaac aaaaacctgc aggaaacgaa gataaatcat 420
   gtcgaaagct acatataagg aacgtgctgc tactcatcct agtcctgttg ctgccaagct 480
   atttaatatc atgcacgaaa agcaaacaaa cttgtgtgct tcattggatg ttcgtaccac 540
   caaggaatta ctggagttag ttgaagcatt aggtcccaaa atttgtttac taaaaacaca 600
   tgtggatatc ttgactgatt tttccatgga gggcacagtt aagccgctaa aggcattatc 660
   cgccaagtac aattttttac tcttcgaaga cagaaaattt gctgacattg gtaatacagt 720
   caaattgcag tactctgcgg gtgtatacag aatagcagaa tgggcagaca ttacgaatgc 780
   acacggtgtg gtgggcccag gtattgttag cggtttgaag caggcggcag aagaagtaac 840
   aaaggaacct agaggccttt tgatgttagc agaattgtca tgcaagggct ccctatctac 900
   tggagaatat actaagggta ctgttgacat tgcgaagagc gacaaagatt ttgttatcgg 960
   ctttattgct caaagagaca tgggtggaag agatgaaggt tacgattggt tgattatgac 1020
   acccggtgtg ggtttagatg acaagggaga cgcattgggt caacagtata gaaccgtgga 1080
   tgatgtggtc tctacaggat ctgacattat tattgttgga agaggactat ttgcaaaggg 1140
   aagggatgct aaggtagagg gtgaacgtta cagaaaagca ggctgggaag catatttgag 1200
   aagatgcggc cagcaaaact aaaaaactgt attataagta aatgcatgta tactaaactc 1260
   acaaattaga gcttcaattt aattatatca gttattaccc tatgcggtgt gaaataccgc 1320
   acagatgcgt aaggagaaaa taccgcatca gga 1353
<210> 9
   <211> 2474
   <212> DNA
   <213> Saccharomyces cerevisiae
<400> 9
   tcctgatgcg gtattttctc cttacgcatc tgtgcggtat ttcacaccgc atatcgacgg 60
   tcgaggagaa cttctagtat atccacatac ctaatattat tgccttatta aaaatggaat 120
   cccaacaatt acatcaaaat ccacattctc ttcaaaatca attgtcctgt acttccttgt 180
   tcatgtgtgt tcaaaaacgt tatatttata ggataattat actctatttc tcaacaagta 240
   attggttgtt tggccgagcg gtctaaggcg cctgattcaa gaaatatctt gaccgcagtt 300
   aactgtggga atactcaggt atcgtaagat gcaagagttc gaatctctta gcaaccatta 360
   tttttttcct caacataacg agaacacaca ggggcgctat cgcacagaat caaattcgat 420
   gactggaaat tttttgttaa tttcagaggt cgcctgacgc atataccttt ttcaactgaa 480
   aaattgggag aaaaaggaaa ggtgagaggc cggaaccggc ttttcatata gaatagagaa 540
   gcgttcatga ctaaatgctt gcatcacaat acttgaagtt gacaatatta tttaaggacc 600
   tattgttttt tccaataggt ggttagcaat cgtcttactt tctaactttt cttacctttt 660
   acatttcagc aatatatata tatatttcaa ggatatacca ttctaatgtc tgcccctatg 720
   tctgccccta agaagatcgt cgttttgcca ggtgaccacg ttggtcaaga aatcacagcc 780
   gaagccatta aggttcttaa agctatttct gatgttcgtt ccaatgtcaa gttcgatttc 840
   gaaaatcatt taattggtgg tgctgctatc gatgctacag gtgtcccact tccagatgag 900
   gcgctggaag cctccaagaa ggttgatgcc gttttgttag gtgctgtggg tggtcctaaa 960
   tggggtaccg gtagtgttag acctgaacaa ggtttactaa aaatccgtaa agaacttcaa 1020
   ttgtacgcca acttaagacc atgtaacttt gcatccgact ctcttttaga cttatctcca 1080
   atcaagccac aatttgctaa aggtactgac ttcgttgttg tcagagaatt agtgggaggt 1140
   atttactttg gtaagagaaa ggaagacgat ggtgatggtg tcgcttggga tagtgaacaa 1200
   tacaccgttc cagaagtgca aagaatcaca agaatggccg ctttcatggc cctacaacat 1260
   gagccaccat tgcctatttg gtccttggat aaagctaatg ttttggcctc ttcaagatta 1320
   tggagaaaaa ctgtggagga aaccatcaag aacgaattcc ctacattgaa ggttcaacat 1380
   caattgattg attctgccgc catgatccta gttaagaacc caacccacct aaatggtatt 1440
   ataatcacca gcaacatgtt tggtgatatc atctccgatg aagcctccgt tatcccaggt 1500
   tccttgggtt tgttgccatc tgcgtccttg gcctctttgc cagacaagaa caccgcattt 1560
   ggtttgtacg aaccatgcca cggttctgct ccagatttgc caaagaataa ggttgaccct 1620
   atcgccacta tcttgtctgc tgcaatgatg ttgaaattgt cattgaactt gcctgaagaa 1680
   ggtaaggcca ttgaagatgc agttaaaaag gttttggatg caggtatcag aactggtgat 1740
   ttaggtggtt ccaacagtac caccgaagtc ggtgatgctg tcgccgaaga agttaagaaa 1800
   atccttgctt aaaaagattc tcttttttta tgatatttgt acataaactt tataaatgaa 1860
   attcataata gaaacgacac gaaattacaa aatggaatat gttcataggg tagacgaaac 1920
   tatatacgca atctacatac atttatcaag aaggagaaaa aggaggatag taaaggaata 1980
   caggtaagca aattgatact aatggctcaa cgtgataagg aaaaagaatt gcactttaac 2040
   attaatattg acaaggagga gggcaccaca caaaaagtta ggtgtaacag aaaatcatga 2100
   aactacgatt cctaatttga tattggagga ttttctctaa aaaaaaaaaa atacaacaaa 2160
   taaaaaacac tcaatgacct gaccatttga tggagtttaa gtcaatacct tcttgaacca 2220
   tttcccataa tggtgaaagt tccctcaaga attttactct gtcagaaacg gccttacgac 2280
   gtagtcgata tggtgcactc tcagtacaat ctgctctgat gccgcatagt taagccagcc 2340
   ccgacacccg ccaacacccg ctgacgcgcc ctgacgggct tgtctgctcc cggcatccgc 2400
   ttacagacaa gctgtgaccg tctccgggag ctgcatgtgt cagaggtttt caccgtcatc 2460
   accgaaacgc gcga 2474
<210> 10
   <211> 1177
   <212> DNA
   <213> Saccharomyces cerevisiae
<400> 10
   aattcccgtt ttaagagctt ggtgagcgct aggagtcact gccaggtatc gtttgaacac 60
   ggcattagtc agggaagtca taacacagtc ctttcccgca attttctttt tctattactc 120
   ttggcctcct ctagtacact ctatattttt ttatgcctcg gtaatgattt tcattttttt 180
   ttttccccta gcggatgact cttttttttt cttagcgatt ggcattatca cataatgaat 240
   tatacattat ataaagtaat gtgatttctt cgaagaatat actaaaaaat gagcaggcaa 300
   gataaacgaa ggcaaagatg acagagcaga aagccctagt aaagcgtatt acaaatgaaa 360
   ccaagattca gattgcgatc tctttaaagg gtggtcccct agcgatagag cactcgatct 420
   tcccagaaaa agaggcagaa gcagtagcag aacaggccac acaatcgcaa gtgattaacg 480
   tccacacagg tatagggttt ctggaccata tgatacatgc tctggccaag cattccggct 540
   ggtcgctaat cgttgagtgc attggtgact tacacataga cgaccatcac accactgaag 600
   actgcgggat tgctctcggt caagctttta aagaggccct actggcgcgt ggagtaaaaa 660
   ggtttggatc aggatttgcg cctttggatg aggcactttc cagagcggtg gtagatcttt 720
   cgaacaggcc gtacgcagtt gtcgaacttg gtttgcaaag ggagaaagta ggagatctct 780
   cttgcgagat gatcccgcat tttcttgaaa gctttgcaga ggctagcaga attaccctcc 840
   acgttgattg tctgcgaggc aagaatgatc atcaccgtag tgagagtgcg ttcaaggctc 900
   ttgcggttgc cataagagaa gccacctcgc ccaatggtac caacgatgtt ccctccacca 960
   aaggtgttct tatgtagtga caccgattat ttaaagctgc agcatacgat atatatacat 1020
   gtgtatatat gtatacctat gaatgtcagt aagtatgtat acgaacagta tgatactgaa 1080
   gatgacaagg taatgcatca ttctatacgt gtcattctga acgaggcgcg ctttcctttt 1140
   ttctttttgc tttttctttt tttttctctt gaactcg 1177
<210> 11
   <211> 3008
   <212> DNA
   <213> Saccharomyces cerevisiae
<400> 11
   tgggcaattt catgtttctt caacactaca tatgcgtata tataccaatc taagtctgtg 60
   ctccttcctt cgttcttcct tctgttcgga gattaccgaa tcaaaaaaat ttcaaggaaa 120
   ccgaaatcaa aaaaaagaat aaaaaaaaaa tgatgaattg aaaccccccc cccccccccc 180
   gatgcgccgc gtgcggctgc tggagatggc ggacgcgatg gatatgttct gccaagggtt 240
   gggtcgacgc taccttaaga gagacgcgtg cggccgcaag cttgcatgcc tgcaggtcga 300
   tcgactctag aaatcgatag atctgaatta attcttgaat aatacataac ttttcttaaa 360
   agaatcaaag acagataaaa tttaagagat attaaatatt agtgagaagc cgagaatttt 420
   gtaacaccaa cataacactg acatctttaa caacttttaa ttatgataca tttcttacgt 480
   catgattgat tattacagct atgctgacaa atgactcttg ttgcatggct acgaaccggg 540
   taatactaag tgattgactc ttgctgacct tttattaaga actaaatgga caatattatg 600
   gagcatttca tgtataaatt ggtgcgtaaa atcgttggat ctctcttcta agtacatcct 660
   actataacaa tcaagaaaaa caagaaaatc ggacaaaaca atcaagtatg gattctagaa 720
   cagttggtat attaggaggg ggacaattgg gacgtatgat tgttgaggca gcaaacaggc 780
   tcaacattaa gacggtaata ctagatgctg aaaattctcc tgccaaacaa ataagcaact 840
   ccaatgacca cgttaatggc tccttttcca atcctcttga tatcgaaaaa ctagctgaaa 900
   aatgtgatgt gctaacgatt gagattgagc atgttgatgt tcctacacta aagaatcttc 960
   aagtaaaaca tcccaaatta aaaatttacc cttctccaga aacaatcaga ttgatacaag 1020
   acaaatatat tcaaaaagag catttaatca aaaatggtat agcagttacc caaagtgttc 1080
   ctgtggaaca agccagtgag acgtccctat tgaatgttgg aagagatttg ggttttccat 1140
   tcgtcttgaa gtcgaggact ttggcatacg atggaagagg taacttcgtt gtaaagaata 1200
   aggaaatgat tccggaagct ttggaagtac tgaaggatcg tcctttgtac gccgaaaaat 1260
   gggcaccatt tactaaagaa ttagcagtca tgattgtgag gtctgttaac ggtttagtgt 1320
   tttcttaccc aattgtagag actatccaca aggacaatat ttgtgactta tgttatgcgc 1380
   ctgctagagt tccggactcc gttcaactta aggcgaagtt gttggcagaa aatgcaatca 1440
   aatcttttcc cggttgtggt atatttggtg tggaaatgtt ctatttagaa acaggggaat 1500
   tgcttattaa cgaaattgcc ccaaggcctc acaactctgg acattatacc attgatgctt 1560
   gcgtcacttc tcaatttgaa gctcatttga gatcaatatt ggatttgcca atgccaaaga 1620
   atttcacatc tttctccacc attacaacga acgccattat gctaaatgtt cttggagaca 1680
   aacatacaaa agataaagag ctagaaactt gcgaaagagc attggcgact ccaggttcct 1740
   cagtgtactt atatggaaaa gagtctagac ctaacagaaa agtaggtcac ataaatatta 1800
   ttgcctccag tatggcggaa tgtgaacaaa ggctgaacta cattacaggt agaactgata 1860
   ttccaatcaa aatctctgtc gctcaaaagt tggacttgga agcaatggtc aaaccattgg 1920
   ttggaatcat catgggatca gactctgact tgccggtaat gtctgccgca tgtgcggttt 1980
   taaaagattt tggcgttcca tttgaagtga caatagtctc tgctcataga actccacata 2040
   ggatgtcagc atatgctatt tccgcaagca agcgtggaat taaaacaatt atcgctggag 2100
   ctggtggggc tgctcacttg ccaggtatgg tggctgcaat gacaccactt cctgtcatcg 2160
   gtgtgcccgt aaaaggttct tgtctagatg gagtagattc tttacattca attgtgcaaa 2220
   tgcctagagg tgttccagta gctaccgtcg ctattaataa tagtacgaac gctgcgctgt 2280
   tggctgtcag actgcttggc gcttatgatt caagttatac aacgaaaatg gaacagtttt 2340
   tattaaagca agaagaagaa gttcttgtca aagcacaaaa gttagaaact gtcggttacg 2400
   aagcttatct agaaaacaag taatatataa gtttattgat atacttgtac agcaaataat 2460
   tataaaatga tatacctatt ttttaggctt tgttatgatt acatcaaatg tggacttcat 2520
   acatagaaat caacgcttac aggtgtcctt ttttaagaat ttcatacata agatctctcg 2580
   aggatccccg ggtaccgagc tcgaattcgc ggccgcccgc gggttaaccc tagggcatgc 2640
   actagtggcc taattggccg acgtcaggtg gcacttttcg gggaaatgtg cgcggaaccc 2700
   ctatttgttt atttttctaa atacattcaa atatgtatcc gctcatgaga caataaccct 2760
   gataaatgct tcaataatat tgaaaaagga agagtatgag tattcaacat ttccgtgtcg 2820
   cccttattcc cttttttgcg gcattttgcc ttcctgtttt tgctcaccca gaaacgctgg 2880
   tgaaagtaaa agatgctgaa gatcagttgg gtgcacgagt gggttacatc gaactggatc 2940
   tcaacagcgg taagatcctt gagagttttc gccccgaaga acgttttcca atgatgagca 3000
   cttttaaa 3008
<210> 12
   <211> 4879
   <212> DNA
   <213> Saccharomyces cerevisiae
<400> 12
   agcagttgct ttctcctatg ggaagagctt tctaagtctg aagaagtaaa cagttctttg 60
   ctatttcaca cttcctggtt gatggtcact tgctgcctga aatatatata tatgtatgac 120
   atatgtactt gttttctttt ttgtgccttt gttacgtcta tattcattga aactgattat 180
   tcgattttct tcttgctgac cgcttctaga ggcatcgcac agttttagcg aggaaaactc 240
   ttcaatagtt ttgccagcgg aattccactt gcaattacat aaaaaattcc ggcggttttt 300
   cgcgtgtgac tcaatgtcga aatacctgcc taatgaacat gaacatcgcc caaatgtatt 360
   tgaagacccg ctgggagaag ttcaagatat ataagtaaca agcagccaat agtataaaaa 420
   aaaatctgag tttattacct ttcctggaat ttcagtgaaa aactgctaat tatagagaga 480
   tatcacagag ttactcacta atgactaacg aaaaggtctg gatagagaag ttggataatc 540
   caactctttc agtgttacca catgactttt tacgcccaca acaagaacct tatacgaaac 600
   aagctacata ttcgttacag ctacctcagc tcgatgtgcc tcatgatagt ttttctaaca 660
   aatacgctgt cgctttgagt gtatgggctg cattgatata tagagtaacc ggtgacgatg 720
   atattgttct ttatattgcg aataacaaaa tcttaagatt caatattcaa ccaacgtggt 780
   catttaatga gctgtattct acaattaaca atgagttgaa caagctcaat tctattgagg 840
   ccaatttttc ctttgacgag ctagctgaaa aaattcaaag ttgccaagat ctggaaagga 900
   cccctcagtt gttccgtttg gcctttttgg aaaaccaaga tttcaaatta gacgagttca 960
   agcatcattt agtggacttt gctttgaatt tggataccag taataatgcg catgttttga 1020
   acttaattta taacagctta ctgtattcga atgaaagagt aaccattgtt gcggaccaat 1080
   ttactcaata tttgactgct gcgctaagcg atccatccaa ttgcataact aaaatctctc 1140
   tgatcaccgc atcatccaag gatagtttac ctgatccaac taagaacttg ggctggtgcg 1200
   atttcgtggg gtgtattcac gacattttcc aggacaatgc tgaagccttc ccagagagaa 1260
   cctgtgttgt ggagactcca acactaaatt ccgacaagtc ccgttctttc acttatcgcg 1320
   acatcaaccg cacttctaac atagttgccc attatttgat taaaacaggt atcaaaagag 1380
   gtgatgtagt gatgatctat tcttctaggg gtgtggattt gatggtatgt gtgatgggtg 1440
   tcttgaaagc cggcgcaacc ttttcagtta tcgaccctgc atatccccca gccagacaaa 1500
   ccatttactt aggtgttgct aaaccacgtg ggttgattgt tattagagct gctggacaat 1560
   tggatcaact agtagaagat tacatcaatg atgaattgga gattgtttca agaatcaatt 1620
   ccatcgctat tcaagaaaat ggtaccattg aaggtggcaa attggacaat ggcgaggatg 1680
   ttttggctcc atatgatcac tacaaagaca ccagaacagg tgttgtagtt ggaccagatt 1740
   ccaacccaac cctatctttc acatctggtt ccgaaggtat tcctaagggt gttcttggta 1800
   gacatttttc cttggcttat tatttcaatt ggatgtccaa aaggttcaac ttaacagaaa 1860
   atgataaatt cacaatgctg agcggtattg cacatgatcc aattcaaaga gatatgttta 1920
   caccattatt tttaggtgcc caattgtatg tccctactca agatgatatt ggtacaccgg 1980
   gccgtttagc ggaatggatg agtaagtatg gttgcacagt tacccattta acacctgcca 2040
   tgggtcaatt acttactgcc caagctacta caccattccc taagttacat catgcgttct 2100
   ttgtgggtga cattttaaca aaacgtgatt gtctgaggtt acaaaccttg gcagaaaatt 2160
   gccgtattgt taatatgtac ggtaccactg aaacacagcg tgcagtttct tatttcgaag 2220
   ttaaatcaaa aaatgacgat ccaaactttt tgaaaaaatt gaaagatgtc atgcctgctg 2280
   gtaaaggtat gttgaacgtt cagctactag ttgttaacag gaacgatcgt actcaaatat 2340
   gtggtattgg cgaaataggt gagatttatg ttcgtgcagg tggtttggcc gaaggttata 2400
   gaggattacc agaattgaat aaagaaaaat ttgtgaacaa ctggtttgtt gaaaaagatc 2460
   actggaatta tttggataag gataatggtg aaccttggag acaattctgg ttaggtccaa 2520
   gagatagatt gtacagaacg ggtgatttag gtcgttatct accaaacggt gactgtgaat 2580
   gttgcggtag ggctgatgat caagttaaaa ttcgtgggtt cagaatcgaa ttaggagaaa 2640
   tagatacgca catttcccaa catccattgg taagagaaaa cattacttta gttcgcaaaa 2700
   atgccgacaa tgagccaaca ttgatcacat ttatggtccc aagatttgac aagccagatg 2760
   acttgtctaa gttccaaagt gatgttccaa aggaggttga aactgaccct atagttaagg 2820
   gcttaatcgg ttaccatctt ttatccaagg acatcaggac tttcttaaag aaaagattgg 2880
   ctagctatgc tatgccttcc ttgattgtgg ttatggataa actaccattg aatccaaatg 2940
   gtaaagttga taagcctaaa cttcaattcc caactcccaa gcaattaaat ttggtagctg 3000
   aaaatacagt ttctgaaact gacgactctc agtttaccaa tgttgagcgc gaggttagag 3060
   acttatggtt aagtatatta cctaccaagc cagcatctgt atcaccagat gattcgtttt 3120
   tcgatttagg tggtcattct atcttggcta ccaaaatgat tttaccttta aagaaaaagc 3180
   tgcaagttga tttaccattg ggcacaattt tcaagtatcc aacgataaag gcctttgccg 3240
   cggaaattga cagaattaaa tcatcgggtg gatcatctca aggtgaggtc gtcgaaaatg 3300
   tcactgcaaa ttatgcggaa gacgccaaga aattggttga gacgctacca agttcgtacc 3360
   cctctcgaga atattttgtt gaacctaata gtgccgaagg aaaaacaaca attaatgtgt 3420
   ttgttaccgg tgtcacagga tttctgggct cctacatcct tgcagatttg ttaggacgtt 3480
   ctccaaagaa ctacagtttc aaagtgtttg cccacgtcag ggccaaggat gaagaagctg 3540
   catttgcaag attacaaaag gcaggtatca cctatggtac ttggaacgaa aaatttgcct 3600
   caaatattaa agttgtatta ggcgatttat ctaaaagcca atttggtctt tcagatgaga 3660
   agtggatgga tttggcaaac acagttgata taattatcca taatggtgcg ttagttcact 3720
   gggtttatcc atatgccaaa ttgagggatc caaatgttat ttcaactatc aatgttatga 3780
   gcttagccgc cgtcggcaag ccaaagttct ttgactttgt ttcctccact tctactcttg 3840
   acactgaata ctactttaat ttgtcagata aacttgttag cgaagggaag ccaggcattt 3900
   tagaatcaga cgatttaatg aactctgcaa gcgggctcac tggtggatat ggtcagtcca 3960
   aatgggctgc tgagtacatc attagacgtg caggtgaaag gggcctacgt gggtgtattg 4020
   tcagaccagg ttacgtaaca ggtgcctctg ccaatggttc ttcaaacaca gatgatttct 4080
   tattgagatt tttgaaaggt tcagtccaat taggtaagat tccagatatc gaaaattccg 4140
   tgaatatggt tccagtagat catgttgctc gtgttgttgt tgctacgtct ttgaatcctc 4200
   ccaaagaaaa tgaattggcc gttgctcaag taacgggtca cccaagaata ttattcaaag 4260
   actacttgta tactttacac gattatggtt acgatgtcga aatcgaaagc tattctaaat 4320
   ggaagaaatc attggaggcg tctgttattg acaggaatga agaaaatgcg ttgtatcctt 4380
   tgctacacat ggtcttagac aacttacctg aaagtaccaa agctccggaa ctagacgata 4440
   ggaacgccgt ggcatcttta aagaaagaca ccgcatggac aggtgttgat tggtctaatg 4500
   gaataggtgt tactccagaa gaggttggta tatatattgc atttttaaac aaggttggat 4560
   ttttacctcc accaactcat aatgacaaac ttccactgcc aagtatagaa ctaactcaag 4620
   cgcaaataag tctagttgct tcaggtgctg gtgctcgtgg aagctccgca gcagcttaag 4680
   gttgagcatt acgtatgata tgtccatgta caataattaa atatgaatta ggagaaagac 4740
   ttagcttctt ttcgggtgat gtcacttaaa aactccgaga ataatatata ataagagaat 4800
   aaaatattag ttattgaata agaactgtaa atcagctggc gttagtctgc taatggcagc 4860
   ttcatcttgg tttattgta 4879
<210> 13
   <211> 1475
   <212> DNA
   <213> Artificial Sequence
<220>
   <223> Description of Artificial sequence: Synthetic polynucleotide
<400> 13
   ttaggtctag agatctgttt agcttgcctc gtccccgccg ggtcacccgg ccagcgacat 60
   ggaggcccag aataccctcc ttgacagtct tgacgtgcgc agctcagggg catgatgtga 120
   ctgtcgcccg tacatttagc ccatacatcc ccatgtataa tcatttgcat ccatacattt 180
   tgatggccgc acggcgcgaa gcaaaaatta cggctcctcg ctgcagacct gcgagcaggg 240
   aaacgctccc ctcacagacg cgttgaattg tccccacgcc gcgcccctgt agagaaatat 300
   aaaaggttag gatttgccac tgaggttctt ctttcatata cttcctttta aaatcttgct 360
   aggatacagt tctcacatca catccgaaca taaacaacca tgggtaagga aaagactcac 420
   gtttcgaggc cgcgattaaa ttccaacatg gatgctgatt tatatgggta taaatgggct 480
   cgcgataatg tcgggcaatc aggtgcgaca atctatcgat tgtatgggaa gcccgatgcg 540
   ccagagttgt ttctgaaaca tggcaaaggt agcgttgcca atgatgttac agatgagatg 600
   gtcagactaa actggctgac ggaatttatg cctcttccga ccatcaagca ttttatccgt 660
   actcctgatg atgcatggtt actcaccact gcgatccccg gcaaaacagc attccaggta 720
   ttagaagaat atcctgattc aggtgaaaat attgttgatg cgctggcagt gttcctgcgc 780
   cggttgcatt cgattcctgt ttgtaattgt ccttttaaca gcgatcgcgt atttcgtctc 840
   gctcaggcgc aatcacgaat gaataacggt ttggttgatg cgagtgattt tgatgacgag 900
   cgtaatggct ggcctgttga acaagtctgg aaagaaatgc ataagctttt gccattctca 960
   ccggattcag tcgtcactca tggtgatttc tcacttgata accttatttt tgacgagggg 1020
   aaattaatag gttgtattga tgttggacga gtcggaatcg cagaccgata ccaggatctt 1080
   gccatcctat ggaactgcct cggtgagttt tctccttcat tacagaaacg gctttttcaa 1140
   aaatatggta ttgataatcc tgatatgaat aaattgcagt ttcatttgat gctcgatgag 1200
   tttttctaat cagtactgac aataaaaaga ttcttgtttt caagaacttg tcatttgtat 1260
   agttttttta tattgtagtt gttctatttt aatcaaatgt tagcgtgatt tatatttttt 1320
   ttcgcctcga catcatctgc ccagatgcga agttaagtgc gcagaaagta atatcatgcg 1380
   tcaatcgtat gtgaatgctg gtcgctatac tgctgtcgat tcgatactaa cgccgccatc 1440
   cagtgtcgaa aacgagctct cgagaaccct taata 1475
<210> 14
   <211> 931
   <212> DNA
   <213> Artificial Sequence
<220>
   <223> Description of Artificial Sequence: Synthetic polynucleotide
<400> 14
   ttcaaatatg tatccgctca tgagacaata accctgataa atgcttcaat aatattgaaa 60
   aaggaagagt atgagtattc aacatttccg tgtcgccctt attccctttt ttgcggcatt 120
   ttgccttcct gtttttgctc acccagaaac gctggtgaaa gtaaaagatg ctgaagatca 180
   gttgggtgca cgagtgggtt acatcgaact ggatctcaac agcggtaaga tccttgagag 240
   ttttcgcccc gaagaacgtt ttccaatgat gagcactttt aaagttctgc tatgtggcgc 300
   ggtattatcc cgtattgacg ccgggcaaga gcaactcggt cgccgcatac actattctca 360
   gaatgacttg gttgagtact caccagtcac agaaaagcat cttacggatg gcatgacagt 420
   aagagaatta tgcagtgctg ccataaccat gagtgataac actgcggcca acttacttct 480
   gacaacgatc ggaggaccga aggagctaac cgcttttttg cacaacatgg gggatcatgt 540
   aactcgcctt gatcgttggg aaccggagct gaatgaagcc ataccaaacg acgagcgtga 600
   caccacgatg cctgcagcaa tggcaacaac gttgcgcaaa ctattaactg gcgaactact 660
   tactctagct tcccggcaac aattaataga ctggatggag gcggataaag ttgcaggacc 720
   acttctgcgc tcggcccttc cggctggctg gtttattgct gataaatctg gagccggtga 780
   gcgtgggtct cgcggtatca ttgcagcact ggggccagat ggtaagccct cccgtatcgt 840
   agttatctac acgacgggga gtcaggcaac tatggatgaa cgaaatagac agatcgctga 900
   gataggtgcc tcactgatta agcattggta a 931
<210> 15
   <211> 1382
   <212> DNA
   <213> Artificial Sequence
<220>
   <223> Description of Artificial Sequence: Synthetic polynucleotide
<400> 15
   ttctcatgtt tgacagctta tcatcgataa gctttaatgc ggtagtttat cacagttaaa 60
   ttgctaacgc agtcaggcac cgtgtatgaa atctaacaat gcgctcatcg tcatcctcgg 120
   caccgtcacc ctggatgctg taggcatagg cttggttatg ccggtactgc cgggcctctt 180
   gcgggatatc gtccattccg acagcatcgc cagtcactat ggcgtgctgc tagcgctata 240
   tgcgttgatg caatttctat gcgcacccgt tctcggagca ctgtccgacc gctttggccg    300
   ccgcccagtc ctgctcgctt cgctacttgg agccactatc gactacgcga tcatggcgac    360
   cacacccgtc ctgtggatcc tctacgccgg acgcatcgtg gccggcatca ccggcgccac    420
   aggtgcggtt gctggcgcct atatcgccga catcaccgat ggggaagatc gggctcgcca    480
   cttcgggctc atgagcgctt gtttcggcgt gggtatggtg gcaggccccg tggccggggg    540
   actgttgggc gccatctcct tgcatgcacc attccttgcg gcggcggtgc tcaacggcct    600
   caacctacta ctgggctgct tcctaatgca ggagtcgcat aagggagagc gtcgaccgat    660
   gcccttgaga gccttcaacc cagtcagctc cttccggtgg gcgcggggca tgactatcgt    720
   cgccgcactt atgactgtct tctttatcat gcaactcgta ggacaggtgc cggcagcgct    780
   ctgggtcatt ttcggcgagg accgctttcg ctggagcgcg acgatgatcg gcctgtcgct    840
   tgcggtattc ggaatcttgc acgccctcgc tcaagccttc gtcactggtc ccgccaccaa    900
   acgtttcggc gagaagcagg ccattatcgc cggcatggcg gccgacgcgc tgggctacgt    960
   cttgctggcg ttcgcgacgc gaggctggat ggccttcccc attatgattc ttctcgcttc   1020
   cggcggcatc gggatgcccg cgttgcaggc catgctgtcc aggcaggtag atgacgacca   1080
   tcagggacag cttcaaggat cgctcgcggc tcttaccagc ctaacttcga tcattggacc   1140
   gctgatcgtc acggcgattt atgccgcctc ggcgagcaca tggaacgggt tggcatggat   1200
   tgtaggcgcc gccctatacc ttgtctgcct ccccgcgttg cgtcgcggtg catggagccg   1260
   ggccacctcg acctgaatgg aagccggcgg cacctcgcta acggattcac cactccaaga   1320
   attggagcca atcaattctt gcggagaact gtgaatgcgc aaaccaaccc ttggcagaac   1380
   at                                                                  1382
<210> 16
   <211> 679
   <212> DNA
   <213> Artificial Sequence
<220>
   <223> Description of Artificial sequence: Synthetic polynucleotide
<400> 16
   cccaatggca tcgtaaagaa cattttgagg catttcagtc agttgctcaa tgtacctata     60
   accagaccgt tcagctggat attacggcct ttttaaagac cgtaaagaaa aataagcaca    120
   agttttatcc ggcctttatt cacattcttg cccgcctgat gaatgctcat ccggaattcc    180
   gtatggcaat gaaagacggt gagctggtga tatgggatag tgttcaccct tgttacaccg    240
   ttttccatga gcaaactgaa acgttttcat cgctctggag tgaataccac gacgatttcc    300
   ggcagtttct acacatatat tcgcaagatg tggcgtgtta cggtgaaaac ctggcctatt    360
   tccctaaagg gtttattgag aatatgtttt tcgtctcagc caatccctgg gtgagtttca    420
   ccagttttga tttaaacgtg gccaatatgg acaacttctt cgcccccgtt ttcaccatgg    480
   gcaaatatta tacgcaaggc gacaaggtgc tgatgccgct ggcgattcag gttcatcatg 540
   ccgtttgtga tggcttccat gtcggcagaa tgcttaatga attacaacag tactgcgatg 600
   agtggcaggg cggggcgtaa tttttttaag gcagttattg gtgcccttaa acgcctggtt 660
   gctacgcctg aataagtga 679
<210> 17
   <211> 875
   <212> DNA
   <213> Artificial Sequence
<220>
   <223> Description of Artificial Sequence: Synthetic polynucleotide
<400> 17
   ttgccgggtg acgcacaccg tggaaacgga tgaaggcacg aacccagttg acataagcct 60
   gttcggttcg taaactgtaa tgcaagtagc gtatgcgctc acgcaactgg tccagaacct 120
   tgaccgaacg cagcggtggt aacggcgcag tggcggtttt catggcttgt tatgactgtt 180
   tttttgtaca gtctatgcct cgggcatcca agcagcaagc gcgttacgcc gtgggtcgat 240
   gtttcatgtt atggagcagc aacgatgtta cgcagcagca acgatgttac gcagcagggc 300
   agtcgcccta aaacaaagtt aggtggctca agtatgggca tcattcgcac atgtaggctc 360
   ggccctgacc aagtcaaatc catgcgggct gctcttgatc ttttcggtag tgagttcgga 420
   gacgtagcca cctactccca acatcagccg gactccgatt acctcgggaa cttgctccgt 480
   agtaagacat tcatcgcgct tgctgccttc gaccaagaag cggttgttgg cgctctcgcg 540
   gcttacgttc tgcccaggtt tgagcagccg cgtagtgaga tctatatcta tgatctcgca 600
   gtctccggcg agcaccggag gcagggcatt gccaccgcgc tcatcaatct cctcaagcat 660
   gaggccaacg cgcttggtgc ttatgtgatc tacgtgcaag cagattacgg tgacgatccc 720
   gcagtggctc tctatacaaa gttgggcata cgggaagaag tgatgcactt tgatatcgac 780
   ccaagtaccg ccacctaaca attcgttcaa gccgagatcg gcttcccggc cgcggagttg 840
   ttcggtaaat tgtcacaacg ccgcggccat cggca 875
<210> 18
   <211> 1582
   <212> DNA
   <213> Artificial Sequence
<220>
   <223> Description of Artificial sequence: Synthetic polynucleotide
<400> 18
   actagtatga aattaaatgg atatttggta catttaattc cacaaaaatg tccaatactt 60
   aaaatacaaa attaaaagta ttagttgtaa acttgactaa cattttaaat tttaaatttt 120
   ttcctaatta tatattttac ttgcaaaatt tataaaaatt ttatgcattt ttatatcata 180
   ataataaaac ctttattcat ggtttataat ataataattg tgatgactat gcacaaagca 240
   gttctagtcc catatatata actatatata acccgtttaa agatttattt aaaaatatgt 300
   gtgtaaaaaa tgcttatttt taattttatt ttatataagt tataatatta aatacacaat 360
   gattaaaatt aaataataat aaatttaacg taacgatgag ttgttttttt attttggaga 420
   tacacgcata tggtaccagt atctttcaca agtcttttag cagcatctcc accttcacgt 480
   gcaagttgcc gtccagctgc tgaagtggaa tcagttgcag tagaaaaacg tcaaacaatt 540
   caaccaggta caggttacaa taacggttac ttttattctt actggaatga tggacacggt 600
   ggtgttacat atactaatgg acctggtggt caatttagtg taaattggag taactcaggc 660
   aattttgttg gaggaaaagg ttggcaacct ggtacaaaga ataaggtaat caatttctct 720
   ggtagttaca accctaatgg taattcttat ttaagtgtat acggttggag ccgtaaccca 780
   ttaattgaat attatattgt agagaacttt ggtacataca acccttcaac aggtgctact 840
   aaattaggtg aagttacttc agatggatca gtttatgata tttatcgtac tcaacgcgta 900
   aatcaaccat ctataattgg aactgccact ttctaccaat actggagtgt aagacgtaat 960
   catcgttcaa gtggtagtgt taatacagca aaccacttta atgcatgggc tcaacaaggt 1020
   ttaacattag gtacaatgga ctatcaaatt gtagctgttg aaggttattt ttcatcaggt 1080
   agtgcttcta tcactgttag cggtaccggt gaaaacttat actttcaagg ctcaggtggc 1140
   ggtggaagtg attacaaaga tgatgatgat aaaggaaccg gttaatctag acttagcttc 1200
   aactaactct agctcaaaca actaattttt ttttaaacta aaataaatct ggttaaccat 1260
   acctggttta ttttagttta gtttatacac acttttcata tatatatact taatagctac 1320
   cataggcagt tggcaggacg tccccttacg ggacaaatgt atttattgtt gcctgccaac 1380
   tgcctaatat aaatattagt ggacgtcccc ttccccttac gggcaagtaa acttagggat 1440
   tttaatgctc cgttaggagg caaataaatt ttagtggcag ttgcctcgcc tatcggctaa 1500
   caagttcctt cggagtatat aaatatcctg ccaactgccg atatttatat actaggcagt 1560
   ggcggtacca ctcgacacta gt 1582
<210> 19
   <211> 2017
   <212> DNA
   <213> Artificial Sequence
<220>
   <223> Description of Artificial sequence: Synthetic polynucleotide
<400> 19
   actagtatga aattaaatgg atatttggta catttaattc cacaaaaatg tccaatactt 60
   aaaatacaaa attaaaagta ttagttgtaa acttgactaa cattttaaat tttaaatttt 120
   ttcctaatta tatattttac ttgcaaaatt tataaaaatt ttatgcattt ttatatcata 180
   ataataaaac ctttattcat ggtttataat ataataattg tgatgactat gcacaaagca 240
   gttctagtcc catatatata actatatata acccgtttaa agatttattt aaaaatatgt 300
   gtgtaaaaaa tgcttatttt taattttatt ttatataagt tataatatta aatacacaat 360
   gattaaaatt aaataataat aaatttaacg taacgatgag ttgttttttt attttggaga 420
   tacacgcata tggtaccaca caagttcaca ggtgttaacg ctaaattcca gcaaccagca 480
   ttaagaaatt tatctccagt ggtagttgag cgcgaacgtg aggaatttgt aggattcttt 540
   ccacaaattg ttcgtgactt aactgaagat ggtattggtc atccagaagt aggtgacgct 600
   gtagctcgtc ttaaagaagt attacaatac aacgcacctg gtggtaaatg caatagaggt 660
   ttaacagttg ttgcagctta ccgtgaactt tctggaccag gtcaaaaaga cgctgaaagt 720
   cttcgttgtg ctttagcagt aggatggtgt attgaattat tccaagcctt tttcttagtt 780
   gctgacgata taatggacca gtcattaact agacgtggtc aattatgttg gtacaagaaa 840
   gaaggtgttg gtttagatgc aataaatgat tcttttcttt tagaaagctc tgtgtatcgc 900
   gttcttaaaa agtattgccg tcaacgtcca tattatgtac atttattaga gctttttctt 960
   caaacagctt accaaacaga attaggacaa atgttagatt taatcactgc tcctgtatct 1020
   aaggtagatt taagccattt ctcagaagaa cgttacaaag ctattgttaa gtataaaact 1080
   gctttctatt cattctattt accagttgca gcagctatgt atatggttgg tatagattct 1140
   aaagaagaac atgaaaacgc aaaagctatt ttacttgaga tgggtgaata cttccaaatt 1200
   caagatgatt atttagattg ttttggcgat cctgctttaa caggtaaagt aggtactgat 1260
   attcaagata acaaatgttc atggttagtt gtgcaatgct tacaaagagt aacaccagaa 1320
   caacgtcaac ttttagaaga taattacggt cgtaaagaac cagaaaaagt tgctaaagtt 1380
   aaagaattat atgaggctgt aggtatgaga gccgcctttc aacaatacga agaaagtagt 1440
   taccgtcgtc ttcaagagtt aattgagaaa cattctaatc gtttaccaaa agaaattttc 1500
   ttaggtttag ctcagaaaat atacaaacgt caaaaaggta ccggtgaaaa cttatacttt 1560
   caaggctcag gtggcggtgg aagtgattac aaagatgatg atgataaagg aaccggttaa 1620
   tctagactta gcttcaacta actctagctc aaacaactaa ttttttttta aactaaaata 1680
   aatctggtta accatacctg gtttatttta gtttagttta tacacacttt tcatatatat 1740
   atacttaata gctaccatag gcagttggca ggacgtcccc ttacgggaca aatgtattta 1800
   ttgttgcctg ccaactgcct aatataaata ttagtggacg tccccttccc cttacgggca 1860
   agtaaactta gggattttaa tgctccgtta ggaggcaaat aaattttagt ggcagttgcc 1920
   tcgcctatcg gctaacaagt tccttcggag tatataaata tcctgccaac tgccgatatt 1980
   tatatactag gcagtggcgg taccactcga cactagt 2017
<210> 20
   <211> 6521
   <212> DNA
   <213> Artificial Sequence
<220>
   <223> Description of Artificial Sequence: Synthetic polynucleotide
<400> 20
   tcgtgtagat aactacgata cgggagggct taccatctgg ccccagtgct gcaatgatac 60
   cgcgagaccc acgctcaccg gctccagatt tatcagcaat aaaccagcca gccggaaggg 120
   ccgagcgcag aagtggtcct gcaactttat ccgcctccat ccagtctatt aattgttgcc 180
   gggaagctag agtaagtagt tcgccagtta atagtttgcg caacgttgtt gccattgcta 240
   caggcatcgt ggtgtcacgc tcgtcgtttg gtatggcttc attcagctcc ggttcccaac 300
   gatcaaggcg agttacatga tcccccatgt tgtgcaaaaa agcggttagc tccttcggtc 360
   ctccgatcgt tgtcagaagt aagttggccg cagtgttatc actcatggtt atggcagcac 420
   tgcataattc tcttactgtc atgccatccg taagatgctt ttctgtgact ggtgagtact 480
   caaccaagtc attctgagaa tagtgtatgc ggcgaccgag ttgctcttgc ccggcgtcaa 540
   tacgggataa taccgcgcca catagcagaa ctttaaaagt gctcatcatt ggaaaacgtt 600
   cttcggggcg aaaactctca aggatcttac cgctgttgag atccagttcg atgtaaccca 660
   ctcgtgcacc caactgatct tcagcatctt ttactttcac cagcgtttct gggtgagcaa 720
   aaacaggaag gcaaaatgcc gcaaaaaagg gaataagggc gacacggaaa tgttgaatac 780
   tcatactctt cctttttcaa tattattgaa gcatttatca gggttattgt ctcatgagcg 840
   gatacatatt tgaatgtatt tagaaaaata aacaaatagg ggttccgcgc acatttcccc 900
   gaaaagtgcc acctgacgtc taagaaacca ttattatcat gacattaacc tataaaaata 960
   ggcgtatcac gaggcccttt cgtctcgcgc gtttcggtga tgacggtgaa aacctctgac 1020
   acatgcagct cccggagacg gtcacagctt gtctgtaagc ggatgccggg agcagacaag 1080
   cccgtcaggg cgcgtcagcg ggtgttggcg ggtgtcgggg ctggcttaac tatgcggcat 1140
   cagagcagat tgtactgaga gtgcaccata ggcggccgcg gcgcgccgtt ccggatctgc 1200
   atcctgcgat gcagatccgg aacataatgg tgcagggcgc tgacttccgc gtttccagac 1260
   tttacgaaac acggaaaccg aagaccattc atgttgttgc tcaggtcgca gacgttttgc 1320
   agcagcagtc gcttcacgtt cgctcgcgta tcggtgattc attctgctaa ccagtaaggc 1380
   aaccccgcca gcctagccgg gtcctcaacg acaggagcac gatcatgcgc acccgtggcc 1440
   aggacccaac gctgcccgag atgcgccgcg tgcggctgct ggagatggcg gacgcgatgg 1500
   atatgttctg ccaagggttg gtttgcgcat tcacagttct ccgcaagaat tgattggctc 1560
   caattcttgg agtggtgaat ccgttagcga ggtgccgccg gcttccattc aggtcgaggt 1620
   ggcccggctc catgcaccgc gacgcaacgc ggggaggcag acaaggtata gggcggcgcc 1680
   tacaatccat gccaacccgt tccatgtgct cgccgaggcg gcataaatcg ccgtgacgat 1740
   cagcggtcca atgatcgaag ttaggctggt aagagccgcg agcgatcctt gaagctgtcc 1800
   ctgatggtcg tcatctacct gcctggacag catggcctgc aacgcgggca tcccgatgcc 1860
   gccggaagcg agaagaatca taatggggaa ggccatccag cctcgcgtcg cgaacgccag 1920
   caagacgtag cccagcgcgt cggccgccat gccggcgata atggcctgct tctcgccgaa 1980
   acgtttggtg gcgggaccag tgacgaaggc ttgagcgagg gcgtgcaaga ttccgaatac 2040
   cgcaagcgac aggccgatca tcgtcgcgct ccagcgaaag cggtcctcgc cgaaaatgac 2100
   ccagagcgct gccggcacct gtcctacgag ttgcatgata aagaagacag tcataagtgc 2160
   ggcgacgata gtcatgcccc gcgcccaccg gaaggagctg actgggttga aggctctcaa 2220
   gggcatcggt cgagcttgac attgtaggac gtttaaacat taccctgtta tccctaggat 2280
   cctacgtata catactccga aggaggacaa atttatttat tgtggtacaa taaataagtg 2340
   gtacaataaa taaattgtat gtaaacccct tccccttcgg gacgtcccct tacgggaata 2400
   taaatattag tggcagttgc ctgccaacaa atttatttat tgtattaaca taggcagtgg 2460
   cggtaccact gccactggcg tcctaatata aatattgggc aactaaagtt tatcgcagta 2520
   ttaacatagg cagtggcggt accactgcca ctggcgtcct ccttcggagt atgtaaacct 2580
   gctaccgcag caaataaatt ttattctatt ttaatactac aatatttaga ttcccgttag 2640
   gggataggcc aggcaattgt cactggcgtc atagtatatc aatattgtaa cagattgaca 2700
   ccctttaagt aaacattttt tttaggattc atatgaaatt aaatggatat ttggtacatt 2760
   taattccaca aaaatgtcca atacttaaaa tacaaaatta aaagtattag ttgtaaactt 2820
   gactaacatt ttaaatttta aattttttcc taattatata ttttacttgc aaaatttata 2880
   aaaattttat gcatttttat atcataataa taaaaccttt attcatggtt tataatataa 2940
   taattgtgat gactatgcac aaagcagttc tagtcccata tatataacta tatataaccc 3000
   gtttaaagat ttatttaaaa atatgtgtgt aaaaaatgct tatttttaat tttattttat 3060
   ataagttata atattaaata cacaatgatt aaaattaaat aataataaat ttaacgtaac 3120
   gatgagttgt ttttttattt tggagataca cgcaatgaca attgcgatcg gtacatatca 3180
   agagaaacgc acatggttcg atgacgctga tgactggctt cgtcaagacc gtttcgtatt 3240
   cgtaggttgg tcaggtttat tactattccc ttgtgcttac tttgcaactc cggtccggcg 3300
   gccgcctcga gacgcttacc agacaaggca gttttttcat tcttttaaag caggcagttc 3360
   tgaaggggaa aagggactgc ctactgcggt cctaggtaaa tacattttta tgcaatttat 3420
   ttcttgtgct agtaggtttc tatactcaca agaagcaacc ccttgacgag agaacgttat 3480
   cctcagagta tttataatcc tgagagggaa tgcactgaag aatattttcc ttatttttta 3540
   cagaaagtaa ataaaatagc gctaataacg cttaattcat ttaatcaatt atggcaacag 3600
   gaacttctaa agctaaacca tcaaaagtaa attcagactt ccaagaacct ggtttagtta 3660
   caccattagg tactttatta cgtccactta actcagaagc aggtaaagta ttaccaggct 3720
   ggggtacaac tgttttaatg gctgtattta tccttttatt tgcagcattc ttattaatca 3780
   ttttagaaat ttacaacagt tctttaattt tagatgacgt ttctatgagt tgggaaactt 3840
   tagctaaagt ttcttaattt tatttaacac aaacataaaa tataaaactg tttgttaagg 3900
   ctagctgcta agtcttcttt tcgctaaggt aaactaagca actcaaccat atttatattc 3960
   ggcagtggca ccgccactgc cactggcctt ccgttaagat aaacgcgtta atagctcact 4020
   tttctttaaa tttaattttt aatttaaagg tgtaagcaaa ttgcctgacg agagatccac 4080
   ttaaaggatg acagtggcgg gctactgcct acttccctcc gggataaaat ttatttgaaa 4140
   aacgttagtt acttcctaac ggagcattga catccccata tttatattag gacgtcccct 4200
   tcgtcgacat taccctgtta tccctaggcc ggcctaagaa accattatta tcatgacatt 4260
   aacctataaa aataggcgta tcacgaggcc ctttcgtctt caagaaattc ggtcgaaaaa 4320
   agaaaaggag agggccaaga gggagggcat tggtgactat tgagcacgtg agtatacgtg 4380
   attaagcaca caaaggcagc ttggagtatg tctgttatta atttcacagg tagttctggt 4440
   ccattggtga aagtttgcgg cttgcagagc acagaggccg cagaatgtgc tctagattcc 4500
   gatgctgact tgctgggtat tatatgtgtg cccaatagaa agagaacaat tgacccggtt 4560
   attgcaagga aaatttcaag tcttgtaaaa gcatataaaa atagttcagg cactccgaaa 4620
   tacttggttg gcgtgtttcg taatcaacct aaggaggatg ttttggctct ggtcaatgat 4680
   tacggcattg atatcgtcca actgcatgga gatgagtcgt ggcaagaata ccaagagttc 4740
   ctcggtttgc cagttattaa aagactcgta tttccaaaag actgcaacat actactcagt 4800
   gcagcttcac agaaacctca ttcgtttatt cccttgtttg attcagaagc aggtgggaca 4860
   ggtgaacttt tggattggaa ctcgatttct gactgggttg gaaggcaaga gagccccgaa 4920
   agcttacatt ttatgttagc tggtggactg acgccagaaa atgttggtga tgcgcttaga 4980
   ttaaatggcg ttattggtgt tgatgtaagc ggaggtgtgg agacaaatgg tgtaaaagac 5040
   tctaacaaaa tagcaaattt cgtcaaaaat gctaagaaat aggttattac tgagtagtat 5100
   ttatttaagt attgtttgtg cacttgcctg caggcctttt gaaaagcaag cataaaagat 5160
   ctaaacataa aatctgtaaa ataacaagat gtaaagataa tgctaaatca tttggctttt 5220
   tgattgattg tacaggaaaa tatacatcgt taattaagcg gccgcgagct tggcgtaatc 5280
   atggtcatag ctgtttcctg tgtgaaattg ttatccgctc acaattccac acaacatacg 5340
   agccggaagc ataaagtgta aagcctgggg tgcctaatga gtgagctaac tcacattaat 5400
   tgcgttgcgc tcactgcccg ctttccagtc gggaaacctg tcgtgccagc tgcattaatg 5460
   aatcggccaa cgcgcgggga gaggcggttt gcgtattggg cgctcttccg cttcctcgct 5520
   cactgactcg ctgcgctcgg tcgttcggct gcggcgagcg gtatcagctc actcaaaggc 5580
   ggtaatacgg ttatccacag aatcagggga taacgcagga aagaacatgt gagcaaaagg 5640
   ccagcaaaag gccaggaacc gtaaaaaggc cgcgttgctg gcgtttttcc ataggctccg 5700
   cccccctgac gagcatcaca aaaatcgacg ctcaagtcag aggtggcgaa acccgacagg 5760
   actataaaga taccaggcgt ttccccctgg aagctccctc gtgcgctctc ctgttccgac 5820
   cctgccgctt accggatacc tgtccgcctt tctcccttcg ggaagcgtgg cgctttctca 5880
   tagctcacgc tgtaggtatc tcagttcggt gtaggtcgtt cgctccaagc tgggctgtgt 5940
   gcacgaaccc cccgttcagc ccgaccgctg cgccttatcc ggtaactatc gtcttgagtc 6000
   caacccggta agacacgact tatcgccact ggcagcagcc actggtaaca ggattagcag 6060
   agcgaggtat gtaggcggtg ctacagagtt cttgaagtgg tggcctaact acggctacac 6120
   tagaagaaca gtatttggta tctgcgctct gctgaagcca gttaccttcg gaaaaagagt 6180
   tggtagctct tgatccggca aacaaaccac cgctggtagc ggtggttttt ttgtttgcaa 6240
   gcagcagatt acgcgcagaa aaaaaggatc tcaagaagat cctttgatct tttctacggg 6300
   gtctgacgct cagtggaacg aaaactcacg ttaagggatt ttggtcatga gattatcaaa 6360
   aaggatcttc acctagatcc ttttaaatta aaaatgaagt tttaaatcaa tctaaagtat 6420
   atatgagtaa acttggtctg acagttacca atgcttaatc agtgaggcac ctatctcagc 6480
   gatctgtcta tttcgttcat ccatagttgc ctgactcccc g 6521
<210> 21
   <211> 46
   <212> DNA
   <213> Artificial Sequence
<220>
   <223> Description of Artificial sequence: Synthetic primer
<400> 21
   ttggttgcgg ccgcttaatt aacgatgtat attttcctgt acaatc 46
<210> 22
   <211> 58
   <212> DNA
   <213> Artificial Sequence
<220>
   <223> Description of Artificial Sequence: Synthetic primer
<400> 22
   gtttaaacat taccctgtta tccctaggcc ggcctaagaa accattatta tcatgaca 58
<210> 23
   <211> 58
   <212> DNA
   <213> Artificial Sequence
<220>
   <223> Description of Artificial Sequence: Synthetic primer
<400> 23
   ggccggccta gggataacag ggtaatgttt aaacgtccta caatgtcaag ctcgaccg 58
<210> 24
   <211> 46
   <212> DNA
   <213> Artificial Sequence
<220>
   <223> Description of Artificial sequence: Synthetic primer
<400> 24
   ttggttgcgg ccgcggcgcg ccgttccgga tctgcatcgc aggatg 46
<210> 25
   <211> 54
   <212> DNA
   <213> Artificial Sequence
<220>
<220>
   <223> Description of Artificial Sequence: Synthetic primer
<400> 25
   ttggtttagg gataacaggg taatgtcgac aacatgctaa gtttacttgc ccga 54
<210> 26
   <211> 52
   <212> DNA
   <213> Artificial Sequence
<220>
   <223> Description of Artificial sequence: Synthetic primer
<400> 26
   actccggtcc ggcggccgcc tcgagacgac ttgtccgctt catcagacac gg 52
<210> 27
   <211> 52
   <212> DNA
   <213> Artificial Sequence
<220>
   <223> Description of Artificial sequence: Synthetic primer
<400> 27
   cgtctcgagg cggccgccgg accggagttg caaagtaagc acaagggaat ag 52
<210> 28
   <211> 59
   <212> DNA
   <213> Artificial Sequence
<220>
   <223> Description of Artificial Sequence: Synthetic primer
<400> 28
   tggttattac cctgttatcc ctaggatcct acgtatacat actccgaagg aggacaaat 59
<210> 29
   <211> 48
   <212> DNA
   <213> Artificial Sequence
<220>
   <223> Description of Artificial sequence: Synthetic primer
<400> 29
   cgtctcgagg cggccgccgg accggagtca tccgccccat ctaataaa 48
<210> 30
   <211> 56
   <212> DNA
   <213> Artificial Sequence
<220>
   <223> Description of Artificial Sequence: Synthetic primer
<400> 30
   ttggttatta ccctgttatc cctaggatcc tacgtacagt ggcggtacca caataa 56
<210> 31
   <211> 53
   <212> DNA
   <213> Artificial Sequence
<220>
   <223> Description of Artificial sequence: Synthetic primer
<400> 31
   ttggtttagg gataacaggg taatgtcgac gtatgtaaac cccttcgggc aac 53
<210> 32
   <211> 50
   <212> DNA
   <213> Artificial Sequence
<220>
   <223> Description of Artificial sequence: Synthetic primer
<400> 32
   actccggtcc ggcggccgcc tcgagacgcc gtaaacagat aggaatgacg 50
<210> 33
   <211> 48
   <212> DNA
   <213> Artificial Sequence
<220>
   <223> Description of Artificial sequence: Synthetic primer
<400> 33
   cgtctcgagg cggccgccgg accggagtga atccaggcat cttgggta 48
<210> 34
   <211> 56
   <212> DNA
   <213> Artificial Sequence
<220>
   <223> Description of Artificial sequence: Synthetic primer
<400> 34
   ttggttatta ccctgttatc cctaggatcc tacgtaggga aaggtgcaac tacctg 56
<210> 35
   <211> 50
   <212> DNA
   <213> Artificial Sequence
<220>
   <223> Description of Artificial Sequence: Synthetic primer
<400> 35
   ttggtttagg gataacaggg taatgtcgac aacatgcttg gcactggttt 50
<210> 36
   <211> 54
   <212> DNA
   <213> Artificial Sequence
<220>
   <223> Description of Artificial sequence: Synthetic primer
<400> 36
   actccggtcc ggcggccgcc tcgagacgtg gtaatttatt tggtaatttg gtca 54
<210> 37
   <211> 48
   <212> DNA
   <213> Artificial Sequence
<220>
   <223> Description of Artificial sequence: Synthetic primer
<400> 37
   cgtctcgagg cggccgccgg accggagtaa ccaaccattg tgtgacca 48
<210> 38
   <211> 56
   <212> DNA
   <213> Artificial Sequence
<220>
   <223> Description of Artificial sequence: Synthetic primer
<400> 38
   ttggttatta ccctgttatc cctaggatcc tacgtaagag tacgggatgt gggatg 56
<210> 39
   <211> 53
   <212> DNA
   <213> Artificial Sequence
<220>
   <223> Description of Artificial sequence: Synthetic primer
<400> 39
   ttggtttagg gataacaggg taatgtcgac ccataagtaa actccctttt gga 53
<210> 40
   <211> 50
   <212> DNA
   <213> Artificial Sequence
<220>
   <223> Description of Artificial Sequence: Synthetic primer
<400> 40
   actccggtcc ggcggccgcc tcgagacgta aaattgtttg tgtggtctgg 50
<210> 41
   <211> 52
   <212> DNA
   <213> Artificial Sequence
<220>
   <223> Description of Artificial Sequence: Synthetic primer
<400> 41
   cgtctcgagg cggccgccgg accggagtaa atgtaacttt tgttgtcgat cc 52
<210> 42
   <211> 60
   <212> DNA
   <213> Artificial Sequence
<220>
   <223> Description of Artificial sequence: Synthetic primer
<400> 42
   ttggttatta ccctgttatc cctaggatcc tacgtagggt aaataaattt tagtggacgt 60
<210> 43
   <211> 54
   <212> DNA
   <213> Artificial Sequence
<220>
   <223> Description of Artificial Sequence: Synthetic primer
<400> 43
   ttggtttagg gataacaggg taatgtcgac gaaggggacg tcctaatata aata 54
<210> 44
   <211> 50
   <212> DNA
   <213> Artificial Sequence
<220>
   <223> Description of Artificial Sequence: Synthetic primer
<400> 44
   actccggtcc ggcggccgcc tcgagacgct taccagacaa ggcagttttt 50
<210> 45
   <211> 48
   <212> DNA
   <213> Artificial Sequence
<220>
   <223> Description of Artificial sequence: Synthetic primer
<400> 45
   cgtctcgagg cggccgccgg accggagtgc tgctggttat gcagttga 48
<210> 46
   <211> 56
   <212> DNA
   <213> Artificial Sequence
<220>
   <223> Description of Artificial Sequence: Synthetic primer
<400> 46
   ttggttatta ccctgttatc cctaggatcc tacgtagagg accaaatcct gcgtta 56
<210> 47
   <211> 50
   <212> DNA
   <213> Artificial Sequence
<220>
   <223> Description of Artificial Sequence: Synthetic primer
<400> 47
   ttggtttagg gataacaggg taatgtcgac tttgcttgcc tacaagagca 50
<210> 48
   <211> 48
   <212> DNA
   <213> Artificial Sequence
<220>
   <223> Description of Artificial sequence: Synthetic primer
<400> 48
   actccggtcc ggcggccgcc tcgagacgtt gcattaaaat ccggaagg 48
<210> 49
   <211> 36
   <212> DNA
   <213> Artificial Sequence
<220>
   <223> Description of Artificial Sequence: Synthetic primer
<400> 49
   ttggttcccg ggtcgcgcgt ttcggtgatg acggtg 36
<210> 50
   <211> 42
   <212> DNA
   <213> Artificial Sequence
<220>
   <223> Description of Artificial sequence: Synthetic primer
<400> 50
   ttggttgtcg acccgcggtg atgcggtatt ttctccttac gc 42
<210> 51
   <211> 36
   <212> DNA
   <213> Artificial Sequence
<220>
   <223> Description of Artificial sequence: Synthetic primer
<400> 51
   ttggttcccg ggtcctgatg cggtattttc tcctta 36
<210> 52
   <211> 36
   <212> DNA
   <213> Artificial Sequence
<220>
   <223> Description of Artificial sequence: Synthetic primer
<400> 52
   ttggttcccg ggtcgcgcgt ttcggtgatg acggtg 36
<210> 53
   <211> 36
   <212> DNA
   <213> Artificial Sequence
<220>
   <223> Description of Artificial sequence: Synthetic primer
<400> 53
   ttggttcccg ggaagcttgc atgcctgcag gtcgat 36
<210> 54
   <211> 36
   <212> DNA
   <213> Artificial Sequence
<220>
   <223> Description of Artificial Sequence: Synthetic primer
<400> 54
   ttggttcccg ggagcagttg ctttctccta tgggaa 36
<210> 55
   <211> 36
   <212> DNA
   <213> Artificial Sequence
<220>
   <223> Description of Artificial Sequence: Synthetic primer
<400> 55
   ttggttcccg ggttaggtct agagatctgt ttagct 36
<210> 56
   <211> 50
   <212> DNA
   <213> Artificial Sequence
<220>
   <223> Description of Artificial sequence: Synthetic primer
<400> 56
   ttggttgtcg acggccggcc actagttcgc gcgtttcggt gatgacggtg 50
<210> 57
   <211> 50
   <212> DNA
   <213> Artificial Sequence
<220>
   <223> Description of Artificial Sequence: Synthetic primer
<400> 57
   ttggttgtcg acggccggcc actagttgat gcggtatttt ctccttacgc 50
<210> 58
   <211> 50
   <212> DNA
   <213> Artificial Sequence
<220>
   <223> Description of Artificial sequence: Synthetic primer
<400> 58
   ttggttgtcg acggccggcc actagtgatc ctcgagagat cttatgtatg 50
<210> 59
   <211> 50
   <212> DNA
   <213> Artificial Sequence
<220>
   <223> Description of Artificial Sequence: Synthetic primer
<400> 59
   ttggttgtcg acggccggcc actagttaca ataaaccaag atgaagctgc 50
<210> 60
   <211> 50
   <212> DNA
   <213> Artificial Sequence
<220>
   <223> Description of Artificial Sequence: Synthetic primer
<400> 60
   ttggttgtcg acggccggcc actagttatt aagggttctc gagagctcgt 50
<210> 61
   <211> 39
   <212> DNA
   <213> Artificial Sequence
<220>
   <223> Description of Artificial sequence: Synthetic primer
<400> 61
   ttggttcccg gggatatcaa tacattcaaa tatgtatcc 39
<210> 62
   <211> 41
   <212> DNA
   <213> Artificial Sequence
<220>
   <223> Description of Artificial Sequence: Synthetic primer
<400> 62
   ttggttcccg gggatatcat ccttttaaat taaaaatgaa g 41
<210> 63
   <211> 42
   <212> DNA
   <213> Artificial Sequence
<220>
   <223> Description of Artificial sequence: Synthetic primer
<400> 63
   ttggttcccg gggatatctt ctcatgtttg acagcttatc at 42
<210> 64
   <211> 42
   <212> DNA
   <213> Artificial sequence
<220>
   <223> Description of Artificial Sequence: Synthetic primer
<400> 64
   aaccaacccg gggatatcat gttctgccaa gggttggttt gc 42
<210> 65
   <211> 42
   <212> DNA
   <213> Artificial Sequence
<220>
   <223> Description of Artificial sequence: Synthetic primer
<400> 65
   ttggttcccg gggatatccc caatccaggt cctgaccgtt ct 42
<210> 66
   <211> 42
   <212> DNA
   <213> Artificial Sequence
<220>
   <223> Description of Artificial sequence: synthetic primer
<400> 66
   aaccaacccg gggatatctc acttattcag gcgtagcaac ca 42
<210> 67
   <211> 42
   <212> DNA
   <213> Artificial Sequence
<220>
   <223> Description of Artificial Sequence: Synthetic primer
<400> 67
   ttggttcccg gggatatctt gccgggtgac gcacaccgtg ga 42
<210> 68
   <211> 42
   <212> DNA
   <213> Artificial Sequence
<220>
   <223> Description of Artificial sequence: Synthetic primer
<400> 68
   aaccaacccg gggatatctg ccgatggccg cggcgttgtg ac 42
<210> 69
   <211> 23
   <212> DNA
   <213> Artificial Sequence
<220>
   <223> Description of Artificial Sequence: Synthetic primer
<400> 69
   catactactc agtgcagctt cac 23
<210> 70
   <211> 25
   <212> DNA
   <213> Artificial Sequence
<220>
   <223> Description of Artificial Sequence: Synthetic primer
<400> 70
   gtgaaggagc atgttcggca cacag 25
<210> 71
   <211> 25
   <212> DNA
   <213> Artificial Sequence
<220>
   <223> Description of Artificial sequence: Synthetic primer
<400> 71
   ctgtgtgccg aacatgctcc ttcac 25
<210> 72
   <211> 24
   <212> DNA
   <213> Artificial Sequence
<220>
   <223> Description of Artificial sequence: Synthetic primer
<400> 72
   ttgtcatatt actagttggt gtgg 24
<210> 73
   <211> 24
   <212> DNA
   <213> Artificial Sequence
<220>
   <223> Description of Artificial sequence: Synthetic primer
<400> 73
   ggtcggcgac aactcaatcg acag 24
<210> 74
   <211> 23
   <212> DNA
   <213> Artificial Sequence
<220>
   <223> Description of Artificial sequence: Synthetic primer
<400> 74
   caacggatgt tttattgcct ttg 23
<210> 75
   <211> 24
   <212> DNA
   <213> Artificial Sequence
<220>
   <223> Description of Artificial Sequence: Synthetic primer
<400> 75
   gcaaggattt tcttaacttc ttcg 24
<210> 76
   <211> 25
   <212> DNA
   <213> Artificial Sequence
<220>
   <223> Description of Artificial Sequence: Synthetic primer
<400> 76
   atgaagtcct ttgttactgt gccgc 25
<210> 77
   <211> 23
   <212> DNA
   <213> Artificial Sequence
<220>
   <223> Description of Artificial sequence: Synthetic primer
<400> 77
   caggaattcg ctaaagcctg tgg 23
<210> 78
   <211> 24
   <212> DNA
   <213> Artificial Sequence
<220>
   <223> Description of Artificial Sequence: Synthetic primer
<400> 78
   gagaataaaa gtctaagatg tgcg 24
<210> 79
   <211> 24
   <212> DNA
   <213> Artificial Sequence
<220>
   <223> Description of Artificial sequence: Synthetic primer
<400> 79
   gacatatatg aacatcgcgg agtg 24
<210> 80
   <211> 24
   <212> DNA
   <213> Artificial Sequence
<220>
   <223> Description of Artificial Sequence: Synthetic primer
<400> 80
   cttcaatgca tcagcactac caac 24
<210> 81
   <211> 23
   <212> DNA
   <213> Artificial Sequence
<220>
   <223> Description of Artificial sequence: Synthetic primer
<400> 81
   ggtcagattg ccctgtcgtt ctc 23
<210> 82
   <211> 24
   <212> DNA
   <213> Artificial Sequence
<220>
   <223> Description of Artificial Sequence: Synthetic primer
<400> 82
   cagtttcatt tgatgctcga tgag 24
<210> 83
   <211> 25
   <212> DNA
   <213> Artificial Sequence
<220>
   <223> Description of Artificial sequence: Synthetic primer
<400> 83
   ggtagaattg tccgttagtt gttta 25
<210> 84
   <211> 25
   <212> DNA
   <213> Artificial Sequence
<220>
   <223> Description of Artificial Sequence: Synthetic primer
<400> 84
   aacagacgta gtaagaacca ccagc 25
<210> 85
   <211> 25
   <212> DNA
   <213> Artificial Sequence
<220>
   <223> Description of Artificial sequence: Synthetic primer
<400> 85
   ctccagaagc gttaatgtct ggctt 25
<210> 86
   <211> 24
   <212> DNA
   <213> Artificial Sequence
<220>
   <223> Description of Artificial sequence: Synthetic primer
<400> 86
   cgaccatcag ggacagcttc aagg 24
<210> 87
   <211> 20
   <212> DNA
   <213> Artificial Sequence
<220>
   <223> Description of Artificial Sequence: Synthetic primer
<400> 87
   ggtctccaga acttgctgct 20
<210> 88
   <211> 20
   <212> DNA
   <213> Artificial Sequence
<220>
   <223> Description of Artificial sequence: Synthetic primer
<400> 88
   cctatcccct aacgggaatc 20
<210> 89
   <211> 22
   <212> DNA
   <213> Artificial Sequence
<220>
   <223> Description of Artificial Sequence: Synthetic primer
<400> 89
   agattttgtg taatgccgaa gt 22
<210> 90
   <211> 20
   <212> DNA
   <213> Artificial Sequence
<220>
   <223> Description of Artificial sequence: Synthetic primer
<400> 90
   tgccgtaatc attgaccaga 20
<210> 91
   <211> 20
   <212> DNA
   <213> Artificial Sequence
<220>
   <223> Description of Artificial Sequence: Synthetic primer
<400> 91
   ggtgcgtaaa atcgttggat 20
<210> 92
   <211> 20
   <212> DNA
   <213> Artificial Sequence
<220>
   <223> Description of Artificial Sequence: Synthetic primer
<400> 92
   tttttcggcg tacaaaggac 20
<210> 93
   <211> 20
   <212> DNA
   <213> Artificial Sequence
<220>
   <223> Description of Artificial sequence: Synthetic primer
<400> 93
   ctcgcctatc ggctaacaag 20
<210> 94
   <211> 20
   <212> DNA
   <213> Artificial Sequence
<220>
   <223> Description of Artificial Sequence: Synthetic primer
<400> 94
   cacaagaagc aaccccttga 20
<210> 95
   <211> 23
   <212> DNA
   <213> Artificial Sequence
<220>
   <223> Description of Artificial Sequence: Synthetic primer
<400> 95
   aaatttaacg taacgatgag ttg 23
<210> 96
   <211> 23
   <212> DNA
   <213> Artificial Sequence
<220>
   <223> Description of Artificial sequence: Synthetic primer
<400> 96
   gcactacctg atgaaaaata acc 23
<210> 97
   <211> 23
   <212> DNA
   <213> Artificial Sequence
<220>
   <223> Description of Artificial Sequence: Synthetic primer
<400> 97
   ggaaggggac gtaggtacat aaa 23
<210> 98
   <211> 23
   <212> DNA
   <213> Artificial Sequence
<220>
   <223> Description of Artificial Sequence: Synthetic primer
<400> 98
   ttagaacgtg ttttgttccc aat 23
<210> 99
   <211> 22
   <212> DNA
   <213> Artificial Sequence
<220>
   <223> Description of Artificial Sequence: Synthetic primer
<400> 99
   cgttcttctg agaaatggct ta 22

## Description

### CROSS-REFERENCE

This application claims priority to and benefit of U.S. Provisional Application No. 60/978,024 (filed October 5, 2007), which application is incorporated herein by reference.

### BACKGROUND OF THE INVENTION

For the functional analysis of many genes, investigators need to isolate and manipulate large DNA fragments. The advent of genomics and the study of genomic regions of DNA have generated a need for vectors capable of carrying large DNA regions.

In general, two types of yeast vector systems are presently available. The first type of vector is one capable of transferring small insert DNA between yeast and bacteria. A second type of vector is a fragmenting vector which creates interstitial or terminal deletions in yeast artificial chromosomes (YACs). The small insert shuttle vectors are able to recombine with and recover homologous sequences. They are centromere-based and replicate stably and autonomously in yeast, but also contain a high-copy origin of replication for maintenance as bacterial plasmids. However, these vectors are limited by their small insert capacity. The second type of vector (also known as fragmenting vectors) has recombinogenic sequences, but is unable to transfer the recovered insert DNA to bacteria for large preparations of DNA.

Researchers use fragmentation techniques to narrow down the region of interest in YACs. However, isolating sufficient quantities of YAC DNA from agarose gels for microinjection or electroporation remains cumbersome. Purification remains a problem when the YAC comigrates with an endogenous chromosome. In addition, YACs may be chimeric or contain additional DNA regions that are not required for the particular functional study.

Types of vectors available for cloning large fragments in bacteria are cosmids, P1s and bacterial artificial chromosomes (BACs). These vectors are limited to bacteria and cannot be shuttled to yeast for modification by homologous recombination. Bacterial vectors are also limited in their use for transforming plants and algae. For example, though chloroplasts are thought to originate from the endosymbiosis of photosynthetic bacteria into eukaryotic hosts translation of chloroplasts in more complex. Adding to the complexity of genetically engineering plants and algae is the presence of multiple chloroplasts with multiple copies of the chloroplast genome. Thus, there exists a need for developing a method to express proteins from large fragments of DNA in the chloroplasts of plants and algae

### SUMMARY OF THE INVENTION

The present invention relates to compositions and methods of isolating, characterizing, and/or modifying large DNA, including entire genomes of bacteria and chloroplasts. The compositions include shuttle vectors into which target DNA may be inserted. The methods include modifying or manipulating target DNA by removing, adding or rearranging portions and introducing the modified DNA into a host.

One aspect of the present invention provides an isolated vector comprising a yeast element, a bacterial origin of replication, and at least 20 kb genomic DNA. In some vectors, the yeast element is a yeast centromere, a yeast autonomous replicating sequence, yeast auxotrophic marker, or a combination thereof. The DNA may be from a non-vascular photosynthetic organism, for example a macroalgae, microalgae, *Ch. vulgaris, C. reinhardtii, D. salina*, *S. quadricanda* or *H. pluvalis.* In some embodiments, the genomic DNA is modified, for example by insertion of a heterologous or homologous polynucleotide, deletion of one or more nucleic acid bases, mutation of one or more nucleic acid bases, rearrangement of one or more polynucleotides, or a combination thereof. In some instances, the modification is synthetic. Vectors of the present invention, when transformed into a host cell, may result in production of a product not naturally produced by the host cell. Some examples of such products include biomass-degrading enzymes, a fatty acids, terpenes or terpenoids. In some host cells, expression of the vector results in an increase production of a product naturally produced by said host cell, for example, a biomass-degrading enzyme, a terpene or a terpenoid. The vectors of the present invention may further comprise one or more selection markers, for example, a yeast marker, a yeast antibiotic resistance marker, a yeast auxotrophic marker, a bacterial marker, a bacterial antibiotic resistance marker, a bacterial auxotrophic marker, an algae marker, an algae antibiotic resistance marker, an algae auxotrophic marker, or a combination thereof. Vectors of the present invention may also contain chloroplast genomic DNA which comprises 1) 1-200 genes; 2) all essential chloroplast genes;and/or 3) 30-400 kb.

Also described herein is a host cell comprising the vectors described herein. Exemplary host cells may be naturally non-photosynthetic or photosynthetic and include, for example, *Saccharomyces cerevisiae, Escherichia coli*, macroalgae, microalgae, *Ch. vulgaris, C. reinhardtii, D. salina, S. quadricanda* or H. *pluvalis.*

In another aspect of the invention, a method for producing a vector is provided where the method involves inserting targeting DNA into a vector - where the vector comprises a yeast centromere, a yeast autonomous replicating sequence, and a bacterial origin of replication, transforming an organism with the vector and capturing a portion of a chloroplast genome, thus producing a vector with a portion of a chloroplast genome. In some instances, the targeting DNA is chloroplast genomic DNA. This method may be used to capture a portion of a genome which is 10-400 kb in length. In some instances, the capturing step occurs by recombination. The captured portion of a chloroplast genome may be co-transformed into an organism with a vector, thus the recombination step may occur in vivo. Organisms used to practice methods disclosed herein may be eukaryotic and/or photosynthetic. In some instances, the organism is a non-vascular photosynthetic organism, for example a macroalgae, microalgae, *Ch. vulgaris, C. reinhardtii, D. salina, S. quadricanda* or *H. pluvalis.* Organisms used to practice methods disclosed herein may also be non-photosynthetic, for example yeast. In some instances, a non-photosynthetic organism may contain exogenous chloroplast DNA. In some embodiments, an additional step of modifying a portion of a chloroplast genome is utilized. A modification may be achieved through homologous recombination. Such recombination may occur in an organism, for example a eukaryotic and/or photosynthetic organism. In some instances, the organism is a non-vascular photosynthetic organism, for example a macroalgae, microalgae, *Ch. vulgaris, C. reinhardtii, D. salina, S. quadricanda* or *H. pluvalis.* In other instances, the organism may be non-photosynthetic, for example a yeast. In embodiments with a modification step, the step may comprise addition of a polynucleotide, deletion of one or more nucleic acid bases, mutation of one or more nucleic acid bases, rearrangement or a polynucleotide, or combination thereof.

Further disclosed herein is an isolated vector comprising essential chloroplast genes, a selectable marker and a manipulation in one or more nucleic acids in the vector. In some instances, essential chloroplast genes are cloned from a non-vascular photoshynthetic organism such as macroalgae, microalgae, *Ch. vulgaris, C. reinhardtii, D. salina, S. quadricanda* or *H. pluvalis.* Essential chloroplast genes for use in the vectors described herein may be synthetic. The vectors described herein may further comprise an expression cassette, which may further comprise a region for integration into target DNA, for example organelle DNA. The vectors described herein may also contain one or more selection markers, for example, an auxotrophic marker, an antibiotic resistance marker, a chloroplast marker, or combinations thereof. In some instances, the essential chloroplast genes are those required for chloroplast function, photosynthesis, carbon fixation, production of one or more hydrocarbons, or a combination thereof. Essential chloroplast genes may comprise up to 200 genes and/or consist of up to 400 kb. In some of the vectors described herein a manipulation in one or more nucleic acids is an addition, deletion, mutation, or rearrangement. In some instances, expression of the vector in a host cell produces a product not naturally produced by said host cell. In other instances, expression of a vector of the present invention results in an increase production of a product naturally produced by said host cell. Examples of such products are biomass degrading enzymes, fatty acids, terpenes or terpenoids.

As described herein, one aspect of the present invention is an isolated chloroplast comprising a vector of the present invention. In another aspect, a host cell comprising a vector of the present invention is provided. Host cells useful in the present invention may be naturally non-photosynthetic or naturally photosynthetic. Examples of organisms useful for the present invention include *Saccharomyces cerevisiae, Escherichia coli,* macroalgae, microalgae, *Ch. vulgaris, C. reinhardtii, D. salina, S. quadricanda* or *H. pluvalis.*

In another aspect of the present invention, a method for transforming a cell or organism is provided where the method comprises inserting into said cell or organism a vector comprising all essential chloroplast genes and optionally one or more genes not naturally occurring in said cell or organism. In some embodiments, the method further comprises the step of eliminating substantially all chloroplast genomes in said cell or organism. A cell or organism useful for this method may be photosynthetic, non-photosynthetic and/or eukaryotic. A cell or organism useful for this method may be non-vascular. In some instances, the vector for use in this method may also comprise an expression cassette and the expression cassette may be capable of integrating into non-nuclear DNA. In one embodiment the one or more genes not naturally occurring in the cell or organism is a gene in the isoprenoid pathway, MVA pathway, or MEP pathway. In another embodiment, the essential chloroplast genes are those that are required for chloroplast function, photosynthesis, carbon fixation, production of one or more hydrocarbons, or a combination thereof.

Further provided herein is a method for modifying an organism comprising the steps of transforming the organism with a vector comprising one or more polynucleotides sufficient to perform chloroplast function. In some instances, a vector useful for this method further comprises a sequence for production or secretion of a compound from said organism. In some instances, the compound is an isoprenoid. In other instances, the vector comprises all essential chloroplast genes. In still other instances, the essential chloroplast genes are rearranged or mutated. An organism useful for some embodiments comprises essentially no chloroplast genome prior to transformation.

Yet another method provided herein is a method for making a product from an organism comprising the step of transforming said organism with a vector comprising at least 20 kb of genomic DNA and one or more of the following: (i) a gene not naturally occurring in said organism; (ii) a deletion in a gene naturally occurring in said organism; (iii) a rearrangement of genes naturally occurring in said organism; and (iv) a mutation in a gene naturally occurring in said organism. In some instances, the organism is naturally photosynthetic. In other instances, the additional genes encode enzymes in the isoprenoid pathway, MVA pathway, or MEP pathway. In still another embodiment, the present disclosure provides a method for transforming a cell or organism comprising inserting into said cell or organism a chloroplast and a vector comprising all essential chloroplast genes.

The present disclosure also provides a method of producing an artificial chloroplast genome comprising the steps of: (a) providing a vector comprising one or more essential chloroplast genes; (b) adding to said vector a DNA fragment; (c) transforming a cell or organism with the vector produced by step (b); and (d) determining whether chloroplast function exists with said added DNA fragment. In some instances, the added DNA fragments comprises one or more coding regions for an enzyme in the isoprenoid, MVA or MEP pathway.

The present disclosure also provides a shuttle vector comprising a chloroplast genome. A genome may be modified. Also provided herein is a vector comprising an isolated, functional chloroplast genome. A chloroplast genome useful in such a vector may be modified.

Further provided herein is a method of producing an artificial chloroplast genome comprising the steps of: (a) providing a vector comprising all essential chloroplast genes; and (b) removing, adding, mutating, or rearranging DNA from the chloroplast genome. Such a method may further comprise the steps of transforming a redacted genome into a host organism; and (d) determining chloroplast function in the host organism. In some instances, steps (b), (c), and (d) are repeated. In still other instances, the chloroplast genome is from an organism selected from the group consisting of: macroalgae, microalgae, *Ch. vulgaris, C. reinhardtii, D. salina, S. quadricanda* or *H. pluvalis.* In other instances, the host organism is selected from the group consisting of: macroalgae, microalgae, *Ch*. *vulgaris, C. reinhardtii, D. salina, S. quadricanda* or *H. pluvalis.* For some embodiments, the method may further comprise the step of removing redundant DNA from a chloroplast genome. In other embodiments, the vector comprises all or substantially all of a chloroplast genome. A chloroplast genome useful in the present invention may be cloned from a photosynthetic organism or may be a synthetic chloroplast genome. In some instances, the vector further comprises a gene not naturally occurring in the host organism, for example a gene from the isoprenoid pathway, MVA pathway, or MEP pathway.

Yet another method provided herein is a method of producing an artificial chloroplast genome comprising the steps of: (a) providing a vector comprising an entire chloroplast genome; (b) deleting a portion of said entire chloroplast genome; and (c) determining whether chloroplast function exists without said deleted portion. In another aspect of the present invention, a composition comprising an isolated and functional chloroplast genome is provided. In some instances, a composition comprises a modification to said chloroplast genome.

Further provided herein is an ex vivo vector comprising a nucleic acid comprising at least about 10% of a chloroplast genome and a manipulation in one or more nucleic acids in the vector. In some instances, the nucleic acid is cloned from a non-vascular photosynthetic organism, for example a macroalgae, microalgae, *Ch. vulgaris, C. reinhardtii, D. salina, S. quadricanda* or *H. pluvalis.* In some instances, the nucleic acid is synthetic. A vector of the present invention may further comprise an expression cassette and an expression cassette may further comprise a region for integration into target DNA. In some instances, the target DNA is organelle DNA. A vector useful in the present invention may further comprise one or more selection markers, for example an auxotrophic marker, an antibiotic resistance marker, a chloroplast marker, or combinations thereof. In some embodiments, a manipulation in one or more nucleic acids in a vector may be an addition, deletion, mutation, or rearrangement. Expression of the vector may result in production of a product not naturally produced by a host cell and/or an increase production of a product naturally produced by a host cell. Examples of some products of the present invention include a terpene, terpenoid, fatty acid, or biomass degrading enzyme.

Also provided herein is an ex vivo vector comprising a nucleic acid comprising at least about 20 kilobases of a chloroplast genome and a manipulation in one or more nucleic acids in said vector. In some instances, the nucleic acid is cloned from a non-vascular photosynthetic organism, for example a macroalgae, microalgae, *Ch. vulgaris, C. reinhardtii, D. salina, S. quadricanda* or *H. pluvalis.* In some instances, the nucleic acid is synthetic. A vector of the present invention may further comprise an expression cassette and an expression cassette may further comprise a region for integration into target DNA. In some instances, the target DNA is organelle DNA. A vector useful in the present invention may further comprise one or more selection markers, for example an auxotrophic marker, an antibiotic resistance marker, a chloroplast marker, or combinations thereof. In some embodiments, a manipulation in one or more nucleic acids in a vector may be an addition, deletion, mutation, or rearrangement. Expression of the vector may result in production of a product not naturally produced by a host cell and/or an increase production of a product naturally produced by a host cell. Examples of some products of the present invention include a terpene, terpenoid, fatty acid, or biomass degrading enzyme.

Further provided herein is a method of producing a vector containing a reconstructed genome, comprising: introducing two or more vectors into a host cell, wherein the vectors comprise fragments of a genome, recombining the vectors into a single vector comprising at least about 90% of a genome, thereby producing a vector containing a reconstructed genome. In some instances, the host cell is eukaryotic, for example, S. cerevisiae. In other instances, the genome is an organelle genome. The organelle may be a chloroplast, for example a chloroplast from an alga, particularly a microalga such as *Ch. vulgaris, C. reinhardtii, D. salina, S. quadricanda* or *H. pluvalis.* In some instances, the two or more vectors comprise a selectable marker. In other instances, at least one of said fragments is synthetic. In still other instances, a further step comprising modifying a portion of the genome is useful in this method. Such a modification may comprise an addition, deletion, mutation, or rearrangement. In other embodiments, the modification is the addition of an exogenous nucleic acid which results in the production or increased production of a terpene, terpenoid, fatty acid or biomass degrading enzyme.

### INCORPORATION BY REFERENCE

All publications and patent applications mentioned in this specification are herein incorporated by reference to the same extent as if each individual publication or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

In the figures, the following abbreviations are used: *HIS3*: yeast *HIS3* gene; *TRPI:* yeast *TRPI* gene; *URA3:* yeast *URA3* gene; *ADE2*: yeast *ADE2* gene; *LYS2*: yeast *LYS2* gene; TEL: yeast telomere; CEN: yeast centromere; ARS: autonomously replicating sequences, yeast origin of replication; 5FOA: 5-fluoroorotic acid; Kan: kanamycin resistance gene; P1 plasmid rep: P1 plasmid replicon; p1 lytic rep: p1 lytic replicon.

Figure 1 provides a general description of a hybrid vector of the present invention. Figure 1A) Vector schematic. Figure 1B) DNA shuttling between organisms.

Figure 2 is a schematic showing construction of a hybrid vector.

Figure 3 is a schematic showing sites of integration in chloroplast genome DNA. Circled numbers indicate target sites for modification. The box indicates the site targeted by the hybrid gap-filling vector

Figures 4 A, B, and C is a schematic of selectable markers for modification and/or stabilization.

Figure 5 is a schematic for introduction of hybrid vector into chloroplast genome DNA.

Figure 6 is PCR data showing integration of hybrid vector (and stabilization vector) in algae.

Figure 7 shows analysis of captured DNA. Figure 7A) Restriction digest with *Eco*RI of isolated vectors containing chloroplast(L, ladder; C, parent hybrid vector; 1, Clone 1; and 2; Clone 2). Figure 7B) Restriction digest with *Eco*RI of isolates of Clone 1 that were passaged through yeast (L, ladder; C,; 1, Clone 1; and A-M; yeast isolates). Figure 7C) Southern analysis of Clones I and 2 digested with *Eco*RI and probed with radioactive *Hind*III-digested total DNA from *C*. *reinhardtii.*

Figures 8 A and B is a schematic showing architecture of isolated ex vivo vectors containing chloroplast genome DNA.

Figure 9 shows growth of parent and transformed algae cells under various selection conditions.

Figure 10 is a schematic of restriction analysis for manipulation vectors. Figure 10A) Schematic of vector architecture. Figure 10B) Analysis of modified vector by restriction analysis with *Eco*RI

Figure 11 shows modification of a chloroplast genome to produce a biomass-degrading enzyme. Figure 11A) PCR screen of isolated transformants. Figure 11B) Endoxylanase activity from isolated transformants.

Figures 12 shows a PCR screen of isolated transformants modified to produce isoprenoids with the FPP synthase expression cassette targeted to site 3 (Figure 12A) or site 4 (Figure 12B).

Figure 13 is a schematic showing capture of a partial chloroplast genome using recombination in yeast.

Figure 14 is a schematic showing capture of an entire chloroplast genome using recombination in yeast.

Figure 15 is a schematic showing reassembly of an entire chloroplast genome using recombination in yeast.

### DETAILED DESCRIPTION OF THE INVENTION

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

Technical and scientific terms used herein have the meanings commonly understood by one of ordinary skill in the art to which the instant invention pertains, unless otherwise defined. Reference is made herein to various materials and methodologies known to those of skill in the art. Standard reference works setting forth the general principles of recombinant DNA technology include Sambrook et al., "Molecular Cloning: A Laboratory Manual", 2d ed., Cold Spring Harbor Laboratory Press, Plainview, N.Y., 1989; Kaufman et al., eds., "Handbook of Molecular and Cellular Methods in Biology and Medicine", CRC Press, Boca Raton, 1995; and McPherson, ed., "Directed Mutagenesis: A Practical Approach", IRL Press, Oxford, 1991. Standard reference literature teaching general methodologies and principles of yeast genetics useful for selected aspects of the invention include: Sherman et al. "Laboratory Course Manual Methods in Yeast Genetics", Cold Spring Harbor Laboratory, Cold Spring Harbor, N.Y., 1986 and Guthrie et al., "Guide to Yeast Genetics and Molecular Biology", Academic, New York, 1991.

Any suitable materials and/or methods known to those of skill can be utilized in carrying out the instant invention. Materials and/or methods for practicing the instant invention are described. Materials, reagents and the like to which reference is made in the following description and examples are obtainable from commercial sources, unless otherwise noted. This invention teaches methods and describes tools for capturing and modifying large pieces of DNA. It is especially useful for modifying genomic DNA, including the whole genome of an organism or organelle, or a part thereof. Novel prophetic uses of the invention are also described. The invention relates to the manipulation and delivery of large nucleic acids. The invention further relates to recombinational cloning vectors and systems and to methods of using the same.

Contemporary methods for genetically engineering genomes (e.g., chloroplast genomes) are time intensive (> 1 month) and allow for only a limited number of manipulations at a time. If multiple modifications to a target genome are desired, the process must be iterated, further increasing the time required to generate a desired strain. Because metabolic engineering and/or synthetic biology require numerous modifications to a genome, these technologies are not amenable to rapid introduction of modifications to a genome. Thus, a new technology that allows for multiple modification of the chloroplast genome in a short amount of time will enable the application of metabolic engineering and/or synthetic biology to chloroplast genomes. The disclosure herein describes such technology.

The instant invention provides a versatile, recombinational approach to the capture, cloning, and manipulation of large nucleic acids from target cells and organelles (e.g., chloroplasts). One aspect of the present invention provides a recombinational cloning system. More specifically, the invention provides vectors, which in some embodiments, rely on homologous recombination technologies to mediate the isolation and manipulation of large nucleic acid segments. Another aspect of the present invention provides methods for using such recombinational cloning vectors to clone, to manipulate and to deliver large nucleic acids to target cells and/or organelles such as chloroplasts.

In one preferred embodiment, homologous recombination is performed in vitro. In a particularly preferred embodiment of the invention, homologous recombination is performed in vivo. In a more preferred embodiment, homologous recombination occurs in an algae cell. In another more preferred embodiment, homologous recombination occurs in a yeast cell. In one preferred embodiment, homologous recombination occurs in *Saccharomyces cerevisiae or Saccharomyces pombe.* In yeast, the combination of efficient recombination processes and the availability of numerous selectable markers provides for rapid and complex engineering of target DNA sequences. Once all of the modifications are made to an ex vivo vector containing chloroplast genome DNA, the entire vector can be introduced into a chloroplast in a single transformation step. Thus, employing yeast technology will enable the application of metabolic engineering and/or synthetic biology to chloroplast genomes. One aspect of the present invention provides an isolated vector comprising a yeast element, a bacterial origin of replication, and at least 20 kb genomic DNA. In some vectors, the yeast element is a yeast centromere, a yeast autonomous replicating sequence, or a combination thereof. The DNA may be from a non-vascular photosynthetic organism, for example a macroalgae, microalgae, *Ch. vulgaris, C. reinhardtii, D. salina, S. quadricanda* or *H. pluvalis.* In some embodiments, the genomic DNA is modified, for example by insertion of a heterologous or homologous polynucleotide, deletion of one or more nucleic acid bases, mutation of one or more nucleic acid bases, rearrangement of one or more polynucleotides, or a combination thereof. In some instances, the modification is synthetic. Vectors of the present invention, when transformed into a host cell, may result in production of a product not naturally produced by the host cell. Some examples of such products include biomass-degrading enzymes, a fatty acids, terpenes or terpenoids. In some host cells, expression of the vector results in an increase production of a product naturally produced by said host cell, for example, a biomass-degrading enzyme, a terpene or a terpenoid. The vectors of the present invention may further comprise one or more selection markers, for example, a yeast marker, a yeast antibiotic resistance marker, a bacterial marker, a bacterial antibiotic resistance marker, an algae marker, an algae antibiotic resistance marker or a combination thereof. Vectors of the present invention may also contain chloroplast genomic DNA which comprises 1) 1-200 genes; 2) all essential chloroplast genes;and/or 3) 30-400 kb.

Also described herein is a host cell comprising the vectors described herein. Exemplary host cells may be naturally non-photosynthetic or photosynthetic and include, for example, *Saccharomyces cerevisiae, Escherichia coli*, macroalgae, microalgae, *Ch. vulgaris, C. reinhardtii, D. salina, S. quadricanda* or H. *pluvalis.*

In another aspect of the invention, a method for producing a vector is provided where the method involves inserting targeting DNA into a vector - where the vector comprises a yeast centromere, a yeast autonomous replicating sequence, and a bacterial origin of replication, transforming an organism with the vector and capturing a portion of a chloroplast genome, thus producing a vector with a portion of a chloroplast genome. In some instances, the targeting DNA is chloroplast genomic DNA. This method may be used to capture a portion of a genome which is 10-400 kb in length. In some instances, the capturing step occurs by recombination. The captured portion of a chloroplast genome may be co-transformed into an organism with a vector, thus the recombination step may occur in vivo. Organisms used to practice methods disclosed herein may be eukaryotic and/or photosynthetic. In some instances, the organism is a non-vascular photosynthetic organism, for example a macroalgae, microalgae, *Ch. vulgaris, C. reinhardtii, D. salina, S. quadricanda* or *H. pluvalis.* Organisms used to practice methods disclosed herein may also be non-photosynthetic, for example yeast. In some instances, a non-photosynthetic organism may contain exogenous chloroplast DNA. In some embodiments, an additional step of modifying a portion of a chloroplast genome is utilized. A modification may be achieved through homologous recombination. Such recombination may occur in an organism, for example a eukaryotic and/or photosynthetic organism. In some instances, the organism is a non-vascular photosynthetic organism, for example a macroalgae, microalgae, *Ch. vulgaris, C. reinhardtii, D. salina, S. quadricanda* or *H. pluvalis.* In other instances, the organism may be non-photosynthetic, for example a yeast. In embodiments with a modification step, the step may comprise addition of a polynucleotide, deletion of one or more nucleic acid bases, mutation of one or more nucleic acid bases, rearrangement or a polynucleotide, or combination thereof.

Further disclosed herein is an isolated vector comprising essential chloroplast genes, a selectable marker and a manipulation in one or more nucleic acids in the vector. In some instances, essential chloroplast genes are cloned from a non-vascular photoshynthetic organism such as macroalgae, microalgae, *Ch. vulgaris, C. reinhardtii, D. salina*, *S. quadricanda* or *H. pluvalis.* Essential chloroplast genes for use in the vectors described herein may be synthetic. The vectors described herein may further comprise an expression cassette, which may further comprise a region for integration into target DNA, for example organelle DNA. The vectors described herein may also contain one or more selection markers, for example, an auxotrophic marker, an antibiotic resistance marker, a chloroplast marker, or combinations thereof. In some instances, the essential chloroplast genes are those required for chloroplast function, photosynthesis, carbon fixation, production of one or more hydrocarbons, or a combination thereof. Essential chloroplast genes may comprise up to 200 genes and/or consist of up to 400 kb. In some of the vectors described herein a manipulation in one or more nucleic acids is an addition, deletion, mutation, or rearrangement. In some instances, expression of the vector in a host cell produces a product not naturally produced by said host cell. In other instances, expression of a vector of the present invention results in an increase production of a product naturally produced by said host cell. Examples of such products are biomass degrading enzymes, fatty acids, terpenes or terpenoids.

As described herein, one aspect of the present invention is an isolated chloroplast comprising a vector of the present invention. In another aspect, a host cell comprising a vector of the present invention is provided. Host cells useful in the present invention may be naturally non-photosynthetic or naturally photosynthetic. Examples of organisms useful for the present invention include *Saccharomyces cerevisiae, Escherichia coli,* macroalgae, microalgae, *Ch. vulgaris, C. reinhardtii, D. salina, S. quadricanda* or *H. pluvalis.*

In another aspect of the present invention, a method for transforming a cell or organism is provided where the method comprises inserting into said cell or organism a vector comprising all essential chloroplast genes and optionally one or more genes not naturally occurring in said cell or organism. In some embodiments, the method further comprises the step of eliminating substantially all chloroplast genomes in said cell or organism. A cell or organism useful for this method may be photosynthetic, non-photosynthetic and/or eukaryotic. A cell or organism useful for this method may be non-vascular. In some instances, the vector for use in this method may also comprise an expression cassette and the expression cassette may be capable of integrating into non-nuclear DNA. In one embodiment the one or more genes not naturally occurring in the cell or organism is a gene in the isoprenoid pathway, MVA pathway, or MEP pathway. In another embodiment, the essential chloroplast genes are those that are required for chloroplast function, photosynthesis, carbon fixation, production of one or more hydrocarbons, or a combination thereof.

Further provided herein is a method for modifying an organism comprising the steps of transforming the organism with a vector comprising one or more polynucleotides sufficient to perform chloroplast function. In some instances, a vector useful for this method further comprises a sequence for production or secretion of a compound from said organism. In some instances, the compound is an isoprenoid. In other instances, the vector comprises all essential chloroplast genes. In still other instances, the essential chloroplast genes are rearranged or mutated. An organism useful for some embodiments comprises essentially no chloroplast genome prior to transformation.

Yet another method provided herein is a method for making a product from an organism comprising the step of transforming said organism with a vector comprising at least 20 kb of genomic DNA and one or more of the following: (i) a gene not naturally occurring in said organism; (ii) a deletion in a gene naturally occurring in said organism; (iii) a rearrangement of genes naturally occurring in said organism; and (iv) a mutation in a gene naturally occurring in said organism. In some instances, the organism is naturally photosynthetic. In other instances, the additional genes encode enzymes in the isoprenoid pathway, MVA pathway, or MEP pathway. In still another embodiment, the present disclosure provides a method for transforming a cell or organism comprising inserting into said cell or organism a chloroplast and a vector comprising all essential chloroplast genes.

The present disclosure also provides a method of producing an artificial chloroplast genome comprising the steps of: (a) providing a vector comprising one or more essential chloroplast genes; (b) adding to said vector a DNA fragment; (c) transforming a cell or organism with the vector produced by step (b); and (d) determining whether chloroplast function exists with said added DNA fragment. In some instances, the added DNA fragments comprises one or more coding regions for an enzyme in the isoprenoid, MVA or MEP pathway.

The present disclosure also provides a shuttle vector comprising a chloroplast genome. A genome may be modified. Also provided herein is a vector comprising an isolated, functional chloroplast genome. A chloroplast genome useful in such a vector may be modified.

Further provided herein is a method of producing an artificial chloroplast genome comprising the steps of: (a) providing a vector comprising all essential chloroplast genes; and (b) removing, adding, mutating, or rearranging DNA from the chloroplast genome. Such a method may further comprise the steps of transforming a redacted genome into a host organism; and (d) determining chloroplast function in the host organism. In some instances, steps (b), (c), and (d) are repeated. In still other instances, the chloroplast genome is from an organism selected from the group consisting of: macroalgae, microalgae, *Ch. vulgaris, C. reinhardtii, D. salina, S. quadricanda* or *H. pluvalis.* In other instances, the host organism is selected from the group consisting of: macroalgae, microalgae, *Ch. vulgaris, C. reinhardtii, D. salina, S. quadricanda* or *H. pluvalis.* For some embodiments, the method may further comprise the step of removing redundant DNA from a chloroplast genome. In other embodiments, the vector comprises all or substantially all of a chloroplast genome. A chloroplast genome useful in the present invention may be cloned from a photosynthetic organism or may be a synthetic chloroplast genome. In some instances, the vector further comprises a gene not naturally occurring in the host organism, for example a gene from the isoprenoid pathway, MVA pathway, or MEP pathway.

Yet another method provided herein is a method of producing an artificial chloroplast genome comprising the steps of: (a) providing a vector comprising an entire chloroplast genome; (b) deleting a portion of said entire chloroplast genome; and (c) determining whether chloroplast function exists without said deleted portion. In another aspect of the present invention, a composition comprising an isolated and functional chloroplast genome is provided. In some instances, a composition comprises a modification to said chloroplast genome.

Further provided herein is an ex vivo vector comprising a nucleic acid comprising at least about 10% of a chloroplast genome and a manipulation in one or more nucleic acids in the vector. In some instances, the nucleic acid is cloned from a non-vascular photosynthetic organism, for example a macroalgae, microalgae, *Ch. vulgaris, C. reinhardtii, D. salina, S. quadricanda* or *H. pluvalis.* In some instances, the nucleic acid is synthetic. A vector of the present invention may further comprise an expression cassette and an expression cassette may further comprise a region for integration into target DNA. In some instances, the target DNA is organelle DNA. A vector useful in the present invention may further comprise one or more selection markers, for example an auxotrophic marker, an antibiotic resistance marker, a chloroplast marker, or combinations thereof. In some embodiments, a manipulation in one or more nucleic acids in a vector may be an addition, deletion, mutation, or rearrangement. Expression of the vector may result in production of a product not naturally produced by a host cell and/or an increase production of a product naturally produced by a host cell. Examples of some products of the present invention include a terpene, terpenoid, fatty acid, or biomass degrading enzyme.

Also provided herein is an ex vivo vector comprising a nucleic acid comprising at least about 20 kilobases of a chloroplast genome and a manipulation in one or more nucleic acids in said vector. In some instances, the nucleic acid is cloned from a non-vascular photosynthetic organism, for example a macroalgae, microalgae, *Ch. vulgaris, C. reinhardtii, D. salina, S. quadricanda* or *H. pluvalis.* In some instances, the nucleic acid is synthetic. A vector of the present invention may further comprise an expression cassette and an expression cassette may further comprise a region for integration into target DNA. In some instances, the target DNA is organelle DNA. A vector useful in the present invention may further comprise one or more selection markers, for example an auxotrophic marker, an antibiotic resistance marker, a chloroplast marker, or combinations thereof. In some embodiments, a manipulation in one or more nucleic acids in a vector may be an addition, deletion, mutation, or rearrangement. Expression of the vector may result in production of a product not naturally produced by a host cell and/or an increase production of a product naturally produced by a host cell. Examples of some products of the present invention include a terpene, terpenoid, fatty acid, or biomass degrading enzyme.

Further provided herein is a method of producing a vector containing a reconstructed genome, comprising: introducing two or more vectors into a host cell, wherein the vectors comprise fragments of a genome, recombining the vectors into a single vector comprising at least about 90% of a genome, thereby producing a vector containing a reconstructed genome. In some instances, the host cell is eukaryotic, for example, S. cerevisiae. In other instances, the genome is an organelle genome. The organelle may be a chloroplast, for example a chloroplast from an alga, particularly a microalga such as *Ch. vulgaris, C. reinhardtii, D. salina, S. quadricanda* or *H. pluvalis.* In some instances, the two or more vectors comprise a selectable marker. In other instances, at least one of said fragments is synthetic. In still other instances, a further step comprising modifying a portion of the genome is useful in this method. Such a modification may comprise an addition, deletion, mutation, or rearrangement. In other embodiments, the modification is the addition of an exogenous nucleic acid which results in the production or increased production of a terpene, terpenoid, fatty acid or biomass degrading enzyme.

**Large DNA Cloning and Content**

An advantage of this invention is that it provides for the cloning, manipulation, and delivery of a vector containing chloroplast genome DNA consisting of up to all chloroplast genes (or sequences). The chloroplast genome DNA contained in the vector can be obtained by recombination of a hybrid cloning vector with one contiguous fragment of DNA or by recombination of two or more contiguous fragments of DNA.

The methods and compositions of the present invention may include captured and/or modified large pieces of DNA may comprise DNA from an organelle, such as mitochondrial DNA or plastid DNA (e.g., chloroplast DNA). The captured and/or modified large pieces of DNA may also comprise the entirety of an organelle's genome, e.g., a chloroplast genome. In other embodiments, the captured and/or modified large pieces of DNA comprise a portion of a chloroplast genome. A chloroplast genome may originate from any vascular or non-vascular plant, including algae, bryophytes (e.g., mosses, ferns), gymnosperms (e.g., conifers), and angiosperms (e.g., flowering plants - trees, grasses, herbs, shrubs). A chloroplast genome, or essential portions thereof, may comprise synthetic DNA, rearranged DNA, deletions, additions, and/or mutations. A chloroplast genome, or portions thereof, may comprise a one or more deletions, additions, mutations, and/or rearrangements. The deletions, additions, mutations, and/or rearrangements may be naturally found in an organism, for example a naturally occurring mutation, or may not be naturally found in nature. The chloroplast or plastid genomes of a number of organisms are widely available, for example, at the public database from the NCBI Organelle Genomes section available at http://www.ncbi.nlm.nih.gov/genomes/static/euk_o.html.

The target DNA sequence described herein may comprise at least 1, 2, 3, 4, or 5 deletions, additions, mutations, and/or rearrangements as compared to a control sequence (naturally occurring sequence). In some embodiments, the mutations may be functional or nonfunctional. For example, a functional mutation may have an effect on a cellular function when the mutation is present in a host cell as compared to a control cell without the mutation. A non-functional mutation may be silent in function, for example, there is no discernable difference in phenotype of a host cell without the mutation as compared to a cell with the mutation.

Captured and/or modified large pieces of DNA (e.g., target DNA), may comprise a minimal or minimized chloroplast genome (e.g., the minimum number of genes and/or DNA fragment, required for chloroplast functionality). The captured and/or modified DNA may comprise the essential chloroplast genes, it may comprise a portion or all, or substantially all of the essential chloroplast genes. An essential gene may be a gene that is essential for one or more metabolic processes or biochemical pathways. An essential gene may be a gene required for chloroplast function, such as photosynthesis, carbon fixation, or hydrocarbon production. An essential gene may also be a gene that is essential for gene expression, such as transcription, translation, or other process(es) that affect gene expression. The essential genes may comprise mutations or rearrangements. Essential genes may also comprise a minimally functional set of genes to perform a function. For example, a particular function (e.g., photosynthesis) may be performed ineffciently by a set of genes/gene products, however, this set would still comprise essential genes because the function is still performed.

Modified DNA may comprise at least 5, 10, 15, 20, 25, 30, 40, or 50 essential genes. In some embodiments, the DNA may comprise essential chloroplast genomic sequence of up to 150kb in length. The DNA may comprise essential chloroplast genes as well as non-essential chloroplast gene sequences. The DNA may be single stranded or double stranded, linear or circular, relaxed or supercoiled. The DNA may also be in the form of an expression cassette. For example, an expression cassette may comprise an essential gene to be expressed in a host cell. The expression cassette may comprise one or more essential genes as well as DNA sequences that promote the expression of the essential genes. The expression cassette may also comprise a region for integration into target DNA of a host. The expression cassette may also comprise one or more essential genes and one or more genes not naturally occurring in a host cell comprising the expression cassette. One of ordinary skill in the arts will easily ascertain various combinations of the aforementioned aspects of the expression cassettes.

In other instances, captured and/or modified pieces of DNA may comprise the entire genome of a plastid or organelle. For example, about 10%, 11%, 12%, 13%, 14%, 15%, 16%, 17%, 18%, 19%, 20%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% of a plastid genome, or more. In one embodiment the captured and/or modified large pieces of DNA may comprise 10-100%, 20-100%, 30-100%, 40-100%, 50-100%, 60-100%, 70-100%, 80-100%, or 90-100% of the entire genome of a plastid or cell.

In still other instances, the captured and/or modified large pieces of DNA may comprise about 10 kb, 11 kb, 12 kb, 13 kb, 14 kb, 15 kb, 16 kb, 17 kb, 18 kb, 19 kb, 20 kb, 21 kb, 22 kb, 23 kb, 24 kb, 25 kb, 26 kb, 27 kb, 28 kb, 29 kb, 30 kb, 31 kb, 32 kb, 33 kb, 34 kb, 35 kb, 36 kb, 37 kb, 38 kb, 39 kb, 40 kb, 41 kb, 42 kb, 43 kb, 44 kb, 45 kb, 46 kb, 47 kb, 48 kb, 49 kb, 50 kb, 51 kb, 52 kb, 53 kb, 54 kb, 55 kb, 56 kb, 57 kb, 58 kb, 59 kb, 60 kb, 61 kb, 62 kb, 63 kb, 64 kb, 65 kb, 66 kb, 67 kb, 68 kb, 69 kb, 70 kb, 71 kb, 72 kb, 73 kb, 74 kb, 75 kb, 76 kb, 77 kb, 78 kb, 79 kb, 80 kb, 81 kb, 82 kb, 83 kb, 84 kb, 85 kb, 86 kb, 87 kb, 88 kb, 89 kb, 90 kb, 91 kb, 92 kb, 93 kb, 94 kb, 95 kb, 96 kb, 97 kb, 98 kb, 99 kb, 100 kb, 101 kb, 102 kb, 103 kb, 104 kb, 105 kb, 106 kb, 107 kb, 108 kb, 109 kb, 110 kb, 111 kb, 112 kb, 113 kb, 114 kb, 115 kb, 116 kb, 117 kb, 118 kb, 119 kb, 120 kb, 121 kb,122 kb, 123 kb, 124 kb, 125 kb, 126 kb, 127 kb, 128 kb, 129 kb, 130 kb, 131 kb, 132 kb, 133 kb, 134 kb, 135 kb, 136 kb, 137 kb, 138 kb, 139 kb, 140 kb, 141 kb, 142 kb, 143 kb, 144 kb, 145 kb, 146 kb, 147 kb, 148 kb, 149 kb, 150 kb, 151 kb, 152 kb, 153 kb, 154 kb, 155 kb, 156 kb, 157 kb, 158 kb, 159 kb, 160 kb, 161 kb, 162 kb, 163 kb, 164 kb, 165 kb, 166 kb, 167 kb, 168 kb, 169 kb, 170 kb, 171 kb, 172 kb, 173 kb, 174 kb, 175 kb, 176 kb, 177 kb, 178 kb, 179 kb, 180 kb, 181 kb, 182 kb, 183 kb, 184 kb, 185 kb, 186 kb, 187 kb, 188 kb, 189 kb, 190 kb, 191 kb, 192 kb, 193 kb, 194 kb, 195 kb, 196 kb, 197 kb, 198 kb, 199 kb, 200 kb, 201 kb, 202 kb, 203 kb, 204 kb, 205 kb, 206 kb, 207 kb, 208 kb, 209 kb, 210 kb, 211 kb, 212 kb, 213 kb, 214 kb, 215 kb, 216 kb, 217 kb, 218 kb, 219 kb, 220 kb, 221 kb, 222 kb, 223 kb, 224 kb, 225 kb, 226 kb, 227 kb, 228 kb, 229 kb, 230 kb, 231 kb, 232 kb, 233 kb, 234 kb, 235 kb, 236 kb, 237 kb, 238 kb, 239 kb, 240 kb, 241 kb, 242 kb, 243 kb, 244 kb, 245 kb, 246 kb, 247 kb, 248 kb, 249 kb, 50 kb, 51 kb, 252 kb, 253 kb, 254 kb, 255 kb, 256 kb, 257 kb, 258 kb, 259 kb, 260 kb, 261 kb, 262 kb, 263 kb, 264 kb, 265 kb, 266 kb, 267 kb, 268 kb, 269 kb, 270 kb, 271 kb, 272 kb, 273 kb, 274 kb, 275 kb, 276 kb, 277 kb, 278 kb, 279 kb, 280 kb, 281 kb, 282 kb, 283 kb, 284 kb, 285 kb, 286 kb, 287 kb, 288 kb, 289 kb, 290 kb, 291 kb, 292 kb, 293 kb, 294 kb, 295 kb, 296 kb, 297 kb, 298 kb, 299 kb, 300 kb, 301 kb, 302 kb, 303 kb, 304 kb, 305 kb, 306 kb, 307 kb, 308 kb, 309 kb, 310 kb, 311 kb, 312 kb, 313 kb, 314 kb, 315 kb, 316 kb, 317 kb, 318 kb, 319 kb, 320 kb, 321 kb, 322 kb, 323 kb, 324 kb, 325 kb, 326 kb, 327 kb, 328 kb, 329 kb, 330 kb, 331 kb, 332 kb, 333 kb, 334 kb, 335 kb, 336 kb, 337 kb, 338 kb, 339 kb, 340 kb, 341 kb, 342 kb, 343 kb, 344 kb, 345 kb, 346 kb, 347 kb, 348 kb, 349 kb, 350 kb, 351 kb, 352 kb, 353 kb, 354 kb, 355 kb, 356 kb, 357 kb, 358 kb, 359 kb, 360 kb, 361 kb, 362 kb, 363 kb, 364 kb, 365 kb, 366 kb, 367 kb, 368 kb, 369 kb, 370 kb, 371 kb, 372 kb, 373 kb, 374 kb, 375 kb, 376 kb, 377 kb, 378 kb, 379 kb, 380 kb, 381 kb, 382 kb, 383 kb, 384 kb, 385 kb, 386 kb, 387 kb, 388 kb, 389 kb, 390 kb, 391 kb, 392 kb, 393 kb, 394 kb, 395 kb, 396 kb, 397 kb, 398 kb, 399 kb, 400 kb or more genomic (e.g., nuclear or organelle) DNA. In some embodiments the captured and or modified large pieces of DNA may comprise about 10-400 kb, 50-350 kb, 100-300 kb, 100-200 kb, 200-300 kb, 150-200 kb, 200-250 kb genomic DNA

In still other instances, the captured and or modified large pieces of DNA may comprise about 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121,122, 123, 124, 125, 126, 127, 128, 129, 130, 131, 132, 133, 134, 135, 136, 137, 138, 139, 140, 141, 142, 143, 144, 145, 146, 147, 148, 149, 150,151, 152, 153, 154, 155, 156, 157, 158, 159, 160, 161, 162, 163, 164, 165, 166, 167, 168, 169, 170, 171, 172, 173, 174, 175, 176, 177,178, 179, 180, 181, 182,1 83, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 50, 51, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300 or more open reading frames, partial open reading frames, pseudogenes and/or repeating sequences.

The present invention also provides vectors comprising a cassette-able chloroplast genome or portion thereof (e.g., a removable DNA fragment comprising a chloroplast genome or functional portion thereof). A vector of the present invention may comprise functional chloroplast units (e.g., a unit essential for metabolic processes, photosynthesis, gene expression, photosystem I, photosystem II). Vectors of the present invention may comprise a transplantable chloroplast genome or portion thereof. Additionally, the vectors of the present invention may comprise a transferable chloroplast genome or portion thereof. In other embodiments, the vectors comprise: 1) one or more large pieces of modified DNA; 2) all genes necessary to carry out photosynthesis; 3) all genes required for chloroplast survival and/or function; 4) essential chloroplast genes; and/or 5) sufficient naturally occurring or modified chloroplast genes to perform one or more chloroplast functions, such as photosynthesis. A vector may comprise a portion, substantially all, or all of the essential chloroplast genes. A vector may comprise at least 5, 10, 15, 20, 25, 30, 40, 50, 60, 70, 80, 90, 100 or more essential genes.

A vector may comprise chloroplast DNA of 10, 20, 30, 40, 50, 60, 70, 80, 90, 100, 110, 120, 130, 140, 150, 160, 170, 180, 190, 200, 210, 220, 230, 240, 250 kb or more in length. A vector may comprise essential chloroplast genes as well as non-essential chloroplast gene sequences. A vector may comprise one or more, or all, essential chloroplast genes and/or one or more genes not naturally occurring in a host cell comprising a vector. In some embodiments, a vector may comprise chloroplast genes and genes not naturally occurring in the chloroplast. A vector may comprise one or more essential chloroplast genes and/or one or more DNA sequences or genes involved in chloroplast function, photosynthesis, carbon fixation, and/or hydrocarbon production. For example, a vector may comprise a sequence required for photosynthesis and a sequence involved in the isoprenoid production, MVA, and/or MEP pathways, such as a DNA sequence encoding a terpene synthase, or other polypeptide that produces a hydrocarbon, such as a terpene or isoprenoid. The invention further provides methods for cloning, manipulating and delivering a large target nucleic acid to a cell or particle, such as, for example, yeasts or bacteria. Certain embodiments of this method make use of a hybrid yeast-bacteria cloning system (See, e.g., U.S. Patent Nos. 5,866,404 and 7,083,971 and Hokanson et al., (2003) Human Gene Ther.: 14: 329-339). The vectors herein (e.g., cloning system) is comprised of a shuttle vector that contains elements for function and replication in both yeast and bacteria, allowing it to stably function and replicate in either organism. This composition of functional and replicative elements yields a hybrid system which enjoys the benefits of both genetic engineering systems. The genetics of yeast *(e.g., S. cerevisiae)* are well understood and a powerful assortment of molecular biology tools exists for genetic engineering in yeast.

In another aspect the invention produces a gap-filled vector by homologous recombination among the two arms and the target nucleic acid. In still another embodiment, at least one arm further comprises an origin of replication. In another embodiment of the invention, each arm further comprises a rare restriction endonuclease recognition site. Homologous recombination may be performed *in vitro* or *in vivo,* for example, in a fungal cell (e.g., *S. cerevisiae, Sz. pombe* or *U. maydis*). The invention also provides a eukaryotic host cell harboring the recombinational cloning system or vector according to the invention, for example, in a fungal cell (e.g., *S*. *cerevisiae, Sz. pombe* or *U. maydis*).

A gap-filled linear vector may be converted to a circular vector *in vitro* (e.g. using T4 ligase) or *in vivo,* for example, in a bacterium. The circular vectors of interest can be amplified, purified, cut and used to recover sufficient amounts of DNA to be introduced either directly into a cell or into a suitable delivery system for subsequent delivery to a target cell. The methodology offers great versatility to clone and to modify any large bacterial or non-bacterial genome, and easily facilitate the use thereof as recombinational vectors. Direct delivery of a gap-filled vector into a cell may be performed by methods well known in the field such as, for example, calcium phosphate transformation methodologies or electroporation (see Sambrook et al., supra).

Accordingly, the invention provides a method for producing a recombinant delivery unit including the steps of: (a) producing a gap-filled vector containing a target sequence; (b) optionally circularizing the gap-filled vector segments of the invention; (c) propagating the vector, and (d) introducing the gap-filled vector in a delivery unit.

Bacterial systems are useful for amplifying and purifying DNA, and for functionally testing the genetic modifications and their effect on pathways. One embodiment of the present invention provides cloning system will aid in the cloning and modifying of any large genome and easily facilitate the cloning and introduction of pathways. With the ability to deliver whole pathways, certain embodiments of the present invention allow for a system biological approach to problem solving.

In general, target DNA (e.g., genomic DNA) may be captured by creating sites allowing for homologous recombination in the vector. For example, such sites may be created by, but not limited to, gap-repair cloning, wherein a gap is created in the vector, usually by restrictive enzyme digestion prior to transformation into the yeast. When the target DNA is mixed with the vector, the target DNA is recombined into the vector. This operation is called "gap filling." This recombination can occur in bacteria, yeast, the original host organism, another organism, or in vitro. In some embodiments, recombination is performed in yeast because of the high rate of homologous recombination. Once captured, the target DNA can be modified in many ways including adding, altering, or removing DNA sequences. In some embodiments, the target DNA is genomic DNA. In other embodiments, the target DNA is organelle (e.g., mitochondria or chloroplast) DNA.

In some embodiments, target DNA is modified by adding, altering or removing genes, coding sequences, partial coding sequences, regulatory elements, positive and/or negative selection markers, recombination sites, restriction sites, and/or codon bias sites. For example, the target DNA sequence may be codon biased for expression in the organism being transformed. The skilled artisan is well aware of the "codon-bias" exhibited by a specific host cell in usage of nucleotide codons to specify a given amino acid. Without being bound by theory, by using a host cell's preferred codons, the rate of translation may be greater. Therefore, when synthesizing a gene for improved expression in a host cell, it may be desirable to design the gene such that its frequency of codon usage approaches the frequency of preferred codon usage of the host cell. The codons of the present invention may be A/T rich, for example, A/T rich in the third nucleotide position of the codons. Typically, the A/T rich codon bias is used for algae. In some embodiments, at least 50% of the third nucleotide position of the codons are A or T. In other embodiments, at least 60%, 70%, 80%, 90%, or 99% of the third nucleotide position of the codons are A or T. (see also U.S. Publication No. 2004/0014174).

Such manipulations are well known in the art and can be performed in numerous ways. In some embodiments, the modifications may be performed using cloned sequences. In other embodiments, the modifications may be performed using synthetic DNA.

Genetic manipulations include cloning large pieces of target DNA (e.g., chromosomes, genomes) and/or dividing and reorganizing target DNA based on functional relations between genes, such as metabolic pathways or operons. Genetic manipulations also include introducing and removing metabolic pathways, recombining DNA into functional units (e.g., metabolic pathways, synthetic operons), and/or determining sites of instability in large pieces of DNA (e.g., sites where a native or non-native host tends to delete or recombine a sequence of DNA).

Target DNA may be DNA from a prokaryote. Target DNA may also be genomic DNA, mitochondrial DNA, or chloroplast DNA from a eukaryote. Examples of such organisms from which genomic and/or organelle DNA may serve as target DNA include, but are not limited to *Z*. *mobilis*, algae (e.g., macroalgae or microalgae, such as *Chlamydomonas reinhardtii* ), a rhodophyte, a chlorophyte, a heterokontophyte, a tribophyte, a glaucophyte, a chlorarachniophyte, a euglenoid, a haptophyte, a cryptomonad, a dinoflagellum, or a phytoplankton. One of skill in the art will recognize that these organisms are listed only as examples and that the methods disclosed herein are applicable to the large DNA from any organism, including bacteria, plants, fungi, protists, and animals. Genetic manipulations of the present invention may include stabilizing large pieces of DNA by removing or inserting sequences that force transformed cells to preserve certain sequences of DNA and to stably maintain the sequences in its progeny. Genetic manipulations may also include altering codons of the target DNA, vector DNA, and/or synthetic DNA to reflect any codon bias of the host organism. Additionally, genetic manipulations of the present invention may include determining the minimal set of genes required for an organism to be viable. In another embodiment, the genetic manipulations of the present invention include determining the minimal set of genes required for a certain metabolic pathway to be created or maintained.

The genetic manipulations of the present invention may include determining redundant genes both within a genome, and between two genomes (e.g., redundancy between the nuclear and chloroplast genome). Additionally, the genetic manipulations of the present invention may include determining a functional sequence of DNA that could be artificially synthesized (e.g. the genes in a certain metabolic pathway, the genes of a functional genome). In another embodiment, the genetic manipulations of the present invention include creating DNA and genomes packaged into cassettes (e.g., sites within a vector where genes can be easily inserted or removed). The genetic manipulations of the present invention may also include creating a nuclear or organelle genome that is viable in multiple species (e.g. a transplantable chloroplast genome). Furthermore, the genetic manipulations of the present invention may include a method for testing the viability of any of these manipulations or creations (e.g., transferring a shuttle vector back into a host system and assaying for survival).

**Vectors, Markers and Transformation**

A vector or other recombinant nucleic acid molecule may include a nucleotide sequence encoding a selectable marker. The term or "selectable marker" or "selection marker" refers to a polynucleotide (or encoded polypeptide) that confers a detectable phenotype. A selectable marker generally encodes a detectable polypeptide, for example, a green fluorescent protein or an enzyme such as luciferase, which, when contacted with an appropriate agent (a particular wavelength of light or luciferin, respectively) generates a signal that can be detected by eye or using appropriate instrumentation (Giacomin, Plant Sci. 116:59-72, 1996; Scikantha, J. Bacteriol. 178:121, 1996; Gerdes, FEBS Lett. 389:44-47, 1996; see, also, Jefferson, EMBO J. 6:3901-3907, 1997, fl-glucuronidase). A selectable marker generally is a molecule that, when present or expressed in a cell, provides a selective advantage (or disadvantage) to the cell containing the marker, for example, the ability to grow in the presence of an agent that otherwise would kill the cell.

A selectable marker can provide a means to obtain prokaryotic cells or plant cells or both that express the marker and, therefore, can be useful as a component of a vector of the invention (see, for example, Bock, supra, 2001). Examples of selectable markers include, but are not limited to, those that confer antimetabolite resistance, for example, dihydrofolate reductase, which confers resistance to methotrexate (Reiss, Plant Physiol. (Life Sci. Adv.) 13:143-149, 1994); neomycin phosphotransferase, which confers resistance to the aminoglycosides neomycin, kanamycin and paromycin (Herrera-Estrella, EMBO J. 2:987-995, 1983), hygro, which confers resistance to hygromycin (Marsh, Gene 32:481-485, 1984), trpB, which allows cells to utilize indole in place of tryptophan; hisD, which allows cells to utilize histinol in place of histidine (Hartman, Proc. Natl. Acad. Sci., USA 85:8047, 1988); mannose-6-phosphate isomerase which allows cells to utilize mannose (WO 94/20627); ornithine decarboxylase, which confers resistance to the ornithine decarboxylase inhibitor, 2-(difluoromethyl)-DL-ornidtine (DFMO; McConlogue, 1987, In: Current Communications in Molecular Biology, Cold Spring Harbor Laboratory ed.); and deaminase from *Aspergillus terreus,* which confers resistance to Blasticidin S (Tamura, Biosci. Biotechnol. Biochem. 59:2336-2338, 1995). Additional selectable markers include those that confer herbicide resistance, for example, phosphinothricin acetyltransferase gene, which confers resistance to phosphinothricin (White et al., Nucl. Acids Res. 18:1062, 1990; Spencer et al., Theor. Appl. Genet. 79:625-631, 1990), a mutant EPSPV-synthase, which confers glyphosate resistance (Hinchee et al., BioTechnology 91:915-922, 1998), a mutant acetolactate synthase, which confers imidazolione or sulfonylurea resistance (Lee et al., EMBO J. 7:1241-1248, 1988), a mutant psbA, which confers resistance to atrazine (Smeda et al., Plant Physiol. 103:911-917, 1993), or a mutant protoporphyrinogen oxidase (see U.S. Pat. No. 5,767,373), or other markers conferring resistance to an herbicide such as glufosinate. Selectable markers include polynucleotides that confer dihydrofolate reductase (DHFR) or neomycin resistance for eukaryotic cells and tetracycline; ampicillin resistance for prokaryotes such as *E. coli;* and bleomycin, gentamycin, glyphosate, hygromycin, kanamycin, methotrexate, phleomycin, phosphinotricin, spectinomycin, streptomycin, sulfonamide and sulfonylurea resistance in plants (see, for example, Maliga et al., Methods in Plant Molecular Biology, Cold Spring Harbor Laboratory Press, 1995, page 39).

Methods for nuclear and plastid transformation are routine and well known for introducing a polynucleotide into a plant cell chloroplast (see U.S. Pat. Nos. 5,451,513, 5,545,817, and 5,545,818; WO 95/16783; McBride et al., Proc. Natl. Acad. Sci., USA 91:7301-7305, 1994). In some embodiments, chloroplast transformation involves introducing regions of chloroplast DNA flanking a desired nucleotide sequence, allowing for homologous recombination of the exogenous DNA into the target chloroplast genome. In some instances one to 1.5 kb flanking nucleotide sequences of chloroplast genomic DNA may be used. Using this method, point mutations in the chloroplast 16S rRNA and rps12 genes, which confer resistance to spectinomycin and streptomycin, can be utilized as selectable markers for transformation (Svab et al., Proc. Natl. Acad. Sci., USA 87:8526-8530, 1990), and can result in stable homoplasmic transformants, at a frequency of approximately one per 100 bombardments of target leaves.

Microprojectile mediated transformation also can be used to introduce a polynucleotide into a plant cell chloroplast (Klein et al., Nature 327:70-73, 1987). This method utilizes microprojectiles such as gold or tungsten, which are coated with the desired polynucleotide by precipitation with calcium chloride, spermidine or polyethylene glycol. The microprojectile particles are accelerated at high speed into a plant tissue using a device such as the BIOLISTIC PD-1000 particle gun (BioRad; Hercules Calif.). Methods for the transformation using biolistic methods are well known in the art (see, e.g.; Christou, Trends in Plant Science 1:423-431, 1996). Microprojectile mediated transformation has been used, for example, to generate a variety of transgenic plant species, including cotton, tobacco, corn, hybrid poplar and papaya. Important cereal crops such as wheat, oat, barley, sorghum and rice also have been transformed using microprojectile mediated delivery (Duan et al., Nature Biotech. 14:494-498, 1996; Shimamoto, Curr. Opin. Biotech. 5:158-162, 1994). The transformation of most dicotyledonous plants is possible with the methods described above. Transformation of monocotyledonous plants also can be transformed using, for example, biolistic methods as described above, protoplast transformation, electroporation of partially permeabilized cells, introduction of DNA using glass fibers, the glass bead agitation method, and the like.

Transformation frequency may be increased by replacement of recessive rRNA or r-protein antibiotic resistance genes with a dominant selectable marker, including, but not limited to the bacterial aadA gene (Svab and Maliga, Proc. Natl. Acad. Sci., USA 90:913-917, 1993). Approximately 15 to 20 cell division cycles following transformation are generally required to reach a homoplastidic state. It is apparent to one of skill in the art that a chloroplast may contain multiple copies of its genome, and therefore, the term "homoplasmic" or "homoplasmy" refers to the state where all copies of a particular locus of interest are substantially identical. Plastid expression, in which genes are inserted by homologous recombination into all of the several thousand copies of the circular plastid genome present in each plant cell, takes advantage of the enormous copy number advantage over nuclear-expressed genes to permit expression levels that can readily exceed 10% of the total soluble plant protein.

Any of the nucleotide sequences of target DNA, vector DNA, or synthetic DNA on the vectors of the invention can further include codons biased for expression of the nucleotide sequences in the organism transformed. In some instances, codons in the nucleotide sequences are A/T rich in a third nucleotide position of the codons. For example, at least 50% of the third nucleotide position of the codons may be A or T. In other instances, the codons are G/C rich, for example at least 50% of the third nucleotide positions of the codons may be G or C.

The nucleotide sequences of the shuttle vectors of the present invention can be adapted for chloroplast expression. For example, a nucleotide sequence herein can comprise a chloroplast specific promoter or chloroplast specific regulatory control region. The nucleotide sequences can also be adapted for nuclear expression. For example, a nucleotide sequence can comprise a nuclear specific promoter or nuclear specific regulatory control regions. The nuclear sequences can encode a protein with a targeting sequence that encodes a chloroplast targeting protein (e.g., a chloroplast transit peptide), or a signal peptide that directs a protein to the endomembrane system for deposition in the endoplasmic reticulum or plasma membrane.

In embodiments where a vector encodes genes capable of fuel production, fuel products are produced by altering the enzymatic content of the cell to increase the biosynthesis of specific fuel molecules. For example, nucleotides sequences (e.g., an ORF isolated from an exogenous source) encoding biosynthetic enzymes can be introduced into the chloroplast of a photosynthetic organism. Nucleotide sequences encoding fuel biosynthetic enzymes can also be introduced into the nuclear genome of the photosynthetic organisms. Nucleotide sequences introduced into the nuclear genome can direct accumulation of the biosynthetic enzyme in the cytoplasm of the cell, or may direct accumulation of the biosynthetic enzyme in the chloroplast of the photosynthetic organism.

Any of the nucleotide sequences herein may further comprise a regulatory control sequence. Regulatory control sequences can include one or more of the following: a promoter, an intron, an exon, processing elements, 3' untranslated region, 5' untranslated region, RNA stability elements, or translational enhancers A promoter may be one or more of the following: a promoter adapted for expression in the organism, an algal promoter, a chloroplast promoter, and a nuclear promoter, any of which may be a native or synthetic promoters. A regulatory control sequence can be inducible or autoregulatable. A regulatory control sequence can include autologous and/or heterologous sequences. In some cases, control sequences can be flanked by a first homologous sequence and a second homologous sequence. The first and second homologous sequences can each be at least 500 nucleotides in length. The homologous sequences can allow for either homologous recombination or can act to insulate the heterologous sequence to facilitate gene expression.

Vectors may also comprise sequences involved in producing products useful as biopharmaceuticals, such as, but not limited to, antibodies (including functional portions thereof), interleukins and other immune modulators, and antibiotics. See, e.g., Mayfield et al., (2003) Proc. Nat'l Acad. Sci.: 100 (438-42) and U.S. Pub. No. 2004/0014174.

Vectors of the present invention may comprise a cassette-able bacterial genome or portion thereof (e.g., a removable DNA fragment comprising a bacterial genome or functional portion thereof). Additionally, vectors of the present invention may comprise functional genomic units (e.g., a unit essential for metabolic processes, biochemical pathways, gene expression). Vectors of the present invention may comprise a transplantable bacterial genome or portion thereof. Vectors of the present invention may comprise a transferable bacterial genome or portion thereof.

In some embodiments, the large piece of target DNA is modified. The modified DNA may comprise all genes necessary to carry out ethanologenesis, all genes required for the Entner-Duodorff pathway, the glucose tolerance pathway, the ethanol tolerance pathway, the carboxylic acid byproduct resistance pathway, the acetic acid tolerance pathway, the sugar transport pathway, sugar fermentation pathways, and/or the cellulose and hemicellulose digestive pathways.

Hybrid cloning systems and methods of the invention combine the high versatility of yeast as a system for the capture and manipulation of a given nucleic acid and the high efficiency of bacterial systems for the amplification of such nucleic acid. Recombinational vectors relying on homologous recombination to mediate the isolation, manipulation and delivery of large nucleic acid fragments were constructed. The invention described herein also provides methods for using such recombinational cloning vectors to clone, to manipulate and to deliver large nucleic acids. Finally, the invention provides methods for using such recombinational cloning systems as potentiators of biochemical pathway analysis, organelle analysis, and synthetic chloroplast construction.

The vectors of the present invention may be introduced into yeast. The yeast may be a suitable strain of *Saccharomyces cerevisiae;* however, other yeast models may be utilized. Introduction of vectors into yeast may allow for genetic manipulation of the vectors. Yeast vectors have been described extensively in the literature and methods of manipulating the same also are well known as discussed hereinafter (see e.g., Ketner et al. (1994) Proc. Natl. Acad. Sci. (USA) 91:6186 6190).

Following genetic manipulation, the cloning system may allow for the transition to a bacterial environment, suitable for the preparation of larger quantities of nucleic acids. Representative examples of a bacterial type vectors include, but are not limited to, the P1 artificial chromosome, bacterial artificial chromosome (BAC) and single copy plasmid F factors (Shizuya et al. (1992) Proc. Natl. Acad. Sci. 89:8794 8797). Similarly, bacterial vectors are well known in the art (e.g., Ioannou et al. (1994) Nature 6:84 89). The invention also provides a shuttle vector comprising a yeast selectable marker, a bacterial selectable marker, a telomere, a centromere, a yeast origin of replication, and/or a bacterial origin of replication.

Shuttle vectors of the present invention may enable homologous recombination in yeast to capture and to integrate in a vector of interest a target nucleic acid of interest. Shuttle vectors may allow for the manipulation of target DNA in any of the hosts to which the vectors can be introduced. In some embodiments, after desired manipulations, shuttle vector components may be removed, leaving just the modified target DNA. Such extraction of vector sequences can be performed using standard methodologies and may occur in any host cell. The target nucleic acid of interest can be a large nucleic acid, and can include, for example, a vector, such as a viral vector, including the foreign gene of interest contained therein. The target nucleic acid can also be a bacterial (including archaebacteria and eubacteria), viral, fungal, protist, plant or animal genome, or a portion thereof. For example, the target nucleic acid of one embodiment of the present invention comprises an entire prokaryotic genome. As an additional example, a target nucleic acid of the present invention may comprises the chloroplast genome of a eukaryotic organism.

Shuttle vectors according to the invention may comprise an appropriately oriented DNA that functions as a telomere in yeast and a centromere. Any suitable telomere may be used. Suitable telomeres include without limitation telomeric repeats from many organisms, which can provide telomeric function in yeast. The terminal repeat sequence in humans (TTAGGG)_{N}, is identical to that in trypanosomes and similar to that in yeast ((TG)₁₋₃)_{N} and Tetrahymena (TTGGG)_{N} (Szostak & Blackburn (1982) Cell 29:245 255; Brown (1988) EMBO J. 7:2377 2385; and Moyzis et al. (1988) Proc. Natl. Acad. Sci. 85:6622 6626).

The term "centromere" is used herein to identify a nucleic acid, which mediates the stable replication and precise partitioning of the vectors of the invention at meiosis and at mitosis thereby ensuring proper segregation into daughter cells. Suitable centromeres include, without limitation, the yeast centromere, CEN4, which confers mitotic and meiotic stability on large linear plasmids (Murray & Szostak (1983) Nature 305:189 193; Carbon (1984) Cell 37:351 353; and Clark et al. (1990) Nature 287:504 509)).

In some embodiments, at least one of the two segments of the circular vector according to the invention includes at least one replication system that is functional in a host cell/particle of choice. As it will become apparent hereinafter, one of skill will realize that the manipulation, amplification and/or delivery of a target nucleic acid of choice may entail the use of more than one host cell/particle. Accordingly, more than one replication system functional in each host cell/particle of choice may be included.

When a host cell(s) is a prokaryote, particularly *E*. *coli,* replication system(s) include those which are functional in prokaryotes, such as, for example, P1 plasmid replicon, ori, P1 lytic replicon, ColE1, BAC, single copy plasmid F factors and the like. Either one or both segments, and/or the circular vector, may further include a yeast origin of replication capable of supporting the replication of large nucleic acids. Non-limiting examples of replication regions according to the invention include the autonomously replicating sequence or "ARS element." ARS elements were identified as yeast sequences that conferred high-frequency transformation. *Tetrahymena* DNA termini have been used as ARS elements in yeast along with ARS1 and ARS4 (Kiss et al. (1981) Mol. Cell Biol. 1:535 543; Stinchcomb et al. (1979) Nature 282:39; and Barton & Smith (1986) Mol. Cell Biol. 6:2354). For each segment (e.g., those corresponding to the yeast and bacterial elements of the gap-filling shuttle vector) there may be two or more origins of replication.

The first and/or the second segment according to an aspect of the invention may be joined in a circularized vector form (e.g., plasmid form). Circularization can occur *in vivo* or *in vitro* using the segment of interest. Alternatively, a circular vector of interest can be used. As used herein, the term "vector" designates a plasmid or phage DNA or other nucleic acid into which DNA or other nucleic acid may be cloned. The vector may replicate autonomously in a host cell and may be characterized further by one or a small number of restriction endonuclease recognition sites at which such nucleic acids may be cut in a determinable fashion and into which nucleic acid fragments may be inserted. The vector further may contain a selectable marker suitable for the identification of cells transformed with the vector.

Target nucleic acids of the invention may vary considerably in complexity. The target nucleic acid may include viral, prokaryotic or eukaryotic DNA, cDNA, exonic (coding), and/or intronic (noncoding) sequences. Hence, the target nucleic acid of the invention may include one or more genes. A target nucleic acid may be a chromosome, genome, or operon and/or a portion of a chromosome, genome or operon. A target nucleic acid may comprise coding sequences for all the genes in a pathway, the minimum complement of genes necessary for survival of an organelle, and/or the minimum complement of genes necessary for survival of an organism. A target nucleic acid may comprise *Zymomonas mobilis* DNA sequence, including, but not limited to genomic DNA and/or cDNA. A target nucleic acid may comprise eukaryotic chloroplast DNA sequence, including but not limited to, chloroplast genome DNA and/or cDNA. A target nucleic acid may comprise cyanobacteria DNA, including but not limited to genomic DNA and/or cDNA. The target nucleic acid also may be of any origin and/or nature.

It may be desirable for the gene to also comprise a promoter operably linked to the coding sequence in order to effectively promote transcription. Enhancers, repressors and other regulatory sequences may also be included in order to modulate activity of the gene, as is well known in the art. A gene as provided herein can refer to a gene that is found in the genome of the individual host cell (i.e., endogenous) or to a gene that is not found in the genome of the individual host cell (i.e., exogenous or a "foreign gene"). Foreign genes may be from the same species as the host or from different species. For transfection of a cell using DNA containing a gene with the intent that the gene will be expressed in the cell, the DNA may contain any control sequences necessary for expression of the gene in the required orientation for expression. The term "intron" as used herein, refers to a DNA sequence present in a given gene which is not translated into protein and is generally found between exons.

Genetic elements, or polynucleotides comprising a region that encodes a polypeptide or a region that regulates transcription or translation or other processes important to expression of the polypeptide in a host cell, or a polynucleotide comprising both a region that encodes a polypeptide and a region operably linked thereto that regulates expression. Genetic elements may be comprised within a vector that replicates as an episomal element; that is, as a molecule physically independent of the host cell genome. They may be comprised within mini-chromosomes, such as those that arise during amplification of transfected DNA by methotrexate selection in eukaryotic cells. Genetic elements also may be comprised within a host cell genome; not in their natural state but, rather, following manipulation such as isolation, cloning and introduction into a host cell in the form of purified DNA or in a vector, among others.

Vectors of the present invention may contain sufficient linear identity or similarity (homology) to have the ability to hybridize to a portion of a target nucleic acid made or which is single-stranded, such as a gene, a transcriptional control element or intragenic DNA. Without being bound to theory, such hybridization is ordinarily the result of base-specific hydrogen bonding between complementary strands, preferably to form Watson-Crick base pairs. As a practical matter, such homology can be inferred from the observation of a homologous recombination event. In some embodiments, such homology is from about 8 to about 1000 bases of the linear nucleic acid. In other embodiments, such homology is from about 12 to about 500 bases. One skilled in the art will appreciate that homology may extend over longer stretches of nucleic acids.

Homologous recombination is a type of genetic recombination, a process of physical rearrangement occurring between two strands of DNA. Homologous recombination involves the alignment of similar sequences, a crossover between the aligned DNA strands, and breaking and repair of the DNA to produce an exchange of material between the strands. The process homologous recombination naturally occurs in organisms and is also utilized as a molecular biology technique for introducing genetic changes into organism.

The vectors of the invention may be modified further to include functional entities other than the target sequence which may find use in the preparation of the construct(s), amplification, transformation or transfection of a host cell, and--if applicable--for integration in a host cell. For example, the vector may comprise regions for integration into host DNA. Integration may be into nuclear DNA of a host cell. In some embodiments, integration may be into non-nuclear DNA, such as chloroplast DNA. Other functional entities of the vectors may include, but are not limited to, markers, linkers and restriction sites.

A target nucleic acid may include a regulatory nucleic acid. This refers to any sequence or nucleic acid which modulates (either directly or indirectly, and either up or down) the replication, transcription and/or expression of a nucleic acid controlled thereby. Control by such regulatory nucleic acid may make a nucleic acid constitutively or inducibly transcribed and/or translated. Any of the nucleotide sequences herein may further comprise a regulatory control sequence. Examples of regulatory control sequences can include, without limitation, one or more of the following: a promoter, an intron, an exon, processing elements, 3' untranslated region, 5' untranslated region, RNA stability elements, or translational enhancers. A promoter may be one or more of the following: a promoter adapted for expression in the organism (e.g., bacterial, fungal, viral, plant, mammalian, or protist), an algal promoter, a chloroplast (or other plastid) promoter, a mitochondrial promoter, and a nuclear promoter, any of which may be a native or synthetic promoters. A regulatory control sequence can be inducible or autoregulatable. A regulatory control sequence can include autologous and/or heterologous sequences. In some cases, control sequences can be flanked by a first homologous sequence and a second homologous sequence. The first and second homologous sequences can each be at least 500 nucleotides in length. The homologous sequences can allow for either homologous recombination or can act to insulate the heterologous sequence to facilitate gene expression.

In some instances, target DNA, vector DNA or other DNA present in a shuttle vector of the present invention does not result in production of a polypeptide product but rather allows for secretion of the product from the cell. In these cases, the nucleotide sequence may encode a protein that enhances or initiates or increases the rate of secretion of a product from an organism to the external environment. Thus, segments of vectors and/or vectors of the invention may include a transcriptional regulatory region such as, for example, a transcriptional initiation region. One skilled in the art will appreciate that a multitude of transcriptional initiation sequences have been isolated and are available, including thymidine kinase promoters, beta-actin promoters, immunoglobin promoters, methallothionein promoters, human cytomegalovirus promoters and SV40 promoters.

One embodiment of the invention provides a method of producing a gap-filled vector. A gap-filled vector may undergo homologous recombination and insertion of a target nucleic acid according to the invention by filling in the region (gap) between the sequences homologous to the 5' and the 3' regions of the target nucleic acid. Hence, in some embodiments, one would contact the instant cloning system with a target nucleic acid under conditions that allow homologous recombination.

Another method combines: (i) a first segment including a first nucleic acid homologous to the 5' terminus of a target nucleic acid, a first selectable marker and a first cyclization element; (ii) a target nucleic acid; and (iii) a second segment including a second nucleic acid homologous to the 3' terminus of a target nucleic acid, a second selectable marker and a second cyclization element, under conditions which allow homologous recombination. One embodiment of the invention produces a gap-filled vector by homologous recombination between the two arms and the target nucleic acid. The exchange between the homologous regions found in the arms and the target nucleic acid is effected by homologous recombination at any point between the homologous nucleic acids. With respect to a circular vector of the present invention, the "gap filling" essentially is insertion (i.e., subcloning) of the target sequence into the vector.

Homologous recombination may be effected *in vitro* according to methodologies well known in the art. For example, the method of the invention can be practiced using yeast lysate preparations. Homologous recombination may take place *in vivo.* Hence, the method of the invention may be practiced using any host cell capable of supporting homologous recombination events such as, for example, bacteria, yeast and mammalian cells. One skilled in the art will appreciate that the choice of a suitable host depends on the particular combination of selectable markers used in the cloning system of the method.

Techniques that may be used to introduce the vector into a host cell of interest include calcium phosphate/DNA coprecipitation, electroporation, bacterial-protoplast fusion, microinjection of DNA into the nucleus and so on. One of skill will appreciate that a number of protocols may be used virtually interchangeably, for example, to transfect mammalian cells, as set forth for example in Keown et al. (Meth. Enzymol. 185:527 537, 1990).

Transformation may be achieved by using a soil bacterium, such as *Agrobacterium tumefaciens. Agrobacterium tumefaciens* may carry an engineered plasmid vector, or carrier of selected extra genes. Plant tissue, such as leaves, are cut in small pieces, eg. 10x1 10mm, and soaked for 10 minutes in a fluid containing suspended *Agrobacterium.* Some cells along the cut is transformed by the bacterium, that inserts its DNA into the cell. Placed on selectable rooting and shooting media, the plants will regrow. Some plants species can be transformed just by dipping the flowers into suspension of *Agrobacteria* and then planting the seeds in a selective medium.

Another methodology is use of a "gene gun" approach. The gene gun is part of a method called the biolistic (also known as bioballistic) method, and under certain conditions, DNA (or RNA) become "sticky," adhering to biologically inert particles such as metal atoms (usually tungsten or gold). By accelerating this DNA-particle complex in a partial vacuum and placing the target tissue within the acceleration path, DNA is effectively introduced. Uncoated metal particles could also be shot through a solution containing DNA surrounding the cell thus picking up the genetic material and proceeding into the living cell. A perforated plate stops the shell cartridge but allows the slivers of metal to pass through and into the living cells on the other side. The cells that take up the desired DNA, identified through the use of a marker gene (in plants the use of GUS is most common), are then cultured to replicate the gene and possibly cloned. The biolistic method is most useful for inserting genes into plant cells such as pesticide or herbicide resistance. Different methods have been used to accelerate the particles: these include pneumatic devices; instruments utilizing a mechanical impulse or macroprojectile; centripetal, magnetic or electrostatic forces; spray or vaccination guns; and apparatus based on acceleration by shock wave, such as electric discharge (for example, see Christou and McCabe, 1992, Agracetus, Inc. Particle Gun Transformation of Crop Plants Using Electric Discharge (ACCELL™ Technology)).

Transformation can be performed, for example, according to the method of Cohen et al. (Proc. Natl. Acad. Sci. USA, 69:2110 (1972)), the protoplast method (Mol. Gen. Genet., 168:111 (1979)), or the competent method (J. Mol. Biol., 56:209 (1971)) when the hosts are bacteria (*E. coli, Bacillus subtilis,* and such), the method of Hinnen et al. (Proc. Natl. Acad. Sci. USA, 75:1927 (1978)), or the lithium method (J. Bacteriol., 153:163 (1983)) when the host is *S. cerevisiae,* the method of Graham (Virology, 52:456 (1973)) when the hosts are animal cells, and the method of Summers et al. (Mol. Cell. Biol., 3:2156-2165 (1983)) when the hosts are insect cells. Typically, following a transformation event, potential transformants are plated on nutrient media for selection and/or cultivation.

The nutrient media preferably comprises a carbon source, an inorganic nitrogen source, or an organic nitrogen source necessary for the growth of host cells (transformants). Examples of the carbon source are glucose, dextran, soluble starch, and sucrose, and examples of the inorganic or organic nitrogen source are ammonium salts, nitrates, amino acids, corn steep liquor, peptone, casein, meat extract, soy bean cake, and potato extract. If desired, the media may comprise other nutrients (for example, an inorganic salt (for example, calcium chloride, sodium dihydrogenphosphate, and magnesium chloride), vitamins, antibiotics (for example, tetracycline, neomycin, ampicillin, kanamycin, etc.). Media for some photosynthetic organisms may not require a carbon source as such organisms may be photoautotrophs and, thus, can produce their own carbon sources.

Cultivation and/or selection are performed by methods known in the art. Cultivation and selection conditions such as temperature, pH of the media, and cultivation time are selected appropriately for the vectors, host cells and methods of the present invention. One of skill in the art will recognize that there are numerous specific media and cultivation/selection conditions which can be used depending on the type of host cell (transformant) and the nature of the vector (e.g., which selectable markers are present). The media herein are merely described by way of example and are not limiting.

When the hosts are bacteria, actinomycetes, yeast, or filamentous fungi, media comprising the nutrient source(s) mentioned above are appropriate. When the host is *E. coli,* examples of preferable media are LB media, M9 media (Miller et al. Exp. Mol. Genet., Cold Spring Harbor Laboratory, p.431 (1972)), and so on. When the host is yeast, an example of medium is Burkhoter minimal medium (Bostian, Proc. Natl. Acad. Sci. USA, 77:4505 (1980)).

The selection of vectors in yeast may be accomplished by the use of yeast selectable markers. Examples include, but are not limited to, *HIS3, TRP1, URA3, LEU2* and *ADE* markers. In some embodiments of the invention, a vector or segment thereof may comprise two or more selectable markers. Thus, in one embodiment, a segment of a vector of the present invention may comprise an *ADE* marker to be lost upon homologous recombination with the target nucleic acid, and a *HIS3* marker. The other segment may comprise a *TRP1* marker. Selection is achieved by growing transformed cells on a suitable drop-out selection media (see e.g., Watson et al. (1992) Recombinant DNA, 2.sup.nd ed., Freeman and Co., New York, N.Y.). For example, *NIS3* allows for selection of cells containing the first segment. *TRP1* allows for selection of cells containing the second segment. *ADE* allows screening and selection of clones in which homologous recombination took place. *ADE* enables color selection (red).

Recombinant yeast cells may be selected using the selectable markers described herein according to methods well known in the art. Hence, one skilled in the art will appreciate that recombinant yeast cells harboring a gap-filled vector of the invention may be selected on the basis of the selectable markers included therein. For example, recombinant vectors carrying *HIS3* and *TRP1* may be selected by growing transformed yeast cells in the presence of drop-out selection media lacking histidine and tryptophan. Isolated positive clones may be purified further and analyzed to ascertain the presence and structure of the recombinant vector of the invention by, e.g., restriction analysis, electrophoresis, Southern blot analysis, polymerase chain reaction or the like. The invention further provides gap-filled vectors engineered according to the method of the invention. Such a vector is the product of homologous recombination between the segments or vectors of the invention and a target nucleic acid of choice. The invention also provides a prokaryotic cell and/or a eukaryotic host cell harboring the cloning system or vector according to the invention. The organism can be unicellular or multicellular. The organism may be naturally photosynthetic or naturally non-photosynthetic. Other examples of organisms that can be transformed include vascular and non-vascular organisms. When hosts, such as plant, yeast, animal, algal, or insect cells are used, a vector of the present invention may contain, at least, a promoter, an initiation codon, the polynucleotide encoding a protein, and a termination codon. The vectors of the present invention may also contain, if required, a polynucleotide for gene amplification (marker) that is usually used.

**Products**

The vectors of the present invention may comprise sequences that result in production of a product naturally, or not naturally, produced in the organism comprising the vector. In some instances the product encoded by one or more sequences on a vector is a polypeptide, for example an enzyme. Enzymes utilized in practicing the present invention may be encoded by nucleotide sequences derived from any organism, including bacteria, plants, fungi and animals. Vectors may also comprise nucleotide sequences that affect the production or secretion of a product from the organism. In some instances, such nucleotide sequence(s) encode one or more enzymes that function in isoprenoid biosynthetic pathway. Examples of polypeptides in the isoprenoid biosynthetic pathway include synthases such as C5, C10, C15, C20, C30, and C40 synthases. In some instances, the enzymes are isoprenoid producing enzymes. In some instances, an isoprenoid producing enzyme produces isoprenoids with two phosphate groups (e.g., GPP synthase, FPP synthase, DMAPP synthase). In other instances, isoprenoid producing enzymes produce isoprenoids with zero, one, three or more phosphates or may produce isoprenoids with other functional groups. Polynucleotides encoding enzymes and other proteins useful in the present invention may be isolated and/or synthesized by any means known in the art, including, but not limited to cloning, sub-cloning, and PCR.

An isoprenoid producing enzyme for use in the present invention may also be botryococcene synthase, β-caryophyllene synthase, germacrene A synthase, 8-epicedrol synthase, valencene synthase, (+)-δ-cadinene synthase, germacrene C synthase, (E)-β-farnesene synthase, casbene synthase, vetispiradiene synthase, 5-epi-aristolochene synthase, aristolchene synthase, α-humulene, (E,E)-α-farnesene synthase, (-)-β-pinene synthase, γ-terpinene synthase, limonene cyclase, linalool synthase, (+)-bornyl diphosphate synthase, levopimaradiene synthase, isopimaradiene synthase, (E)-γ-bisabolene synthase, copalyl pyrophosphate synthase, kaurene synthase, longifolene synthase, γ-humulene synthase, δ-selinene synthase, β-phellandrene synthase, terpinolene synthase, (+)-3-carene synthase, syn-copalyl diphosphate synthase, α-terpineol synthase, syn-pimara-7,15-diene synthase, ent-sandaaracopimaradiene synthase, sterner- 13 -ene synthase, E-β-ocimene, S-linalool synthase, geraniol synthase, γ-terpinene synthase, linalool synthase, E-β-ocimene synthase, epi-cedrol synthase, α-zingiberene synthase, guaiadiene synthase, cascarilladiene synthase, cis-muuroladiene synthase, aphidicolan-16b-ol synthase, elizabethatriene synthase, sandalol synthase, patchoulol synthase, zinzanol synthase, cedrol synthase, scareol synthase, copalol synthase, or manool synthase.

Other enzymes which may be produced by vectors of the present invention include biomass-degrading enzymes. Non-limiting examples of biomass-degrading enzymes include: cellulolytic enzymes, hemicellulolytic enzymes, pectinolytic enzymes, xylanases, ligninolytic enzymes, cellulases, cellobiases, softening enzymes (e.g., endopolygalacturonase), amylases, lipases, proteases, RNAses, DNAses, inulinase, lysing enzymes, phospholipases, pectinase, pullulanase, glucose isomerase, endoxylanase, beta-xylosidase, alpha-L-arabinofuranosidase, alpha-glucoronidase, alpha-galactosidase, acetylxylan esterase, and feruloyl esterase. Examples of genes that encode such enzymes include, but are not limited to, amylases, cellulases, hemicellulases, (e.g., β-glucosidase, endocellulase, exocellulase), exo-β-glucanase, endo-β-glucanase and xylanse (endoxylanase and exoxylanse). Examples of ligninolytic enzymes include, but are not limited to, lignin peroxidase and manganese peroxidase from *Phanerochaete chryososporium.* One of skill in the art will recognize that these enzymes are only a partial list of enzymes which could be used in the present invention.

The present invention contemplates making enzymes that contribute to the production of fatty acids, lipids or oils by transforming host cells (e.g., alga cells such as *C*. *reinhardtii, D. salina, H. pluvalis* and cyanobacterial cells) and/or organisms comprising host cells with nucleic acids encoding one or more different enzymes. In some embodiments the enzymes that contribute to the production of fatty acids, lipids or oils are anabolic enzymes. Some examples of anabolic enzymes that contribute to the synthesis of fatty acids include, but are not limited to, acetyl-CoA carboxylase, ketoreductase, thioesterase, malonyltransferase, dehydratase, acyl-CoA ligase, ketoacylsynthase, enoylreductase and a desaturase. In some embodiments the enzymes are catabolic or biodegrading enzymes. In some embodiments, a single enzyme is produced.

Some host cells may be transformed with multiple genes encoding one or more enzymes. For example, a single transformed cell may contain exogenous nucleic acids encoding enzymes that make up an entire fatty acid synthesis pathway. One example of a pathway might include genes encoding an acetyl CoA carboxylase, a malonyltransferase, a ketoacylsynthase, and a thioesterase. Cells transformed with entire pathways and/or enzymes extracted from them, can synthesize complete fatty acids or intermediates of the fatty acid synthesis pathway. In some embodiments constructs may contain multiple copies of the same gene, and/or multiple genes encoding the same enzyme from different organisms, and/or multiple genes with mutations in one or more parts of the coding sequences.

In some instances, a product (e.g. fuel, fragrance, insecticide) is a hydrocarbon-rich molecule, e.g. a terpene. A terpene (classified by the number of isoprene units) can be a hemiterpene, monoterpene, sesquiterpene, diterpene, triterpene, or tetraterpene. In specific embodiments the terpene is a terpenoid (aka isoprenoid), such as a steroid or carotenoid. Subclasses of carotenoids include carotenes and xanthophylls. In specific embodiments, a fuel product is limonene, 1, 8-cineole, α-pinene, camphene, (+)-sabinene, myrcene, abietadiene, taxadiene, farnesyl pyrophosphate, amorphadiene, (E)-α-bisabolene, beta carotene, alpha carotene, lycopene, or diapophytoene. Some of these terpenes are pure hydrocarbons (e.g. limonene) and others are hydrocarbon derivatives (e.g. cineole).

Examples of fuel products include petrochemical products and their precursors and all other substances that may be useful in the petrochemical industry. Fuel products include, for example, petroleum products, and precursors of petroleum, as well as petrochemicals and precursors thereof. The fuel product may be used for generating substances, or materials, useful in the petrochemical industry, including petroleum products and petrochemicals. The fuel or fuel products may be used in a combustor such as a boiler, kiln, dryer or furnace. Other examples of combustors are internal combustion engines such as vehicle engines or generators, including gasoline engines, diesel engines, jet engines, and others. Fuel products may also be used to produce plastics, resins, fibers, elastomers, lubricants, and gels.

Examples of products contemplated herein include hydrocarbon products and hydrocarbon derivative products. A hydrocarbon product is one that consists of only hydrogen molecules and carbon molecules. A hydrocarbon derivative product is a hydrocarbon product with one or more heteroatoms, wherein the heteroatom is any atom that is not hydrogen or carbon. Examples of heteroatoms include, but not limited to, nitrogen, oxygen, sulfur, and phosphorus. Some products are hydrocarbon-rich, wherein as least 50%, 60%, 70%, 80%, 90%, or 95% of the product by weight is made up carbon and hydrogen.

Fuel products, such as hydrocarbons, may be precursors or products conventionally derived from crude oil, or petroleum, such as, but not limited to, liquid petroleum gas, naptha (ligroin), gasoline, kerosene, diesel, lubricating oil, heavy gas, coke, asphalt, tar, and waxes. For example, fuel products may include small alkanes (for example, 1 to approximately 4 carbons) such as methane, ethane, propane, or butane, which may be used for heating (such as in cooking) or making plastics. Fuel products may also include molecules with a carbon backbone of approximately 5 to approximately 9 carbon atoms, such as naptha or ligroin, or their precursors. Other fuel products may be about 5 to about 12 carbon atoms or cycloalkanes used as gasoline or motor fuel. Molecules and aromatics of approximately 10 to approximately 18 carbons, such as kerosene, or its precursors, may also be fuel products. Fuel products may also include molecules, or their precursors, with more than 12 carbons, such as used for lubricating oil. Other fuel products include heavy gas or fuel oil, or their precursors, typically containing alkanes, cycloalkanes, and aromatics of approximately 20 to approximately 70 carbons. Fuel products also includes other residuals from crude oil, such as coke, asphalt, tar, and waxes, generally containing multiple rings with about 70 or more carbons, and their precursors.

**Host Cells and Organisms**

Examples of organisms that can be transformed using the vectors and methods herein include vascular and non-vascular organisms. The organism can be prokaroytic or eukaroytic. The organism can be unicellular or multicellular.

Eukaryotic cells, such as a fungal cell (e.g., *Saccharomyces cerevisiae, Schizosaccharomyces pombe* or *Ustilago maydis*) may be transformed using the methods and compositions of the present invention. Methods for introducing nucleic acids in a fungal/yeast cells are well known in the art. Hence, such a step may be accomplished by conventional transformation methodologies. Non-limiting examples of suitable methodologies include electroporation, alkali cations protocols and spheroplast transformation.

Examples of non-vascular photosynthetic organisms include bryophtyes, such as marchantiophytes or anthocerotophytes. In some instances the organism is a cyanobacteria. In some instances, the organism is algae (e.g., macroalgae or microalgae). The algae can be unicellular or multicellular algae. In some instances the organism is a rhodophyte, chlorophyte, heterokontophyte, tribophyte, glaucophyte, chlorarachniophyte, euglenoid, haptophyte, cryptomonad, dinoflagellum, or phytoplankton.

The methods of the present invention are exemplified using the microalga, *C*. *reinhardtii.* The use of microalgae to express a polypeptide or protein complex according to a method of the invention provides the advantage that large populations of the microalgae can be grown, including commercially (Cyanotech Corp.; Kailua-Kona HI), thus allowing for production and, if desired, isolation of large amounts of a desired product. However, the ability to express, for example, functional polypeptides, including protein complexes, in the chloroplasts of any plant and/or modifiy the chloroplasts or any plant allows for production of crops of such plants and, therefore, the ability to conveniently produce large amounts of the polypeptides. Accordingly, the methods of the invention can be practiced using any plant having chloroplasts, including, for example, macroalgae, for example, marine algae and seaweeds, as well as plants that grow in soil, for example, corn (*Zea mays*), Brassica sp. (e.g., *B. napus, B. rapa, B. juncea*), particularly those Brassica species useful as sources of seed oil, alfalfa (*Medicago sativa*), rice (*Oryza sativa*), rye (*Secale cereale*), sorghum (*Sorghum bicolor*, *Sorghum vulgare*), millet (e.g., pearl millet (*Pennisetum glaucum*), proso millet (*Panicum miliaceum*), foxtail millet (*Setaria italica*), finger millet (*Eleusine coracana*)), sunflower (*Helianthus annuus*), safflower (*Carthamus tinctorius*), wheat (*Triticum aestivum*), soybean (*Glycine max*), tobacco (*Nicotiana tabacum*), potato (*Solanum tuberosum*), peanuts (*Arachis hypogaea*), cotton (*Gossypium barbadense, Gossypium hirsutum*), sweet potato (*Ipomoea batatus*), cassaya (*Manihot esculenta*), coffee (*Cofea* spp.), coconut (*Cocos nucifera*), pineapple (*Ananas comosus*), citrus trees (*Citrus* spp.), cocoa (*Theobroma cacao*), tea (*Camellia sinensis*), banana (*Musa* spp.), avocado (*Persea ultilane*), fig (*Ficus casica*), guava (*Psidium guajava*), mango (*Mangifera indica*), olive (*Olea europaea*), papaya (*Carica papaya*), cashew (*Anacardium occidentale*), macadamia (*Macadamia integrifolia*), almond (*Prunus amygdalus*), sugar beets (*Beta vulgaris*), sugar cane (*Saccharum* spp.), oats, duckweed (*Lemna*), barley, tomatoes (*Lycopersicon esculentum*), lettuce (e.g., *Lactuca sativa*), green beans (*Phaseolus vulgaris*), lima beans (*Phaseolus limensis*), peas (*Lathyrus* spp.), and members of the genus *Cucumis* such as cucumber (*C*. *sativus),* cantaloupe (*C*. *cantalupensis),* and musk melon (*C. melo*). Ornamentals such as azalea (*Rhododendron* spp.), hydrangea (*Macrophylla hydrangea*), hibiscus (*Hibiscus rosasanensis*), roses (*Rosa* spp.), tulips (*Tulipa* spp.), daffodils (*Narcissus* spp.), petunias (*Petunia hybrida*), carnation (*Dianthus caryophyllus*), poinsettia (*Euphorbia pulcherrima*), and chrysanthemum are also included. Additional ornamentals useful for practicing a method of the invention include impatiens, Begonia, Pelargonium, Viola, Cyclamen, Verbena, Vinca, Tagetes, Primula, Saint Paulia, Agertum, Amaranthus, Antihirrhinum, Aquilegia, Cineraria, Clover, Cosmo, Cowpea, Dahlia, Datura, Delphinium, Gerbera, Gladiolus, Gloxinia, Hippeastrum, Mesembryanthemum, Salpiglossos, and Zinnia. Conifers that may be employed in practicing the present invention include, for example, pines such as loblolly pine (*Pinus taeda*), slash pine (*Pinus elliotii*)*,* ponderosa pine (*Pinus ponderosa*), lodgepole pine (*Pinus* contorta), and Monterey pine (*Pinus radiata*)*,* Douglas-fir (*Pseudotsuga menziesii)*; Western hemlock (*Tsuga ultilane*); Sitka spruce (*Picea glauca*); redwood (*Sequoia sempervirens*); true firs such as silver fir (*Abies amabilis*) and balsam fir (*Abies balsamea*); and cedars such as Western red cedar (*Thuja plicata*) and Alaska yellow-cedar (*Chamaecyparis nootkatensis*)*.*

Leguminous plants useful for practicing a method of the invention include beans and peas. Beans include guar, locust bean, fenugreek, soybean, garden beans, cowpea, mung bean, lima bean, fava bean, lentils, chickpea, etc. Legumes include, but are not limited to, *Arachis,* e.g., peanuts, *Vicia,* e.g., crown vetch, hairy vetch, adzuki bean, mung bean, and chickpea, *Lupinus,* e.g., lupine, trifolium, *Phaseolus,* e.g., common bean and lima bean, *Pisum,* e.g., field bean, *Melilotus,* e.g., clover, *Medicago,* e.g., alfalfa, *Lotus,* e.g., trefoil, lens, e.g., lentil, and false indigo. Preferred forage and turf grass for use in the methods of the invention include alfalfa, orchard grass, tall fescue, perennial ryegrass, creeping bent grass, and redtop. Other plants useful in the invention include *Acacia,* aneth, artichoke, arugula, blackberry, canola, cilantro, clementines, escarole, eucalyptus, fennel, grapefruit, honey dew, jicama, kiwifruit, lemon, lime, mushroom, nut, okra, orange, parsley, persimmon, plantain, pomegranate, poplar, radiata pine, radicchio, Southern pine, sweetgum, tangerine, triticale, vine, yams, apple, pear, quince, cherry, apricot, melon, hemp, buckwheat, grape, raspberry, chenopodium, blueberry, nectarine, peach, plum, strawberry, watermelon, eggplant, pepper, cauliflower, *Brassica,* e.g., broccoli, cabbage, ultilan sprouts, onion, carrot, leek, beet, broad bean, celery, radish, pumpkin, endive, gourd, garlic, snapbean, spinach, squash, turnip, ultilane, chicory, groundnut and zucchini. Thus, the compositions contemplated herein include host organisms comprising any of the above nucleic acids. The host organism can be any chloroplast-containing organism.

The term "plant" is used broadly herein to refer to a eukaryotic organism containing plastids, particularly chloroplasts, and includes any such organism at any stage of development, or to part of a plant, including a plant cutting, a plant cell, a plant cell culture, a plant organ, a plant seed, and a plantlet. A plant cell is the structural and physiological unit of the plant, comprising a protoplast and a cell wall. A plant cell can be in the form of an isolated single cell or a cultured cell, or can be part of higher organized unit, for example, a plant tissue, plant organ, or plant. Thus, a plant cell can be a protoplast, a gamete producing cell, or a cell or collection of cells that can regenerate into a whole plant. As such, a seed, which comprises multiple plant cells and is capable of regenerating into a whole plant, is considered plant cell for purposes of this disclosure. A plant tissue or plant organ can be a seed, protoplast, callus, or any other groups of plant cells that is organized into a structural or functional unit. Particularly useful parts of a plant include harvestable parts and parts useful for propagation of progeny plants. A harvestable part of a plant can be any useful part of a plant, for example, flowers, pollen, seedlings, tubers, leaves, stems, fruit, seeds, roots, and the like. A part of a plant useful for propagation includes, for example, seeds, fruits, cuttings, seedlings, tubers, rootstocks, and the like.

Eukaryotic host cells may be a fungal cell (e.g., *S. cerevisiae, Sz. pombe* or *U. maydis*). Examples of prokaryotic host cells include *E. coli* and *B. subtilis,* cyanobacteria and photosynthetic bacteria (e.g. species of the genus *Synechocystis* or the genus *Synechococcus* or the genus *Athrospira*). Examples of non-vascular plants which may be a host organism (or the source of target DNA) include bryophtyes, such as marchantiophytes or anthocerotophytes. In some instances, the organism is algae (e.g., macroalgae or microalgae, such as *Chlamydomonas reinhardtii, Chorella vulgaris, Dunaliella salina, Haematococcus pluvalis, Scenedesmus* ssp.). The algae can be unicellular or multicellular algae. In some instances the organism is a rhodophyte, chlorophyte, heterokontophyte, tribophyte, glaucophyte, chlorarachniophyte, euglenoid, haptophyte, cryptomonad, dinoflagellum, or phytoplankton. In other instancesOne of skill in the art will recognize that these organisms are given merely as examples and other organisms may be substituted where appropriate positive and negative selectable markers are available.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

### EXAMPLES

### Example 1: DNA Purification and Analysis

DNA is isolated and analyzed according to methods known in the art.

To prepare DNA from *Chlamydomonas reinhardtii* to use as a template for PCR, 10⁶ algae cells (from agar plate or liquid culture) are suspended in 10 mM EDTA and heated to 95°C for 10 minutes, then cooled to near 23°C. The solution is added to the PCR mixture directly.

To prepare purified chloroplast DNA from *Chlamydomonas reinhardtii*, 5x10⁸ algae cells are collected from liquid culture by centrifugation at 3000 x g for 10 min, washed once with water, centrifuged at 3000 x g for 10 min, resuspended in 10 mL of lysis solution (10 mM Tris pH=8.0, 10 mM EDTA, 150 mM NaCl, 2% SDS, 2% Sarkosyl, and 25 ug/mL Pronase (Roche)), and incubated at 37°C for 1 hour. The lysate is then gently extracted with phenol/chloroform followed by two chloroform washes. Total DNA is isolated by ethanol precipitation and resuspension in resuspension buffer (10 mM Tris pH=7.4, 1 mM EDTA, and 0.1 mg/mL RNase). Chloroplast DNA can be purified by adding of denaturing solution (200 mM NaOH and 1% SDS (w/v)) is added to the resuspended DNA and inverted several times. Neutralizing solution (3.0 M potassium acetate, pH=5.5) is added, mixed, incubated on ice for 10 min and centrifuged at 15000 RPM for 30 min. The supernatant is decanted and applied to a QIAGEN-tip 500 and the DNA is isolated according to the QIAGEN Plasmid Maxi Kit.

An alternative method for preparing purified chloroplast DNA from *Chlamydomonas reinhardtii* involves embedding algae cells or purified chloroplasts in low-melt agarose plugs to prevent shearing of the DNA. Chloroplasts are isolated by lysing whole cells in a nitrogen decompression chamber and separating intact cells and debris from the chloroplasts by percoll density gradient centrifugation. To lyse the cells and/or chloroplasts, the plugs are incubated at 55°C for 36 hours in lysis buffer (0.5 M EDTA, 1% Sarkosyl, 0.2 mg/mL proteinase K). When lysis is complete, plugs are washed 3 times with TE, and then stored in storage buffer (10 mM Tris pH=7.4, 1 mM EDTA). To release chloroplast DNA into solution, the plugs are washed 3 times with 30 mM NaCl, and melted at 65°C for 10 min. The melted plugs are shifted to 42°C and treated with β-agarase (New England Biolabs) for 1 hour. The solution of DNA can be used directly for downstream applications or ethanol precipitated to concentrate the sample.

To prepare DNA from yeast to use as a template for PCR, 10⁶ yeast cells (from agar plate or liquid culture) are suspended in lysis buffer (6 mM KHPO4, pH=7.5, 6 mM NaCl, 3% glycerol, 1U/mL zymolyase) and heated to 37°C for 30 min, 95°C for 10 minutes, then cooled to near 23°C. The solution is added to the PCR mixture directly.

To prepare plasmid DNA from yeast, desired clones are grown in selective liquid media (e.g., CSM-Trp) to saturation at 30°C. Cells are collected by centrifugation at 3000 x g for 10 minutes and resuspended in 150 uL of lysis buffer (1 M sorbitol, 0.1 M sodium citrate, 0.06 M EDTA pH=7.0, 100 mM beta-mercaptoethanol, and 2.5 mg/mL zymolyase). The solution is incubated for 1 hr at 37°C. 300 uL of denaturing solution (1% SDS and 0.2N NaOH) is added and solution is incubated at 60°C for 15 min. 150 uL of neutralizing solution (3M potassium acetate, pH=4.8) is added and the solution is incubated on ice for 10 min. The solution is centrifuged at 14,000 RPM for 10 min and the supernatant is transferred to another tube. 1 mL of isopropanol is added, the mixture is gently mixed and centrifuged at 14,000 RPM for 10 min. The pellet is washed once with 1 mL of 70% ethanol and centrifuged at 14,000 RPM for 10 min. The DNA pellet is air-dried and resuspended in 60 uL of resuspension buffer (10 mM Tris pH=7.4, 1 mM EDTA, and 0.1 mg/mL RNase).

To prepare plasmid DNA from bacteria, cells are grown to saturation at 37°C in LB containing the appropriate antibiotic (Kan or Amp). If the DNA of interest contains standard replication elements, cells are harvested by centrifugation. If the DNA of interest contains P1 replication elements, saturated cell cultures are diluted 1:20 in LB+Kan+IPTG and grown for 4 hours at 37°C, then harvested. The Plasmid Maxi kit (QIAGEN) is used to prepare plasmid DNA from the cell pellets.

For illustrative purposes, and without limiting the invention to the specific methods described, DNA samples prepared from algae, yeast, or bacteria (in plugs or in solution) are analyzed by pulse-field gel electrophoresis (PFGE), or digested with the appropriate restriction endonuclease (e.g., Smal) and analyzed by PFGE, conventional agarose gel electrophoresis, and/or Southern blot. Standard protocols useful for these purposes are fully described in Gemmill et al. (in "Advances in Genome Biology", Vol. 1, "Unfolding The Genome," pp 217 251, edited by Ram S. Verma).

One of skill will appreciate that many other methods known in the art may be substituted in lieu of the ones specifically described or referenced.

### Example 2: Transformation methods

*E. coli* strains DH10B or Genehog are made electrocompetent by growing the cells to an OD ₆₀₀ of 0.7, then collected and washed twice with ice-cold 10% glycerol, flash frozen in a dry-ice ethanol bath and kept at -80°C. Total yeast or algae DNA is prepared and electroporated into *E*. *coli* by using, for example, a 0.1 cm cuvette at 1,800 V, 200 ohms and 25 mF in a Bio-Rad Gene Pulsar Electroporator. Cells are allowed to recover and clones are selected on agar growth media containing one or more antibiotics, such as kanamycin (50 µg/mL), ampicillin (100 µg/mL), gentamycin (50 µg/mL), tetracycline (51 µg/mL), or chloramphenicol (34 µg/mL).

Yeast strains YPH857, YPH858 or AB1380 may be transformed by the lithium acetate method as described in Sheistl & Geitz (Curr. Genet. 16:339 346, 1989) and Sherman et al., "Laboratory Course Manual Methods in Yeast Genetics" (Cold Spring Harbor Laboratory Press, Cold Spring Harbor, 1986) or a spheroplast method such as the one described by Sipiczki et al., Curr. Microbiol., 12(3):169-173 (1985). Yeast transformants are selected and screened on agar media lacking amino and/or nucleic acids, such as tryptophan, leucine, or uracil. Standard methods for yeast growth and phenotype testing are employed as described by Sherman et al., supra.

Algae strains csc 137c (mt+), W1.1, or W1-1 may be transformed by particle bombardment. Cells are grown to late log phase (approximately 7 days) in TAP medium in the presence or absence of 0.5 mM 5-fluorodeoxyuridine (Gorman and Levine, Proc. Natl. Acad. Sci., USA 54:1665-1669, 1965, which is incorporated herein by reference) at 23°C under constant illumination of 450 Lux on a rotary shaker set at 100 rpm. Fifty ml of cells are harvested by centrifugation at 4,000xg at 23°C for 5 min. The supernatant is decanted and cells resuspended in 4 ml TAP medium for subsequent chloroplast transformation by particle bombardment (Cohen et al., supra, 1998).

One of skill will appreciate that many other transformation methods known in the art may be substituted in lieu of the ones specifically described or referenced herein.

### Example 3: A hybrid gap-filling vector to capture a chloroplast genome

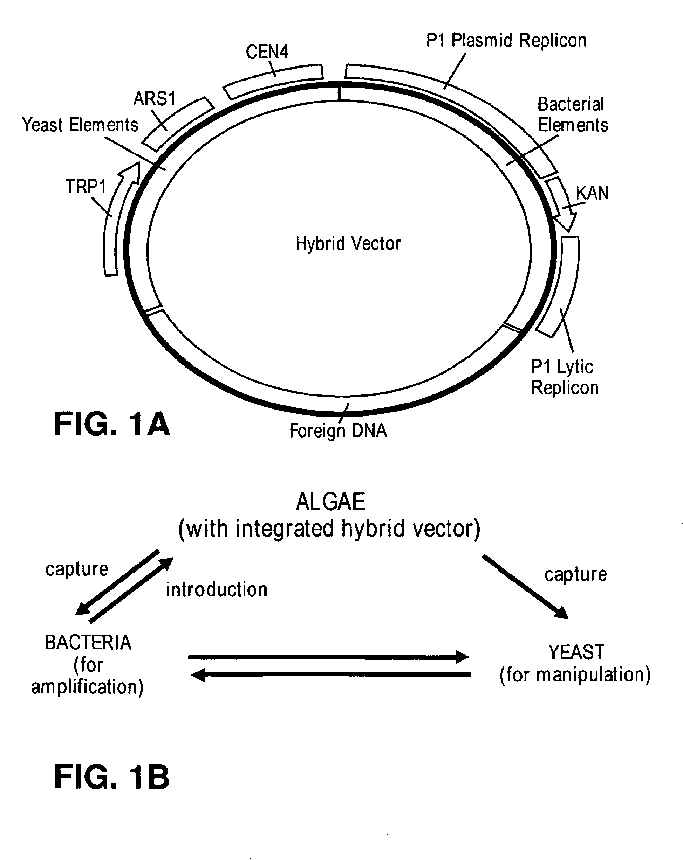

In this example, a system is established using a hybrid gap-filling vector to capture chloroplast DNA (**FIG. 1**). The hybrid gap filling vector backbone contains yeast elements that allow it to function as a yeast artificial plasmid (YAP) and bacterial elements that allow it to function as a plasmid artificial chromosome (PAC). The yeast elements include a yeast selection marker sequence (e.g. *TRP1* or *LEU2*), a yeast centromere sequence (CEN), and a yeast autonomously replicating nucleotide sequence (ARS). Bacterial elements include a P1 or bacterial origin of replication sequence and a bacterial selection maker sequence (e.g. Kan^{r}).

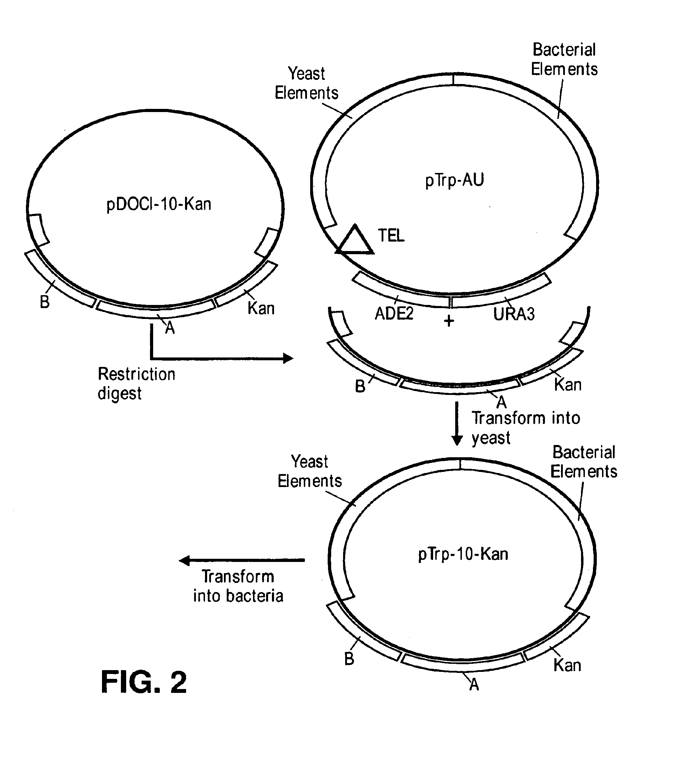

To manipulate the hybrid gap-filling vector, the vector pDOCI (SEQ ID NO. 1) was generated. Portions of pTRP-AU (**FIG. 2**) were amplified using PCR primer pairs that anneal to sites surrounding the region encompassing TEL, *ADE2*, and *URA3*. One pair amplifies a region within the yeast elements (SEQ ID NOs. 21 and 22) and the other pair amplifies a region within the bacterial elements (SEQ ID NOs. 23 and 24). The PCR products were assembled into a single DNA fragment by PCR assembly using a single primer pair (SEQ ID NOs. 21 and 24). The assembled product was digested with *Not*I and ligated to *Not*I-digested pUC-SE (SEQ ID NO. 2) to form pDOCI (SEQ ID NO. 1).

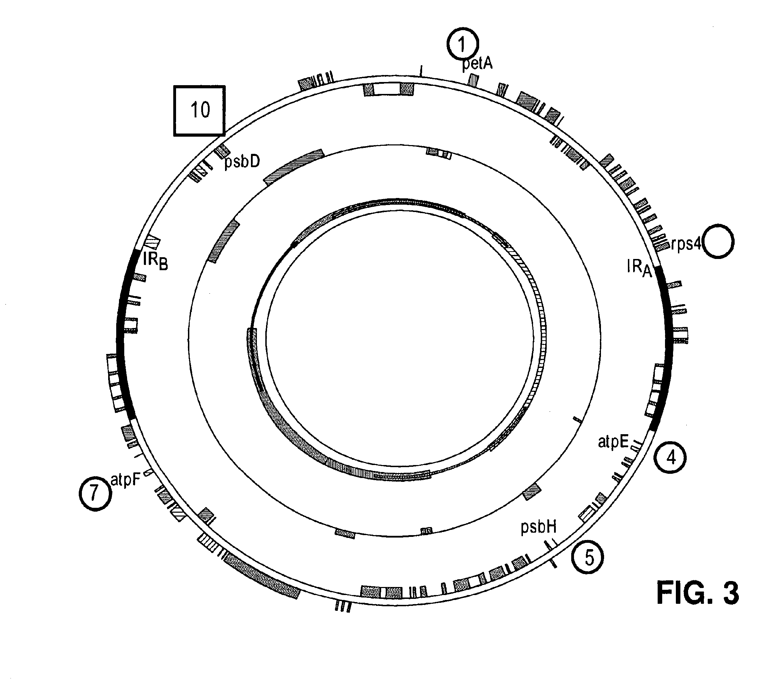

To adapt the hybrid gap-filling vector to capture chloroplast DNA, the vector pDOCI-10 (SEQ ID NO. 3) was generated. Portions of the *C*. *reinhardtii* chloroplast genome were PCR amplified using two primer pairs specific for two adjacent regions (SEQ ID NOs. 25 and 26 and SEQ ID NOs. 27 and 28) near the *psbD* locus. (**Fig. 3**; indicated by number 10 surrounded by a box). Each PCR product was digested with *Not*I and I*-Sce*I and ligated to pDOCI (SEQ ID NO. 1) that was digested with I*-Sce*I to form pDOCI-10 (SEQ ID NO. 3).

To adapt pDOCI-10 to confer antibiotic resistance in algae, a selection marker was cloned. pSE-3HB-Kan (SEQ ID NO. 4), which contains a kanamycin resistance encoding gene from bacteria, which is regulated by the 5' UTR and promoter sequence for the *atpA* gene from *C*. *reinhardtii* and the 3' UTR sequence for the *rbcL* gene from from *C*. *reinhardtii,* was digested with *SnaB*I *,* which liberated the kanamycin resistance cassette. The cassett was ligated to *SnaB*I-digested pDOCI-10 to form pDOCI-10-Kan (SEQ ID NO. 5).

The hybrid gap-filling for capturing chloroplast DNA, pTRP-10-Kan (SEQ ID NO. 6), was constructed using recombination in yeast (Fig. 2). Briefly, pDOCI-10-Kan was digested with *Pac***I** and *Asc*I to liberate the cassette that introduces chloroplast genome-specific elements into the hybrid gap-filling vector. This cassette was transformed along with pTRP-AU into the yeast strain YPH858 using the lithium acetate method. Homologous recombination takes place in vivo in the transformed yeast cells. Transformants that correctly integrated with cassette were isolated based on growth on CSM-Trp agar media containing 5-fluorooratic acid (5-FOA) and by red color. 5-FOA selects for clones that lack a functional *URA3* gene and the red color results when the *ADE2* gene is eliminated. Plasmid DNA was isolated from yeast clones that were grown in CSM-Trp liquid media and transformed into *E. coli* (DH10B). To generate large amounts of pTRP-10-Kan, DH10B cells harboring pTRP-10-Kan were grown to saturation at 37°C in LB+Kan (50 ug/mL), and then diluted 1:20 in LB+Kan+IPTG and grown for 4 hours at 37°C. DNA was prepared from the bacterial culture using the Plasmid Maxi kit (QIAGEN).

The composition of the vector was verified by DNA sequencing of the entire plasmid.

### Example 4: Vectors to stabilize and/or modify chloroplast genome DNA in an exogenous host

Often, large pieces of heterologous DNA are instable in host organisms such as yeast or bacteria. This may be due to multiple factors, including, but not limited to, the presence of toxic gene products or codon bias and/or lack of selective pressure. Therefore, the target DNA within the shuttle vector may be altered within yeast or bacteria. For example, certain portions of a target DNA sequence (e.g., coding regions or promoters) may be deleted or moved by recombination within the host organism. In a similar way, when a shuttle vector carrying the target DNA is transferred back to the organism (or a closely related species) that donated the target DNA, the target DNA can become unstable.

Such sites of instability and susceptible sequences are readily discovered by determining which pieces of genomic DNA elude capture or disappear over time. These sites can be detected by comparing initially isolated gap-filling target DNA plasmids with plasmids isolated from transformed strains which have been sequentially passaged in the laboratory under conditions which select for the presence of the plasmid-encoded selectable marker (e.g., TRP1) or by comparison with native target DNA (e.g., *C*. *reinhardtii* chloroplast DNA). Such comparison may be performed, for example, by restriction fragment length polymorphism (RFLP) analysis or direct sequencing. One of skill in the art will recognize that there are multiple other protocols and methods to determine such differences.

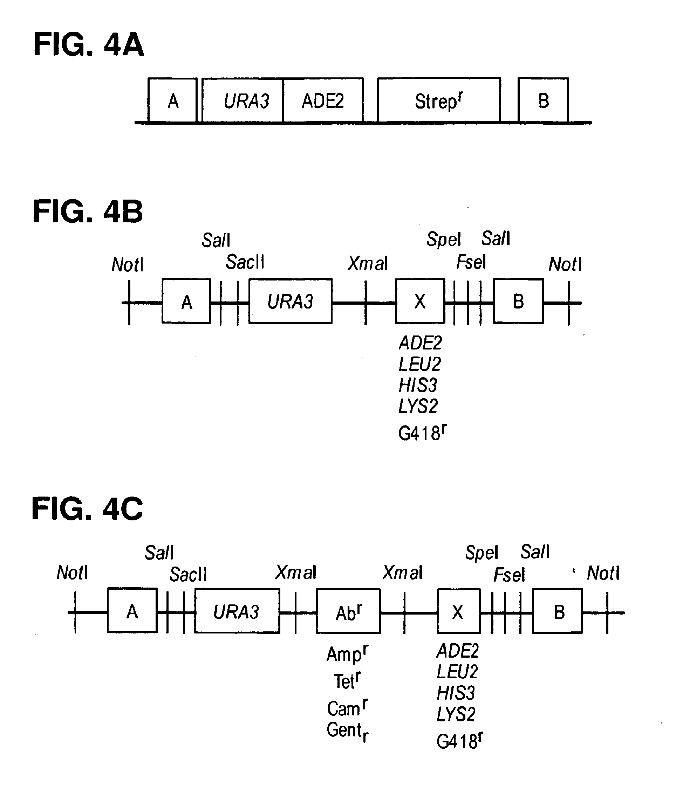

Once identified, such sites and sequences can be forced to remain through selection of markers. **Fig. 4** shows examples of arrangements of selectable markers in the multiple cloning site of a vector.

To generate a stabilization vector containing yeast and algae stability elements, the region of DNA in pTrp-AU encompassing the *ADE2* and *URA3* genes was liberated by digestion with *Sfi*I followed by gel purification of the desired fragment. The fragment was treated with Klenow fragment to create blunt ends and ligated to *Pml*I-degested pSE-3HB-Strep (SEQ ID NO. 7), creating pSE-3HB-Strep-AU (**Fig. 4B**). pSE-3HB-Strep is a vector that targets a streptomycin resistance encoding gene from bacteria, which is regulated by the 5' UTR and promoter sequence for the *atpA* gene from *C*. *reinhardtii* and the 3' UTR sequence for the *rbcL* gene from *C*. *reinhardtii,* to the 3HB locus of the *C*. *reinhardtii* chloroplast genome.

To generate vectors that target other regions of the chloroplast genome, 800-1000 bp regions were amplified using PCR primer pairs that anneal to 5' and 3' regions flanking the sites indicated by numbered circles in **Fig. 3** (site 1-5', SEQ ID NOs. 29 and 30; site 1-3', SEQ ID NOs. 31 and 32; site 3-5', SEQ ID NOs. 33 and 34; site 3-3', SEQ ID NOs. 35 and 36; site 4-5', SEQ ID NOs. 37 and 38; site 4-3', SEQ ID NOs. 39 and 40; site 5-5', SEQ ID NOs. 41 and 42; site 5-3', SEQ ID NOs. 43 and 44; and site 7-5', SEQ ID NOs. 45 and 46; site 7-3', SEQ ID NOs. 47 and 48). Each pair of PCR products was digested with *Not*I and I-*Sce*I, mixed, and ligated to *Not*I-digested pUC-SE (SEQ ID NO. 2), producing plasmids pUC1, pUC3, pUC4, pUC5 and pUC7 (named for their position of integration).

Pairs of yeast selection markers were constructed so that multiple stabilization sites could be employed simultaneously (**FIG. 4B**). Each marker pair contains the *URA3* gene (SEQ ID NO. 8), which was PCR amplified from pRS416. Each marker pair also contains the *LEU2* gene (SEQ ID NO. 9) amplified from pRS415, the *HIS3* gene (SEQ ID NO. 10) amplified from pRS413, the *ADE2* gene (SEQ ID NO. 11) amplified from pTrp-AU, the *LYS2* gene (SEQ ID NO. 12) amplified from *S. cerevisiae* genomic DNA, or the kanMX6 gene (SEQ ID NO. 13) from pFA6a-kanMX6, which confers resistance to the antifungal agent G418. The primers used for the *URA3* gene add the *Xma*I restriction site to the 5' end (SEQ ID NO. 49) and *Sal*I and *Sacl*I to the 3' end (SEQ ID NO. 50). The primers used for the *LEU2, HIS3, ADE2, LYS2,* and *G418^{r}* genes add the *Xma*I restriction site to the 5' end (SEQ ID NO. 51 for *LEU2*, SEQ ID NO. 52 for *HIS3*, SEQ ID NO. 53 for *ADE2,* SEQ ID NO. 54 for *LYS2,* and SEQ ID NO. 55 for *G418^{r}*) and *Sal*I, *Fse*I, and *Spe*I sites to the 3' end (SEQ ID NO. 56 for *LEU2,* SEQ ID NO. 57 for *HIS3,* SEQ ID NO. 58 for *ADE2*, SEQ ID NO. 59 for *LYS2.* and SEQ ID NO. 60 for *G418^{r}*). Each PCR product was digested with *Xma*I and *Sal*I, mixed pairwise, and ligated into the desired integration vector, resulting in *pUC*1*-URA3*/*ADE2,* pUC3-*URA3*/*LEU2,* pUC4-*URA3*/*HIS3,* pUC5-*URA3*/*ADE2,* and pUC7-*URA3*/*LYS2. URA3* is used in each case because it allows for positive and negative selection. Marker pairs can be introduced based on selection for either gene (in the case of a single modification), the non-*URA3* gene in the case of two or more modification. Then the markers can be removed by introducing DNA with terminal sequences homologous to those surrounding the marker pairs and selecting for growth on minimal media containing 5-FOA.

To promote sequence stability in bacteria, antibiotic resistance markers were cloned into the yeast selection marker pairs. The bacterial stability markers include, but are not limited to, the ampicillin resistance gene (Amp^{r} SEQ ID NO. 14) amplified from pET-21a, the tetracycline resistance gene (Tet^{r}, SEQ ID NO. 15) amplified from pBR322, the chloramphenicol resistance gene (Cam^{r}, SEQ ID NO. 16) amplified from pETcoco-1, and the gentamycin resistance gene (Gent^{r}, SEQ ID NO. 17) amplified from pJQ200. For each gene, primer pairs (SEQ ID NOs. 61 and 62 for Amp^{r}, SEQ ID NOs. 63 and 64 for Tet^{r}, SEQ ID NOs. 65 and 66 for Cam^{r}, and SEQ ID NOs. 67 and 68 for Gent^{r}) that add *Xma*I sites to both the 5' and 3' ends were used to PCR amplify the antibiotic resistance fragment. Each PCR product was digested *with Xma*I and ligated into *Xma*I-digested pUC1-*URA3*/*ADE2*, pUC3-*URA3*/*LEU2,* pUC4-*URA3*/*HIS3,* pUC5-*URA3*/*ADE2,* and *pUC7-URA3*/*LYS2.* **Fig. 4C** shows the arrangement of the yeast and bacterial stability markers.

### Example 5; Introduction of hybrid and stabilization vectors into a chloroplast genome

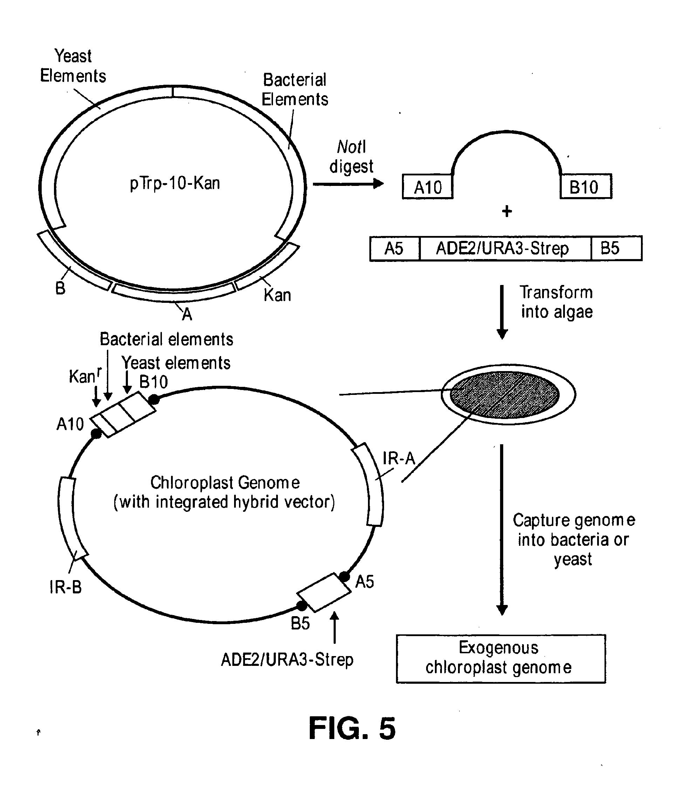

To generate a *C*. *reinhardtii* chloroplast genome that contains a hybrid vector with or without a stabilization vector, pTRP-10-Kan and pSE-3HB-Strep-AU were transformed into algae cells. pTRP-10-Kan was digested with *Not*I to linearize the vector such that the chloroplast targeting elements on are on each end (**FIG. 5**). pSE-3HB-Strep-AU was transformed as circular DNA.

For these experiments, all transformations are carried out on *C*. *reinhardtii* strain 137c (mt+). Cells are grown to late log phase (approximately 7 days) in the presence of 0.5 mM 5-fluorodeoxyuridine in TAP medium (Gorman and Levine, Proc. Natl. Acad. Sci., USA 54:1665-1669,1965, which is incorporated herein by reference) at 23°C under constant illumination of 450 Lux on a rotary shaker set at 100 rpm. Fifty ml of cells are harvested by centrifugation at 4,000xg at 23°C for 5 min. The supernatant is decanted and cells resuspended in 4 ml TAP medium for subsequent chloroplast transformation by particle bombardment (Cohen et al., supra, 1998). All transformations are carried out under kanamycin selection (100 µg/ml) in which resistance is conferred by the gene encoded by the segment in **Fig. 5** labeled "Kan." (Chlamydomonas Stock Center, Duke University).

PCR is used to identify transformed strains. For PCR analysis, 10⁶ algae cells (from agar plate or liquid culture) are suspended in 10 mM EDTA and heated to 95°C for 10 minutes, then cooled to near 23°C. A PCR cocktail consisting of reaction buffer, MgC12, dNTPs, PCR primer pair(s), DNA polymerase, and water is prepared. Algae lysate in EDTA is added to provide a template for the reaction. The magnesium concentration is varied to compensate for amount and concentration of algae lysate and EDTA added. Annealing temperature gradients are employed to determine optimal annealing temperature for specific primer pairs.

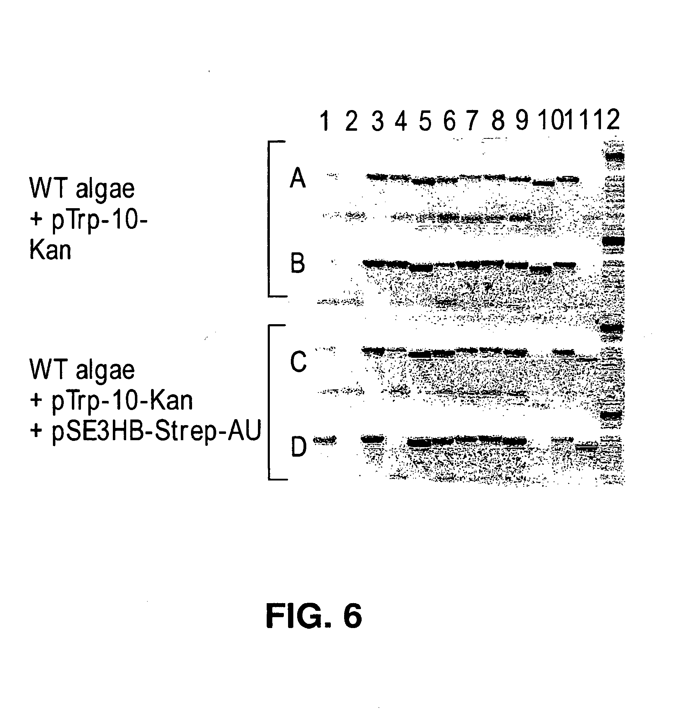

**Fig. 6** shows examples of isolated algae strains that contain pTrp-10-Kan with and without pSE-3HB-Strep-AU. Strains that integrated pTRP-10-Kan were identified using any of a set of primer pairs that amplify different regions in the vector backbone (Lane 1, SEQ ID NOs. 69 and 70; Lane 2, SEQ ID NOs. 71 and 72; Lane 3, SEQ ID NOs. 73 and 74; Lane 4, SEQ ID NOs. 75 and 76; Lane 5, SEQ ID NOs. 77 and 78; Lane 6, SEQ ID NOs. 79 and 80; Lane 7, SEQ ID NOs. 81 and 82; Lane 8, SEQ ID NOs. 83 and 84; and Lane 9, SEQ ID NOs. 85 and 86) or primer pairs that span the junction between pTrp-10-Kan DNA and chloroplast genome DNA (Lane 10, SEQ ID NOs. 87 and 88; Lane 11, SEQ ID NOs. 89 and 90). To identify strains that have integrated pSE-3HB-Strep-AU, primer pairs (SEQ ID NOs. 91 and 92) were used which amplify regions within the *ADE2* gene. Desired clones are those that yield PCR products of expected size.

### Example 6: Capture of a chloroplast genome into an exogenous host

In this example, *C*. *reinhardtii* chloroplast DNA is isolated using standard techniques as described above. *C. reinhardtii* chloroplast DNA containing the pTRP-10-Kan vector with or without pSE-3HB-Strep-AU was used to transform bacteria. Electrocompetent *E. coli* strains DH10B or Genehog were transformed and selected on LB agar growth medium with kanamycin (50 mg/l). DNA from individual clones was isolated by growing the cells to saturation at 37°C in LB liquid growth medium with kanamycin (50 mg/l). Saturated cell cultures are diluted 1:20 in LB+Kan+IPTG and grown at 37°C for 4 hours. The Plasmid Maxi kit (QIAGEN) is used to prepare plasmid DNA from the isolated clones.

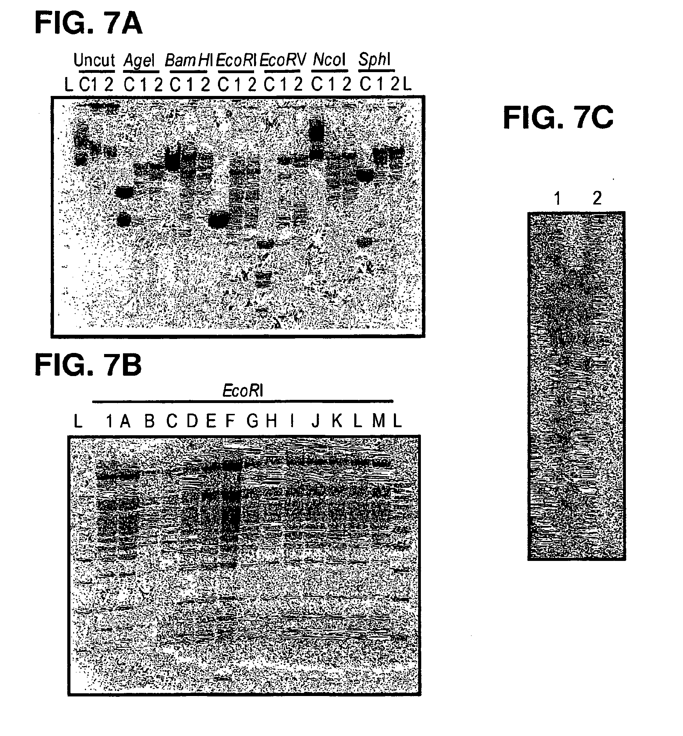

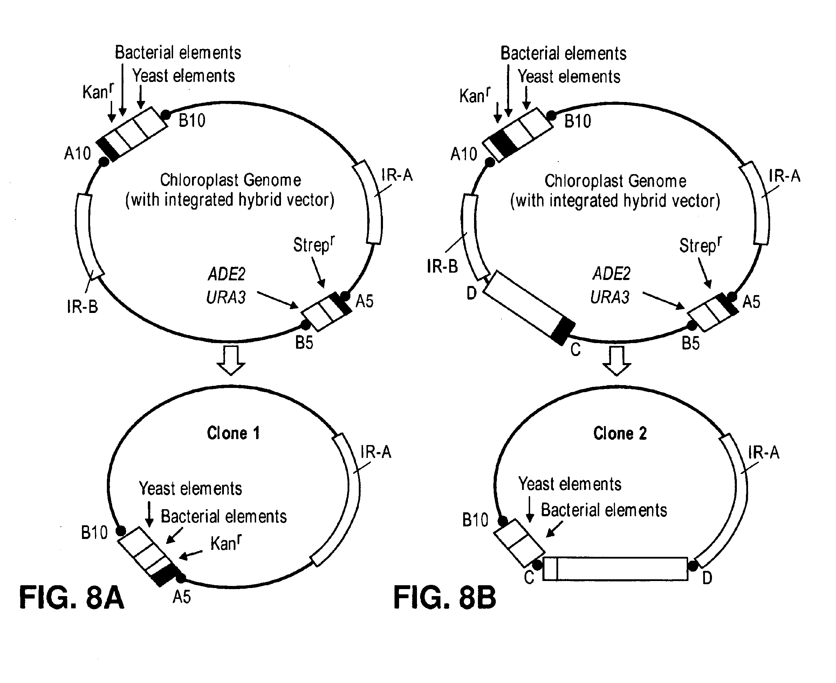

**Fig. 7A** shows the restriction analysis of two types of clones obtained from bacterial transformation (Clone 1 and Clone 2) compared to the parent hybrid vector (Clone C). Clones 1 and 2 are composed of > 100 kb of DNA while the parent hybrid vector is composed of only 23 kb of DNA, demonstrating that large portions of DNA were indeed captured by the hybrid vector. Restriction mapping indicates that Clone I comprises approximately half of the chloroplast genome (**FIG. 8A**). DNA sequencing of the regions flanking the hybrid vector indicate that a recombination event occurred between the 3' UTR of the *C*. *reinhardtii* kanamycin resistance cassette (which is the 3' UTR from the *rbcL* gene in C. *reinhardtii)* and the 3' UTR of the *C. reinhardtii* streptomycin resistance cassette (which is the 3' UTR from the *rbcL* gene in *C. reinhardtii).* Clone 1 retained the *C. reinhardtii* kanamycin resistance cassette, but lost the *C*. *reinhardtii* streptomycin resistance cassette. Restriction mapping indicates that Clone 2 also comprises approximately half of the chloroplast genome (**FIG. 8B**). However, DNA sequencing of the regions flanking the hybrid vector indicate that a different recombination event occurred to give rise to Clone 2. The 5' UTR of the C. *reinhardtii* kanamycin resistance cassette (which is the 5' UTR from the *atpA* gene in *C. reinhardtii)* recombined with the 5' UTR of the *atpA* gene in the *C*. *reinhardtii* chloroplast genome and the inverted repeat B (IR-B in **FIG. 8**) recombined with inverted repeat A (IR-A in **FIG. 8**). Clone 2 lost both the *C*. *reinhardtii* kanamycin resistance cassette and the *C*. *reinhardtii* streptomycin resistance cassette.

To demonstrate that the hybrid vector would support stable replication in yeast, Clone 1 was transformed into the yeast strain AB 1380 by the lithium acetate method and transfomants were selected on CSM-Trp agar media. Transformants were PCR screened using a primer pair (SEQ ID NOs 97 and 98) that amplifies a region with the chloroplast genome DNA of Clone 1. Desired clones are those that give rise to a PCR product of expected size. Individual PCR-positive clones were streaked to generate multiple clones per isolate. DNA was prepared from the isolated yeast clones and transformed into bacteria. Bacteria were PCR screened using a primer pair (SEQ ID NOs 97 and 98) that amplifies a region within the chloroplast genome DNA of Clone 1. Desired clones are those that give PCR products of expected size. DNA was prepared from the isolated bacterial clones and analyzed by restriction digest with *EcoR*I*.* Fig. 7B shows that all isolated clones have restriction maps that are identical to the originally isolated Clone 1. Thus, the hybrid vector supports stable replication in yeast.

To determine if the captured DNA is indeed chloroplast genome DNA, a Southern blot was performed. Clones 1 and 2 prepared from bacteria were digested with *EcoR*I*,* separated by gel electrophoresis, transferred to a membrane, and probed with radioactive *Hind*III-digested total DNA from *C*. *reinhardtii.* **Fig. 7C** shows that the DNA in Clones 1 and 2 give rise to a signal, thus indicating that the captured DNA is chloroplast genome DNA.

### Example 7: Reintroduction of chloroplast genome DNA into algae

For these experiments, all transformations are carried out on either *C*. *reinhardtii* strain W1.1, which expresses SAA from the endogenous *psbA* loci, or W1-1, which expresses LuxAB from the endogenous *psbA* loci. Both W1.1 and W1-1 are resistant to spectinomycin by virtue of transformation of p228, which introduces a mutation in the 16S rRNA. Cells are grown to late log phase (approximately 7 days) in TAP medium (Gorman and Levine, Proc. Natl. Acad. Sci., USA 54:1665-1669, 1965, which is incorporated herein by reference) at 23°C under constant illumination of 450 Lux on a rotary shaker set at 100 rpm. Fifty ml of cells are harvested by centrifugation at 4,000xg at 23°C for 5 min. The supernatant is decanted and cells resuspended in 4 ml HSM medium for subsequent chloroplast transformation by particle bombardment (Cohen et al., supra, 1998). All transformations are carried out on HSM agar.

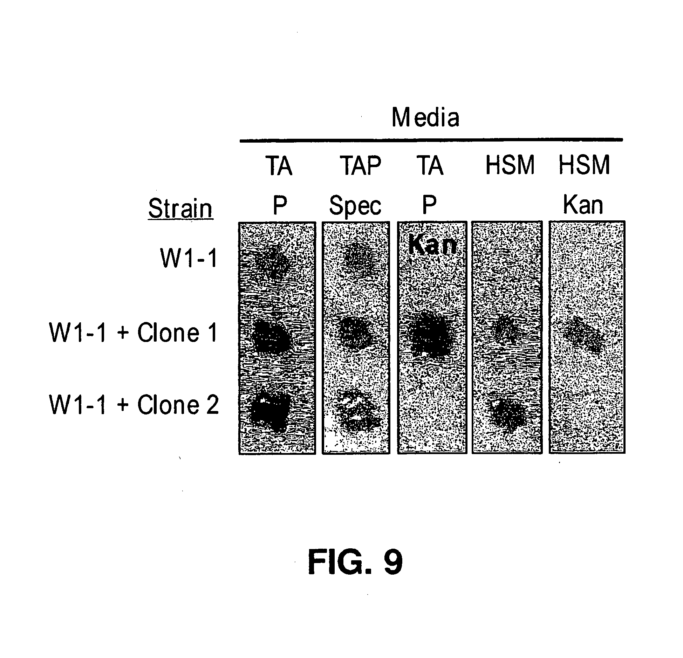

Transformants were selected by growth on HSM, indicating that function of the *psbA* gene locus was restored. Clones were also subsequently checked for growth on TAP, TAP with spectinomycin (150 µg/ml),TAP with kanamycin (100 µg/ml), HSM, and HSM with kanamycin (100 µg/ml). **Fig. 9** shows that W1-1 transformed with Clone 1 is able to grow on all media types. **Fig. 9** also shows that W1-1 transformed with Clone 2 is unable to grow on media containing kanamycin, which is expected since Clone 2 does not contain a Kan resistance marker.

### Example 8: Modification of Chloroplasts for Producing biomass-degrading enzymes

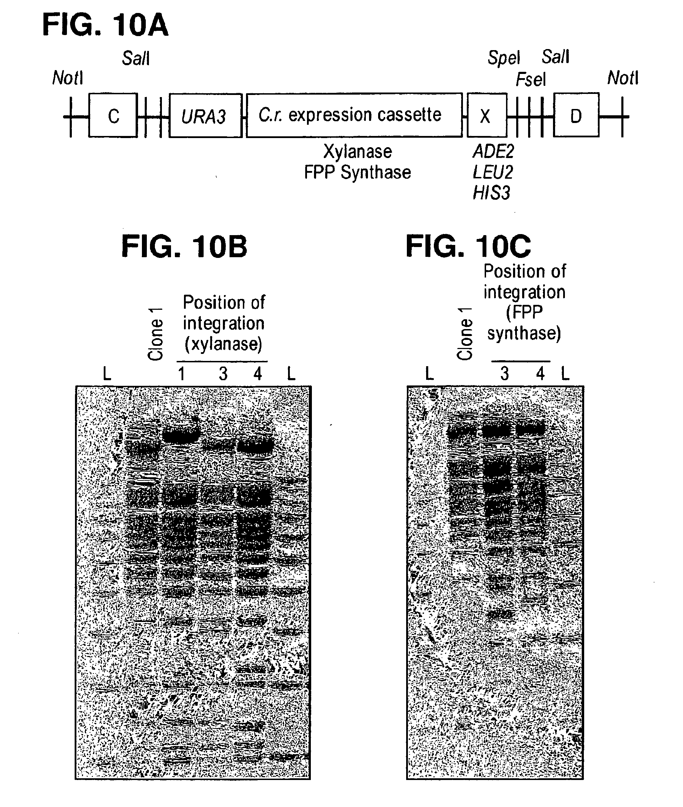

To modify captured chloroplast genome DNA for production of a xylanase, an algae expression cassette was cloned into the yeast marker vectors described in EXAMPLE 4. Briefly, a *C. reinhardtii* chloroplast expression vector was digested with SpeI to liberate a fragment of DNA (SEQ ID NO. 18) with xylanase from *T. reesei* regulated by the 5' UTR for the *psbD* gene from *C. reinhardtii* and the 3' UTR for the *psbA* gene from *C*. *reinhardtii.* The fragment was treated with Klenow fragment to create blunt ends and cloned into *Sma*I *(Xma*I*)* site between the yeast markers in pUC1-*URA3*/*ADE2*, pUC3-*URA3*/*LEU2,* and pUC4-*URA3*/*HIS3.* **Fig. 10A** shows the arrangement of the various elements in the new vectors.

Chloroplast genome DNA was modified by transforming the modification vector into yeast that harbored the captured DNA. For transformation, all modification vectors were linearized by digestion with *Not*I*.* Yeast harboring Clone 1 (from EXAMPLE 6) were grown to saturation in CSM-Trp media at 30°C, then diluted 1:20 in YPAD and grown for 4 hours at 30°C. Yeast were transformed using the lithium-acetate method and transformants were selected for by growth on CSM-Ura media. Transformants were propagated on CSM-Trp-Ura media and then PCR screened using primers specific for the xylanase expression cassette (SEQ ID NOs 95 and 96) and a region within the chloroplast genome DNA (SEQ ID NOs 97 and 98). Desired clones are those that give PCR products of expected size for both reactions. DNA was prepared from the isolated yeast clones and transformed into bacteria. Bacteria were PCR screened using primers specific for the xylanase expression cassette (SEQ ID NOs 95 and 96) and a region within the chloroplast genome DNA (SEQ ID NOs 97 and 98). Desired clones are those that give PCR products of expected size for both reactions. DNA was prepared from the isolated bacterial clones and analyzed by restriction digest with *EcoR*I*.* **Fig. 10B** shows that clones were isolated that have restriction maps that are consistent with integration of the xylanase expression cassette in the desired position.

For these experiments, all transformations are carried out on either *C*. *reinhardtii* strain W 1.1, which expresses SAA from the endogenous *psbA* loci, or W1-1, which expresses LuxAB from the endogenous *psbA* loci. Both W1-1 and W1-1 are resistant to spectinomycin by virtue of transformation of p228, which introduces a mutation in the 16S rRNA. Cells are grown to late log phase (approximately 7 days) in TAP medium (Gorman and Levine, Proc. Natl. Acad. Sci., USA 54:1665-1669, 1965, which is incorporated herein by reference) at 2³°C under constant illumination of 450 Lux on a rotary shaker set at 100 rpm. Fifty ml of cells are harvested by centrifugation at 4,000xg at 2³°C for 5 min. The supernatant is decanted and cells resuspended in 4 ml HSM medium for subsequent chloroplast transformation by particle bombardment (Cohen et al., supra, 1998). All transformations are carried out on HSM agar.

Transformants were identified by growth on HSM, indicating that function of the *psbA* gene locus was restored. PCR is used to identify transformants that also contain the endoxylanase expression cassette. For PCR analysis, 10⁶ algae cells (from agar plate or liquid culture) are suspended in 10 mM EDTA and heated to 95°C for 10 minutes, then cooled to near 23°C. A PCR cocktail consisting of reaction buffer, MgC12, dNTPs, PCR primer pair(s) (Table 2), DNA polymerase, and water is prepared. Algae lysate in EDTA is added to provide a template for the reaction. The magnesium concentration is varied to compensate for amount and concentration of algae lysate and EDTA added. Annealing temperature gradients are employed to determine optimal annealing temperature for specific primer pairs.

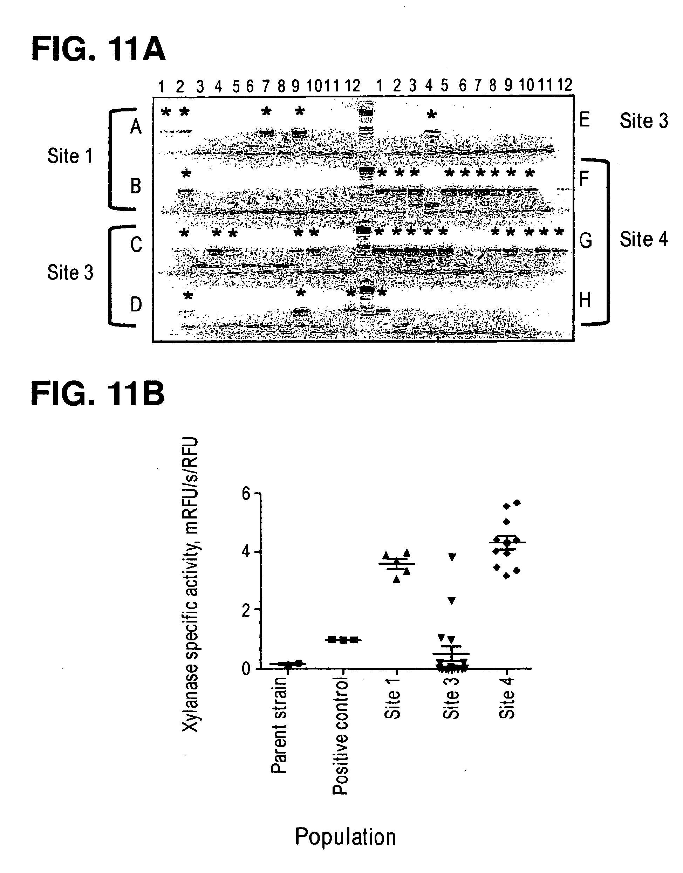

To identify strains that have the endoxylanase expression cassette, a primer pair (SEQ ID NOs 95 and 96) was used which amplifies a region within the endoxylanase expression cassette. Desired clones are those that yield PCR products of expected size. **Fig. 11A** shows that multiple algae strains were obtained that contain the endoxylanase expression cassette (PCR products indicated with asterisk).

To determine whether functional xylanase is expressed, enzyme activity is examined. Patches of cells (approximately 2mg per patch) from TAP agar plates containing kanamycin (100 µg/mL)were resuspended in 50 ul of 100mM sodium acetate pH 4.8 in a round bottom 96 well plate (Coming). Resuspended cells were lysed by addition of 20ul of BugBuster Protein Extraction Reagent (Novagen) and shaken for five minutes at room temperature. Cell lysate (50ul) was transferred to a black 96 well plate, and the chlorophyll fluorescence of the resulting wells was measured in a SpectraMax M2 microplate reader (Molecular Devices), with an excitation wavelength of 440 nm and an emission wavelength of 740 nm, with a 695 nm cutoff filter. The measured chlorophyll signal in RFUs (relative fluorescence units) was used to normalize the xylanase activity signal to the amount of biomass added to the reaction.

After measurement of the chlorophyll fluorescence, xylanase substrate was added. EnzCheck Ultra Xylanase substrate (Invitrogen) was dissolved at a concentration of 50ug/ml in 100mM sodium acetate pH 4.8, and 50ul of substrate was added to each well of the microplate. The fluorescent signal was measured in a SpectraMax M2 microplate reader (Molecular Devices), with an excitation wavelength of 360 nm and an emission wavelength of 460 nm, without a cutoff filter and with the plate chamber set to 42 degrees Celsius. The fluorescence signal was measured for 15 minutes, and the enzyme velocity was calculated with Softmax Pro v5.2 (Molecular Devices). Enzyme velocities were recorded as RFU/minute. Enzyme specific activities were calculated as milliRFU per minute per RFU of chlorophyll fluorescence. **Fig. 11B** shows that multiple algae strains containing the xylanase expression cassette at site 1, 3, and 4 (see **FIGS. 3** and **10**) were obtained that produce functional xylanase enzyme.

### Example 9: Modification of a chloroplast genome to produce terpenes

To modify captured chloroplast genome DNA for production of an FPP synthase, an algae expression cassette was cloned into the yeast marker vectors described in EXAMPLE 4. Briefly, a *C*. *reinhardtii* chloroplast expression vector was digested with SpeI to liberate a fragment of DNA (SEQ ID NO. 19) with FPP synthase from *G. gallus* regulated by the 5' UTR for the *psbD* gene from C. *reinhardtii* and the 3' UTR for the *psbA* gene from C. *reinhardtii.* The fragment was treated with Klenow fragment to create blunt ends and cloned into *Sma*I (*Xma*I) site between the yeast markers in pUC1-*URA3*/*ADE2*, pUC3-*URA3*/*LEU2,* and pUC4-*URA3*/*HIS3.* **Fig. 10A** shows the arrangement of the various elements in the new vectors.

Chloroplast genome DNA was modified by transforming the modification vector into yeast that harbored the captured DNA. For transformation, all modification vectors were linearized by digestion with ***Not*I*.*** Yeast harboring Clone 1 (from EXAMPLE 6) were grown to saturation in CSM-Trp media at 30°C, then diluted 1:20 in YPAD and grown for 4 hours at 30°C. Yeast were transformed using the lithium-acetate method and transformants were selected for by growth on CSM-Ura media. Transformants were propagated on CSM-Trp-Ura media and then PCR screened using primers specific for the FPP synthase expression cassette (SEQ ID NOs 95 and 99) and a region within the chloroplast genome DNA (SEQ ID NOs 97 and 98). Desired clones are those that gave PCR products of expected size for both reactions. DNA was prepared from the isolated yeast clones and transformed into bacteria. Bacteria were PCR screened using primers specific for the FPP synthase expression cassette (SEQ ID NOs 95 and 99) and a region within the chloroplast genome DNA (SEQ IDs 97 and 98). Desired clones are those that gave PCR products of expected size for both reactions. DNA was prepared from the isolated bacterial clones and analyzed by restriction digest. **Fig. 10C** shows that clones were isolated that have restriction maps that are consistent with integration of the FPP synthase expression cassette in the desired position.

For these experiments, all transformations are carried out on either *C*. *reinhardtii* strain W1.1, which expresses SAA from the endogenous *psbA* loci, or W1-1, which expresses LuxAB from the endogenous *psbA* loci. Both W1.1 and W1-1 are resistant to spectinomycin by virtue of transformation of p228, which introduces a mutation in the 16S rRNA. Cells are grown to late log phase (approximately 7 days) in TAP medium (Gorman and Levine, Proc. Natl. Acad. Sci., USA 54:1665-1669, 1965, which is incorporated herein by reference) at 23°C under constant illumination of 450 Lux on a rotary shaker set at 100 rpm. Fifty ml of cells are harvested by centrifugation at 4,000xg at 23°C for 5 min. The supernatant is decanted and cells resuspended in 4 ml HSM medium for subsequent chloroplast transformation by particle bombardment (Cohen et al., supra, 1998). All transformations are carried out on HSM agar.

Transformants were identified by growth on HSM, indicating that function of the *psbA* gene locus was restored. PCR is used to identify transformants that also contain the FPP synthase expression cassette. For PCR analysis, 10⁶ algae cells (from agar plate or liquid culture) are suspended in 10 mM EDTA and heated to 95°C for 10 minutes, then cooled to near 23°C. A PCR cocktail consisting of reaction buffer, MgC12, dNTPs, PCR primer pair(s), DNA polymerase, and water is prepared. Algae lysate in EDTA is added to provide a template for the reaction. The magnesium concentration is varied to compensate for amount and concentration of algae lysate and EDTA added. Annealing temperature gradients are employed to determine optimal annealing temperature for specific primer pairs.

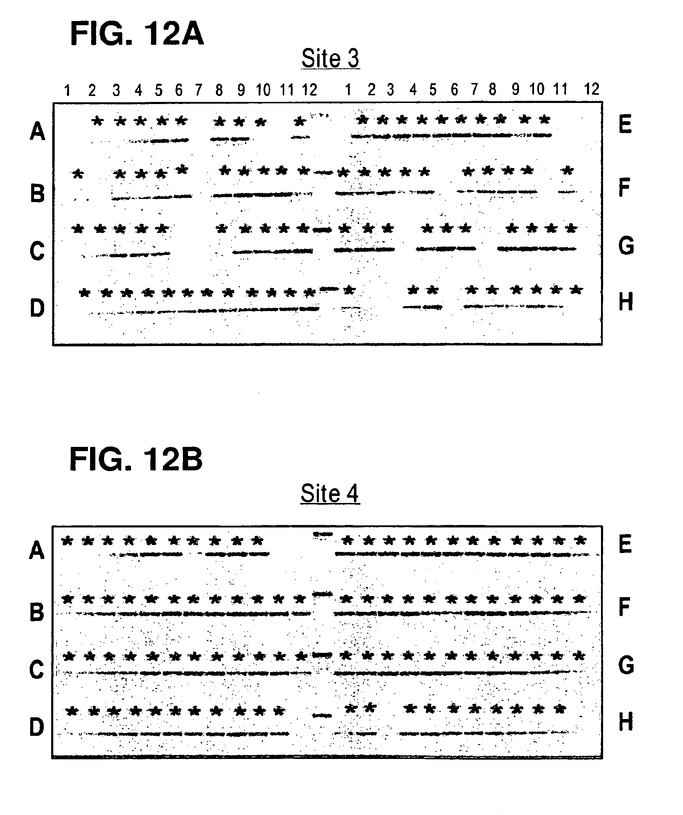

To identify strains that have the FPP synthase expression cassette, a primer pair was used which amplifies a region within the endoxylanase expression cassette. Desired clones are those that yield PCR products of expected size. Multiple algae strains were obtained that contain the FPP synthase expression cassette at (**FIG. 12**, PCR products indicated with asterisk).

### Example 11: Gap-filling a partial chloroplast genome

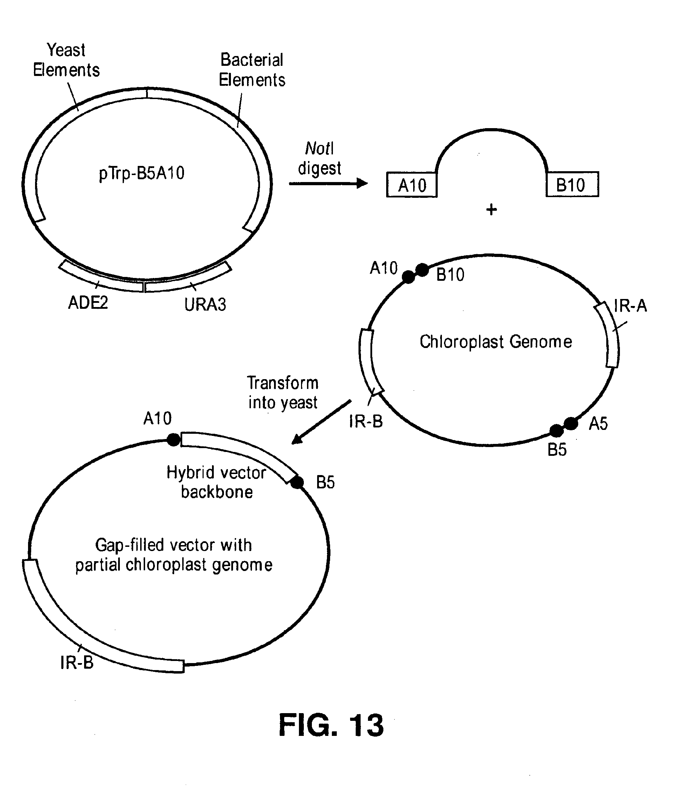

In this example, a system is established using a hybrid gap-filling vector to capture chloroplast DNA by recombination in yeast (**FIG. 13**). To adapt the hybrid gap-filling vector to capture chloroplast DNA, the vector pDOCI-B5A10 (SEQ ID NO. 20) was generated. Portions of the *C*. *reinhardtii* chloroplast genome were PCR amplified using two primer pairs specific for a region adjacent the *psbD* gene (A10, SEQ ID NOs. 27 and 28) and a region adjacent the near the *psbH* gene (B5, SEQ ID NOs. 787 and 788). Each PCR product was digested with *Not*I and I*-Sce*I and ligated to pDOCI (SEQ ID NO. 1) that was digested with I*-Sce*I to form pDOCI-B5A10 (SEQ ID NO. 20).

The hybrid gap-filling for capturing chloroplast DNA, pTRP-B5A10, was constructed using recombination in yeast. Briefly, pDOCI-B5A10 (SEQ ID NO. 20) was digested with *Pac*I and *Asc*I to liberate the cassette that introduces chloroplast genome-specific elements into the hybrid gap-filling vector. This cassette was transformed along with pTRP-AU into the yeast strain YPH858 using the lithium acetate method. Homologous recombination takes place in vivo in the transformed yeast cells. Transformants that correctly integrated with cassette were isolated based on growth on CSM-Trp agar media containing 5-fluorooratic acid (5-FOA) and by red color. 5-FOA selects for clones that lack a functional *URA3* gene and the red color results when the *ADE2* gene is eliminated. Plasmid DNA was isolated from yeast clones that were grown in CSM-Trp liquid media and transformed into *E. coli* (DH10B). To generate large amounts of pTRP-10-Kan, DH10B cells harboring pTRP-10-Kan were grown to saturation at 37°C in LB+Kan (50 ug/mL), and then diluted 1:20 in LB+Kan+IPTG and grown for 4 hours at 37°C. DNA was prepared from the bacterial culture using the Plasmid Maxi kit (QIAGEN).

The desired genomic DNA is captured by using the sites in a gap filling vector that have high homology to the regions of the target genomic DNA (indicated by A10 and B5 in FIG. 13). Linearized gap filling vector DNA and chloroplast genome DNA are used to transform S. *cerevisiae* using the lithium acetate or spheroplast methods as described in EXAMPLE 2. Homologous recombination takes place in vivo in the transformed yeast cells. Once the target DNA is captured by the vector via homologous recombination, the DNA can be stably replicated in both yeast and bacterial systems. As indicated in **FIG. 1****,** the gap filling vector contains the selectable marker TRP 1. Therefore, the yeast cells are plated on tryptophan-free medium to screen for positive transformants. Transformed strains growing on the tryptophan-free medium are screened for the presence of target DNA inserts in the gap filling plasmid. Such screening can be by any known method in the art, for example, restriction enzyme digestion, PCR and/or gel electrophoresis.

Yeast transformants screened by PCR using primers specific for chloroplast genome DNA (SEQ IDs 89 and 90). Desired clones are those that give a PCR product of expected size. DNA is prepared from the isolated yeast clones and transformed into bacteria. Bacterial transformants are PCR screened using primers specific for chloroplast genome DNA (SEQ IDs 89 and 90). Desired clones are those that give a PCR product of expected size. DNA is prepared from the isolated bacterial clones and analyzed by restriction digest with *EcoR*I.

### Example 12: Gap-filling a complete chloroplast genome

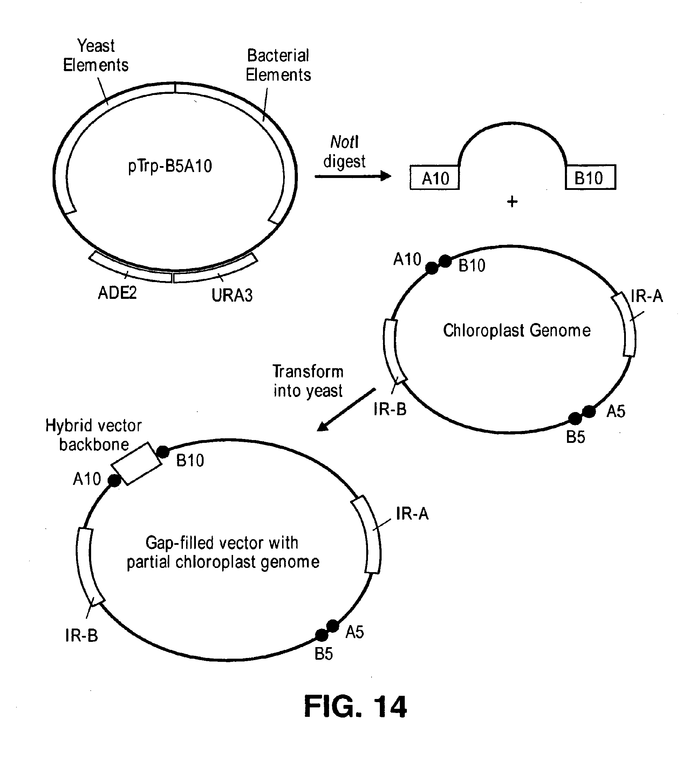

In this example, a system is established using a hybrid gap-filling vector to capture a complete chloroplast genome by recombination in yeast (FIG. 14). The hybrid gap-filling for capturing chloroplast DNA, pTRP-10, was constructed using recombination in yeast. Briefly, pDOCI-10 (described in EXAMPLE 3, SEQ ID NO. 3) was digested with *Pac*I and *Asc*I to liberate the cassette that introduces chloroplast genome-specific elements into the hybrid gap-filling vector. This cassette was transformed along with pTRP-AU into the yeast strain YPH858 using the lithium acetate method. Homologous recombination takes place in vivo in the transformed yeast cells. Transformants that correctly integrated with cassette were isolated based on growth on CSM-Trp agar media containing 5-fluorooratic acid (5-FOA) and by red color. 5-FOA selects for clones that lack a functional *URA3* gene and the red color results when the *ADE2* gene is eliminated. Plasmid DNA was isolated from yeast clones that were grown in CSM-Trp liquid media and transformed into *E. coli* (DH10B). To generate large amounts of pTRP-10-Kan, DH10B cells harboring pTRP-10-Kan were grown to saturation at 37°C in LB+Kan (50 ug/mL), and then diluted 1:20 in LB+Kan+IPTG and grown for 4 hours at 37°C. DNA was prepared from the bacterial culture using the Plasmid Maxi kit (QIAGEN).

The desired genomic DNA is captured by using the sites in a gap filling vector that have high homology to the adjacent regions of the target genomic DNA (indicated by A10 and B10 in **FIG. 14****).** Linearized gap filling vector DNA and chloroplast genome DNA are used to transform *S. cerevisiae* using the lithium acetate or spheroplast methods as described in EXAMPLE 2. Homologous recombination takes place in vivo in the transformed yeast cells. Once the target DNA is captured by the vector via homologous recombination, the DNA can be stably replicated in both yeast and bacterial systems. As indicated in **FIG. 1****,** the gap filling vector contains the selectable marker TRP 1. Therefore, the yeast cells are plated on tryptophan-free medium to screen for positive transformants. Transformed strains growing on the tryptophan-free medium are screened for the presence of target DNA inserts in the gap filling plasmid. Such screening can be by any known method in the art, for example, restriction enzyme digestion, PCR and/or gel electrophoresis.

Yeast transformants screened by PCR using primers specific for chloroplast genome DNA (SEQ IDs 89 and 90). Desired clones are those that give a PCR product of expected size. DNA is prepared from the isolated yeast clones and transformed into bacteria. Bacterial transformants are PCR screened using primers specific for chloroplast genome DNA (SEQ IDs 89 and 90). Desired clones are those that give a PCR product of expected size. DNA is prepared from the isolated bacterial clones and analyzed by restriction digest with *EcoR*I.

Example 13: Reassembly on a complete chloroplast genome

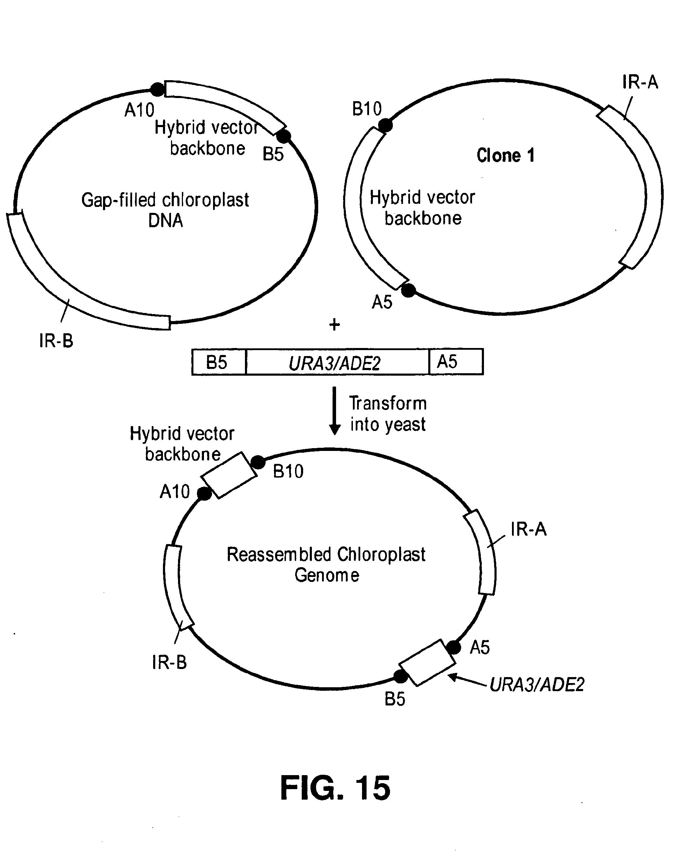

In some instances, the chloroplast genome may be divided into different plasmids, which, in total, comprise the entirety of the genome **(****FIG. 15****).** In such instances, capture of smaller portions may facilitate rapid and complex modification of multiple positions within the genome. The chloroplast genome fragments are then combined to reform a complete (and possibly modified) chloroplast genome.

In this example, the chloroplast is divided between two vectors. One vector consists of the chloroplast genome DNA from B5 to A10, or nucleotide 76,400 to 176,500 (according to the sequence available from NCBI, NC_005353), respectively. This vector may be obtained by the procedure describe in EXAMPLE 11. The other vector consists of the chloroplast genome DNA from B10 to A5, or nucleotide 176,500 to 76,400 (according to the sequence available from NCBI, NC_005353), respectively. This vector is the same one as Clone 1 described in may be obtained by the procedure described in EXAMPLE 6. Both vector share the hybrid vector sequence and can use that as a region of homology for recombination. In this example, the recombination event insert the hybrid vector sequenc between regions A10 and B10 (Fig. 5). However, there is no homology sufficient to facilitate recombination that joins regions A5 and B5 (Fig. 5). Thus, a third vector is added that has selectable markers between sequences homologous to A5 and B5.

The chloroplast genome is reassembled by combining all three vectors and transforming S. *cerevisiae* using the lithium acetate or spheroplast methods as described in EXAMPLE 2. Homologous recombination takes place in vivo in the transformed yeast cells. Once the target DNA is created via homologous recombination, the DNA can be stably replicated in both yeast and bacterial systems. As indicated in **Fig. 15****,** the third vector contains the selectable markers *URA3* and *ADE2.* Therefore, the yeast cells are plated on tryptophan-free medium to screen for positive transformants. Transformed strains growing on the uracil and/or adenine-free medium are screened for the presence of target DNA inserts in the gap filling plasmid. Such screening can be by any known method in the art, for example, restriction enzyme digestion, PCR and/or gel electrophoresis.

Yeast transformants screened by PCR using primers specific for chloroplast genome DNA (SEQ IDs 89 and 90). Desired clones are those that give a PCR product of expected size. DNA is prepared from the isolated yeast clones and transformed into bacteria. Bacterial transformants are PCR screened using primers specific for chloroplast genome DNA (SEQ IDs 89 and 90). Desired clones are those that give a PCR product of expected size. DNA is prepared from the isolated bacterial clones and analyzed by restriction digest with *EcoR*I.

### Example 14: Vectors and methods to remove regions chloroplast genome DNA in an exogenous host

To generate vectors that remove regions of chloroplast genome DNA, 800-1000 bp regions of chloroplast DNA are amplified using PCR primer pairs that anneal to 5' and 3' regions flanking the sites indicated in Fig. 4 (site 1-5', SEQ ID NOs. 1079 and 1080; site 1-3', SEQ ID NOs. 1125 and 1126; site 3-5', SEQ ID NOs. 1083 and 1084; site 3-3', SEQ ID NOs. 1129 and 1130; site 4-5', SEQ ID NOs. 1087 and 1088; site 4-3', SEQ ID NOs. 1133 and 1134; site 5-5', SEQ ID NOs. 789 and 790; site 5-3', SEQ ID NOs. 787 and 788; and site 7-5', SEQ ID NOs. 1091 and 1092; site 7-3', SEQ ID NOs. 1089 and 1090). Pairs of PCR products from the 5' and 3' regions for different sites are digested with *Not*I and I-*Sce*I, mixed, and ligated to *Not*I-digested pUC-SE (SEQ ID NO. 2). Pairs of yeast selection markers (described in EXAMPLE 4) are cloned between the 5' and 3' fragments using *Sal*I*.*

Regions of chloroplast genome DNA are removed by transforming the deletion vector into yeast that harbor the captured DNA. For transformation, all modification vectors were linearized by digestion with *Not*I. Yeast harboring the desired clone are grown to saturation in CSM-Trp media at 30°C, then diluted 1:20 in YPAD and grown for 4 hours at 30°C. Yeast were transformed using the lithium-acetate method and transformants were selected for by growth on CSM-Ura media. Transformants were propagated on CSM-Trp-Ura media and then PCR screened using primers specific for the targeted region and a region of the chloroplast genome DNA not targeted by the deletion vector. Desired clones are those that give not product for the targeted region, but do give a PCR product of expected size for the untargeted area. DNA is prepared from the isolated yeast clones and transformed into bacteria. Bacteria PCR screened using primers specific for the targeted region and a region of the chloroplast genome DNA not targeted by the deletion vector. Desired clones are those that give not product for the targeted region, but do give a PCR product of expected size for the untargeted area. DNA is prepared from the isolated bacterial clones and analyzed by restriction digest.

Various modifications, processes, as well as numerous structures that may be applicable herein will be apparent. Various aspects, features or embodiments may have been explained or described in relation to understandings, beliefs, theories, underlying assumptions, and/or working or prophetic examples, although it will be understood that any particular understanding, belief, theory, underlying assumption, and/or working or prophetic example is not limiting. Although the various aspects and features may have been described with respect to various embodiments and specific examples herein, it will be understood that any of same is not limiting with respect to the full scope of the appended claims or other claims that may be associated with this application.

## Claims

1. A method of assembling a circular chloroplast genome, comprising
obtaining a plurality of vectors, collectively comprising all chloroplast genes necessary to carry out photosynthesis;
wherein each vector in said plurality of vectors shares regions of homology for homologous recombination with one or two other vectors in said plurality of vectors,
wherein at least one vector of said plurality of vectors comprises a yeast DNA replication element and at least one vector of said plurality of vectors comprises a bacterial DNA replication element, and
wherein at least one vector of said plurality of vectors comprises a yeast stability element comprising at least one yeast selection marker;
introducing said plurality of vectors into yeast to assemble said plurality of vectors by homologous recombination to produce an assembled circular chloroplast genome; and
selecting for complete assembly of said circular chloroplast genome by selecting for the presence of said at least one stability element.

2. The method of claim 1, wherein said plurality of vectors comprises two vectors and each of said vectors shares regions of homology for recombination with the other vector.

3. The method of claim 1 or 2, wherein said yeast DNA replication element is a yeast centromere, a yeast autonomous replicating sequence, or both.

4. The method of claim 1 or 2, wherein said bacterial DNA replication element is a P1 replication sequence or an F factor replication sequence.

5. The method of any one of claims 1-4, wherein said assembled circular chloroplast genome comprises a single yeast centromere.

6. The method of any one of claims 1-5, wherein said yeast DNA replication element and said bacterial DNA replication element are contained in the same vector.

7. The method of any one of claims 1-6, wherein said assembled circular chloroplast genome contains at least 140kb of DNA.

8. The method of any one of claims 1-7, where at least one of said vectors comprises synthetic DNA.

9. The method of any one of claims 1-7, wherein at least one of said vectors comprises genomic chloroplast DNA from a photosynthetic organism.

10. The method of claim 9, wherein said photosynthetic organism is an alga.

11. The method of claim 10, wherein the alga is macroalgae or microalgae.

12. The method of claim 11, wherein the microalgae is a *Chlamydomanas sp., Chlorella sp., Dunaliella sp., Haematococcus sp.* or *Scenedesmus sp..*

13. The method of claim 9, wherein said genomic choloplast DNA from a photosynthetic organism is modified.

14. The method of claim 13, wherein said at least one modification is insertion of a heterologous or homologous polynucleotide, deletion of one or more nucleic acid bases, mutation of one or more nucleic acid bases, rearrangement of one or more polynucleotides, or a combination thereof.

15. The method of any one of claims 1-14, wherein said yeast stability element comprises a pair of yeast selection markers.

16. The method of claim 15, wherein said pair of selection markers is a URA3 gene and an additional yeast selection marker.

17. The method of claim 16, wherein said additional marker is an ADE2 gene, a LEU2 gene, a HIS3 gene, a LYS2 gene or a G418 gene.

18. The method of claim 17, wherein for each vector comprising a yeast stability element, said yeast stability element comprises a different pair of yeast selection markers.

19. The method of any one of the preceding claims, further comprising introducing said assembled circular chloroplast genome into a host organism.

20. The method of claim 19, wherein the host organism is a eukaryote, yeast or alga.

21. The method of any one of claims 1-20, further comprising removing said at least one yeast stability element after selecting for complete assembly of said circular chloroplast genome.

22. The method of any one of claims 1-21, further comprising removing said yeast and bacterial replication elements after all desired manipulations.

23. An assembled circular chloroplast genome, comprising
a plurality of vectors, collectively comprising all chloroplast genes necessary for photosynthesis;
wherein said circular chloroplast genome is assembled by a method of any one of claims 1-22.

## Patentansprüche

1. Verfahren zur Assemblierung eines kreisförmigen Chloroplastengenoms, das Folgendes umfasst:
das Erhalten einer Vielzahl von Vektoren, die zusammen alle Chloroplastengene umfassen, die zur Ausführung von Photosynthese benötigt werden;
worin jeder Vektor aus der Vielzahl von Vektoren Homologieregionen zur homologen Rekombination mit einem oder zwei anderen Vektoren aus der Vielzahl von Vektoren teilt,
worin zumindest ein Vektor aus der Vielzahl von Vektoren ein Hefe-DNA-Replikationselement umfasst und zumindest ein Vektor aus der Vielzahl von Vektoren ein Bakterien-DNA-Replikationselement umfasst und
worin zumindest ein Vektor aus der Vielzahl von Vektoren ein Hefestabilitätselement umfasst, das zumindest einen Hefeselektionsmarker umfasst;
das Einführen der Vielzahl von Vektoren in Hefe, um die Vielzahl von Vektoren durch homologe Rekombination zu assemblieren, um ein assembliertes kreisförmiges Chloroplastengenom herzustellen; und
das Selektieren auf eine vollständige Assemblierung des kreisförmigen Chloroplastengenoms durch das Selektieren auf die Gegenwart des zumindest einen Stabilitätselements.

2. Verfahren nach Anspruch 1, worin die Vielzahl von Vektoren zwei Vektoren umfasst und jeder dieser Vektoren Homologieregionen zur Rekombination mit dem jeweils anderen Vektor teilt.

3. Verfahren nach Anspruch 1 oder 2, worin das Hefe-DNA-Replikationselement ein Hefecentromer, eine autonome Hefereplikationssequenz oder beides ist.

4. Verfahren nach Anspruch 1 oder 2, worin das Bakterien-DNA-Replikationselement eine P1-Replikationssequenz oder eine F-Faktor-Replikationssequenz oder beides ist.

5. Verfahren nach einem der Ansprüche 1 bis 4, worin das assemblierte kreisförmige Chloroplastengenom ein einziges Hefecentromer umfasst.

6. Verfahren nach einem der Ansprüche 1 bis 5, worin das Hefe-DNA-Replikationselement und das Bakterien-DNA-Replikationselement in demselben Vektor enthalten sind.

7. Verfahren nach einem der Ansprüche 1 bis 6, worin das assemblierte kreisförmige Chloroplastengenom zumindest 140 kb DNA enthält.

8. Verfahren nach einem der Ansprüche 1 bis 7, worin zumindest einer der Vektoren synthetische DNA umfasst.

9. Verfahren nach einem der Ansprüche 1 bis 7, worin zumindest einer der Vektoren genomische Chloroplasten-DNA aus einem photosynthetischen Organismus umfasst.

10. Verfahren nach Anspruch 9, worin der photosynthetische Organismus eine Alge ist.

11. Verfahren nach Anspruch 10, worin die Alge eine Makroalge oder Mikroalge ist.

12. Verfahren nach Anspruch 11, worin die Mikroalge *Chlamydomanas sp., Chlorella sp., Dunaliella sp., Haematococcus sp.* oder *Scenedesmus sp.* ist.

13. Verfahren nach Anspruch 9, worin die genomische Chloroplasten-DNA aus einem photosynthetischen Organismus modifiziert ist.

14. Verfahren nach Anspruch 13, worin die zumindest eine Modifikation die Insertion eines heterologen oder homologen Polynucleotids, die Deletion einer oder mehrerer Nucleinsäurebasen, die Mutation einer oder mehrerer Nucleinsäurebasen, die Umordnung eines oder mehrerer Polynucleotide oder eine Kombination daraus ist.

15. Verfahren nach einem der Ansprüche 1 bis 14, worin das Hefestabilitätselement ein Paar aus Hefeselektionsmarkern umfasst.

16. Verfahren nach Anspruch 15, worin das Paar aus Hefeselektionsmarkern ein URA3-Gen und ein zusätzlicher Hefeselektionsmarker ist.

17. Verfahren nach Anspruch 16, worin der zusätzliche Marker ein ADE2-Gen, ein LEU2-Gen, ein HIS3-Gen, eine LYS2-Gen oder ein G418-Gen ist.

18. Verfahren nach Anspruch 17, worin bei jedem Vektor, der ein Hefestabilitätselement umfasst, das Hefestabilitätselement ein anderes Paar aus Hefeselektionsmarkern umfasst.

19. Verfahren nach einem der vorangegangenen Ansprüche, das weiters das Einführen des assemblierten kreisförmigen Chloroplastengenoms in einen Wirtsorganismus umfasst.

20. Verfahren nach Anspruch 19, worin der Wirtsorganismus ein Eukaryot, eine Hefe oder eine Alge ist.

21. Verfahren nach einem der Ansprüche 1 bis 20, das weiters das Entfernen des zumindest einen Hefestabilitätselements nach dem Selektieren auf vollständige Assemblierung des kreisförmigen Chloroplastengenoms umfasst.

22. Verfahren nach einem der Ansprüche 1 bis 21, das weiters das Entfernen des Hefe- und des Bakterien-Replikationselements nach sämtlichen gewünschten Manipulationen umfasst.

23. Assembliertes kreisförmiges Chloroplastengenom, das:
eine Vielzahl von Vektoren umfasst, die zusammen alle Chloroplastengene umfassen, die zur Photosynthese benötigt werden;
worin das kreisförmige Chloroplastengenom durch ein Verfahren nach einem der Ansprüche 1 bis 22 assembliert wird.

## Revendications

1. Méthode d'assemblage d'un génome de chloroplaste circulaire, comprenant :
obtenir une pluralité de vecteurs, comprenant collectivement tous les gènes de chloroplaste nécessaires pour exécuter une photosynthèse ;
où chaque vecteur dans ladite pluralité de vecteurs partage des régions d'homologie pour une recombinaison homologue avec un ou deux autres vecteurs dans ladite pluralité de vecteurs ;
où au moins un vecteur de ladite pluralité de vecteurs comprend un élément de réplication d'ADN de levure et au moins un vecteur de ladite pluralité de vecteurs comprend un élément de réplication d'ADN bactérien, et
où au moins un vecteur de ladite pluralité de vecteurs comprend un élément de stabilité de levure comprenant au moins un marqueur de sélection de levure ;
introduire ladite pluralité de vecteurs dans la levure pour assembler ladite pluralité de vecteurs par recombinaison homologue pour produire un génome de chloroplaste circulaire assemblé ; et
sélectionner l'assemblage complet dudit génome de chloroplaste circulaire en sélectionnant la présence dudit au moins un élément de stabilité.

2. Méthode selon la revendication 1, dans laquelle ladite pluralité de vecteurs comprend deux vecteurs, et chacun desdits vecteurs partage des régions d'homologie pour la recombinaison avec l'autre vecteur.

3. Méthode selon la revendication 1 ou 2, dans laquelle ledit élément de réplication d'ADN de levure est un centromère de levure, une séquence de réplication autonome de levure, ou les deux.

4. Méthode selon la revendication 1 ou 2, dans laquelle ledit élément de réplication d'ADN bactérien est une séquence de réplication P1 ou une séquence de réplication de facteur F.

5. Méthode selon l'une quelconque des revendications 1 à 4, dans laquelle ledit génome de chloroplaste circulaire assemblé comprend un seul centromère de levure.

6. Méthode selon l'une quelconque des revendications 1 à 5, dans laquelle ledit élément de réplication d'ADN de levure et ledit élément de réplication d'ADN bactérien se trouvent dans le même vecteur.

7. Méthode selon l'une quelconque des revendications 1 à 6, dans laquelle ledit génome de chloroplaste circulaire assemblé contient au moins 140kb d'ADN.

8. Méthode selon l'une quelconque des revendications 1 à 7, dans laquelle au moins un desdits vecteurs comprend de l'ADN synthétique.

9. Méthode selon l'une quelconque des revendications 1 à 7, dans laquelle au moins un desdits vecteurs comprend l'ADN de chloroplaste génomique d'un organisme photosynthétique.

10. Méthode selon la revendication 9, dans laquelle ledit organisme photosynthétique est une algue.

11. Méthode selon la revendication 10, dans laquelle l'algue est une macro-algue ou une micro-algue.

12. Méthode selon la revendication 11, où la micro-algue est une *Chlamydomanas sp*., *Chlorella sp*., *Dunaliella sp., Haematococcus sp. Ou Scenedesmus sp.*

13. Méthode selon la revendication 9, dans laquelle ledit ADN de chloroplaste génomique d'un organisme photosynthétique est modifié.

14. Méthode selon la revendication 13, dans laquelle au moins une modification est l'insertion d'un polynucléotide hétérologue ou homologue, une délétion d'une ou de plusieurs bases d'acide nucléique, la mutation d'une ou de plusieurs bases d'acide nucléique, le réagencement d'un ou de plusieurs polynucléotides ou une combinaison de ceux-ci.

15. Méthode selon l'une quelconque des revendications 1 à 14, dans laquelle ledit élément de stabilité de levure comprend une paire de marqueurs de sélection de levure.

16. Méthode selon la revendication 15, dans laquelle ladite paire de marqueurs de sélection est un gène URA3 et un marqueur de sélection de levure additionnel.

17. Méthode selon la revendication 16, dans laquelle ledit marqueur additionnel est un gène ADE2, un gène LEU2, un gène HIS3, un gène LYS2 ou un gène G418.

18. Méthode selon la revendication 17, dans laquelle, pour chaque vecteur comprenant un élément de stabilité de levure, ledit élément de stabilité de levure comprend une paire différente de marqueurs de sélection de levure.

19. Méthode selon l'une quelconque des revendications précédentes, comprenant en outre l'introduction dudit génome de chloroplaste circulaire assemblé dans un organisme hôte.

20. Méthode selon la revendication 19, dans laquelle ledit organisme hôte est un eucaryote, une levure ou une algue.

21. Méthode selon l'une quelconque des revendications 1 à 20, comprenant en outre le retrait dudit au moins un élément de stabilité de levure après la sélection pour l'assemblage complète dudit génome de chloroplaste circulaire.

22. Méthode selon l'une quelconque des revendications 1 à 21, comprenant en outre le retrait de ladite levure et des éléments de réplication bactériens après toutes les manipulations souhaitées.

23. Génome de chloroplaste circulaire assemblé, comprenant :
une pluralité de vecteurs, comprenant collectivement tous les gènes de chloroplaste nécessaires pour une photosynthèse ;
où ledit génome de chloroplaste circulaire est assemblé par une méthode selon l'une quelconque des revendications 1 - 22.
